# EUROPÄISCHE PATENTSCHRIFT

(11) **EP 3 294 803 B1**
(45) Veröffentlichungstag und Bekanntmachung des Hinweises auf die Patenterteilung: **09.12.2020**
(21) Anmeldenummer: 16725386.3
(22) Anmeldetag: 10.05.2016
(51) Int. Cl.: C08K 5/31, C07C 279/04, C07C 277/08

(54) **GUANIDINGRUPPEN-HALTIGER KATALYSATOR**
GUANIDINE GROUP CONTAINING CATALYST
CATALYSEUR A BASE DE GUANIDINE

(30) Priorität: 11.05.2015 EP 15167147
(43) Veröffentlichungstag der Anmeldung: 21.03.2018
(73) Patentinhaber: Sika Technology AG, 6340 Baar (CH)
(72) Erfinder: CANNAS, Rita, 8600 Dübendorf (CH); BURCKHARDT, Urs, 8049 Zürich (CH)
(74) Vertreter: Sika Patent Attorneys
(86) Internationale Anmeldenummer: PCT/EP2016/060478
(87) Internationale Veröffentlichungsnummer: WO 2016/180840

(56) Entgegenhaltungen:
- WO-A1-2015/026687
- US-A1- 2013 190 469

## Beschreibung

### Technisches Gebiet

Die Erfindung betrifft Katalysatoren für härtbare Zusammensetzungen, insbesondere für Silangruppen-haltige Zusammensetzungen.

### Stand der Technik

Härtbare Zusammensetzungen spielen eine bedeutende Rolle in vielen technischen Anwendungen, beispielsweise als Klebstoffe, Dichtstoffe oder Beschichtungen. Ihre Aushärtung wird durch Vernetzungsreaktionen bewirkt, welche über freie oder latente Reaktivgruppen wie beispielsweise Isocyanatgruppen, Epoxidgruppen, Hydroxylgruppen, Aminogruppen oder Silangruppen ablaufen, wobei diese nach einem Mischvorgang, durch Erwärmen oder durch Kontakt mit Feuchtigkeit mit sich selbst oder untereinander reagieren und so die in der Zusammensetzung enthaltenen Aufbaukomponenten kovalent zu einem polymeren Netzwerk verbinden. Zur Beschleunigung solcher Vernetzungsreaktionen werden häufig Katalysatoren eingesetzt. Sehr oft handelt es sich dabei um toxikologisch bedenkliche Stoffe, welche eine potentielle Gefährdung von Verarbeiter und Umwelt darstellen, insbesondere nach der Aushärtung der Zusammensetzung, wenn der Katalysator oder dessen Abbauprodukte durch Ausgasen, Migration oder Auswaschen freigesetzt werden.

Bei Raumtemperatur härtbare Zusammensetzungen auf Basis von Silangruppen-haltigen Polymeren sind ausgeprägt mit dieser Problemstellung konfrontiert. Silangruppen-haltige Polymere sind dabei insbesondere Polyorganosiloxane, welche gemeinhin als "Silicone" bzw. "Siliconkautschuke" bezeichnet werden, und Silangruppen-haltige organische Polymere, welche auch als "silanfunktionelle Polymere", "silanmodifizierte Polymere" (SMP) oder "silanterminierte Polymere" (STP) bezeichnet werden. Ihre Vernetzung verläuft über die Kondensation von Silanolgruppen unter Ausbildung von Siloxanbindungen und wird klassischerweise mittels Organozinn-Verbindungen, wie insbesondere Di-alkylzinn(IV)-carboxylaten, katalysiert. Diese zeichnen sich durch eine sehr hohe Aktivität in Bezug auf die Silanol-Kondensation aus und sind sehr hydrolysebeständig; allerdings sind sie gesundheitsschädlich und stark wassergefährdend. Oft werden sie mit weiteren Katalysatoren kombiniert, hauptsächlich mit basischen Verbindungen wie insbesondere Aminen, welche vor allem die vorgeschaltete Hydrolyse der Silangruppen beschleunigen.

Aufgrund einer stärkeren Gewichtung von EHS-Aspekten durch Berufsverbände und Verbraucher sowie schärferer staatlicher Regulierung werden seit einiger Zeit vermehrt Anstrengungen unternommen, die Organozinn-Verbindungen durch andere, weniger toxische Katalysatoren zu ersetzen. So wurden etwa Organotitanate, -zirkonate und -aluminate als alternative Metall-Katalysatoren beschrieben. Diese haben aber zumeist eine geringere katalytische Aktivität in Bezug auf die Silanol-Kondensation und bewirken eine deutlich langsamere Vernetzung. Wegen ihrer mangelnden Hydrolysestabilität können sie beim Lagern der Zusammensetzung durch Restfeuchte der Inhaltsstoffe einen Grossteil ihrer Aktivität verlieren, wodurch sich die Aushärtung stark verlangsamt oder ganz zum Erliegen kommt.

Eine weitere bekannte Alternative zu Organozinn-Verbindungen stellen stark basische Stickstoff-Verbindungen aus der Klasse der Amidine und Guanidine dar, welche in Kombination mit den erwähnten Metall-Katalysatoren oder auch allein eingesetzt werden können. Viele der gebräuchlichen Amidin- und Guanidin-Katalysatoren, wie insbesondere 1,8-Diazabicyclo[5.4.0]undec-7-en (DBU) und 1,1,3,3-Tetramethylguanidin (TMG), sind allerdings leichtflüchtige und geruchsintensive sowie ebenfalls gesundheitsschädliche und umweltgefährdende Stoffe. Ausserdem neigen sie dazu, aufgrund geringer Verträglichkeit in der Zusammensetzung zu migrieren und dadurch Separation, Ausschwitzen oder Substratverschmutzung zu verursachen. Die beschriebene Verwendung von aromatischen, bei Raumtemperatur festen Amidinen und Guanidinen schafft hier Abhilfe, erfordert aber den Einsatz von geeigneten Lösemitteln und bringt Einbussen bei der katalytischen Aktivität und damit der Vernetzungsgeschwindigkeit.

WO2015026687 betrifft eine härtbare Zusammensetzung auf Basis von Silangruppen-haltigen Polymeren, enthaltend ein Silangruppen-haltiges Polymer, einen Vernetzer oder Kettenverlängerer, sowie eine Guanidinverbindung als Kondensationsbeschleuniger. Die Guanidinverbindung wird durch die Umsetzung von einem Diisopropylcarbodiimid mit einem Diamin hergestellt.

### Darstellung der Erfindung

Aufgabe der vorliegenden Erfindung ist es daher, einen Katalysator für die Vernetzung von härtbaren Zusammensetzungen, insbesondere Silangruppen-haltigen Zusammensetzungen, bereitzustellen, welcher eine hohe katalytische Aktivität für die Vernetzungsreaktion besitzt und damit eine rasche Aushärtung der applizierten Zusammensetzung ermöglicht, sowie eine hohe Selektivität für diese Vernetzungsreaktion aufweist und somit die Lagerstabilität der Zusammensetzung nicht über Gebühr beeinträchtigt. Weiterhin soll der Katalysator einen geringen Dampfdruck und eine hohe Verträglichkeit mit der Zusammensetzung aufweisen, so dass er weder zu Separierung oder Migration noch zum Verdampfen neigt, und soll möglichst geruchlos und wenig toxisch sein.

Diese Aufgabe wird durch die Verwendung eines Katalysators der Formel (I) nach Anspruch 1 gelöst. Der Katalysator der Formel (I) enthält eine aliphatische Guanidin-Gruppe und zeigt eine hohe katalytische Aktivität, während aromatische Guanidine kaum oder gar nicht katalytisch aktiv sind. Im Gegensatz zu vielen aus dem Stand der Technik bekannten aliphatische Amidin- oder Guanidin-Gruppen aufweisenden Katalysatoren ist der Katalysator der Formel (I) bei Raumtemperatur weitgehend geruchlos und nicht flüchtig. Er zeigt eine hohe kalatytische Aktivität bei guter Selektivität, insbesondere in Zusammensetzungen auf Basis von Silangruppen-haltigen Polymeren. Dies ist besonders überraschend, würde man doch aufgrund seines relativ hohen Molekulargewichtes und der starken intermolekularen Wechselwirkungen über Wasserstoffbrücken eine verminderte Aktivität im Vergleich mit kleineren, unpolareren und somit mobileren Guanidinen erwarten.

Mit diesen Eigenschaften ist der Katalysator der Formel (I) besonders geeignet für die Verwendung in Zusammensetzungen auf Basis von Silangruppen-haltigen Polymeren, wo er als alleiniger Katalysator oder in Kombination mit weiteren Katalysatoren eine schnelle Aushärtung zu einem mechanisch hochwertigen und beständigen Material ermöglicht, ohne die Lagerfähigkeit der unausgehärteten Zusammensetzung zu beeinträchtigen. Er ist sowohl vor als auch nach der Aushärtung ausgezeichnet verträglich mit der Zusammensetzung und neigt weder zu Separierung noch zu Migration, im Gegensatz zu vielen ähnlichen Zusammensetzungen mit Amidin- oder Guanidin-Katalysatoren nach dem Stand der Technik, wo Katalysator-bedingte Migrationseffekte eine starke Rolle spielen. Er ermöglicht emissions- und geruchsarme Produkte, welche weder schmierige oder klebrige Oberflächen aufweisen noch Substratverschmutzung verursachen. Schliesslich ist der Katalysator der Formel (I) in einem überraschend einfachen Verfahren ohne Hilfsstoffe aus handelsüblichen, preisgünstigen Ausgangsmaterialien herstellbar.

Weitere Aspekte der Erfindung sind Gegenstand weiterer unabhängiger Ansprüche. Besonders bevorzugte Ausführungsformen der Erfindung sind Gegenstand der abhängigen Ansprüche.

### Wege zur Ausführung der Erfindung

Gegenstand der Erfindung ist die Verwendung eines Katalysators der Formel (I), 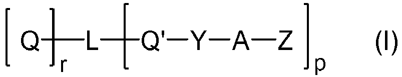 wobei
p für eine ganze Zahl von 1 bis 6 steht und r für eine ganze Zahl von 0 bis 5 steht, wobei (p+r) für eine ganze Zahl von 1 bis 6 steht,
L für
   einen (p+r)-wertigen Kohlenwasserstoffrest mit einem mittleren Molekulargewicht im Bereich von 14 bis 20'000 g/mol, welcher gegebenenfalls Heteroatome, insbesondere Sauerstoff oder Stickstoff oder Silicium in Form von Ether-, tertiären Amino-, Ester-, Amid-, Urethan-, Harnstoff-, Uretdion-, Isocyanurat-, Biuret-, Allophanat-, Uretonimin-, Iminooxadiazindion-, Oxadiazintrion- oder Alkoxysilan-Gruppen, aufweist,
   oder für einen (p+r+1)-wertigen Kohlenwasserstoffrest mit 4 bis 12 C-Atomen, welcher zusammen mit Q' einen gegebenenfalls substituierten 5- oder 6-gliedrigen Ring bildet,
   oder für eine kovalente Bindung,
   oder für einen Wasserstoff-Rest steht,
Q für eine Reaktivgruppe ausgewählt aus Epoxid, Aziridin, Carbonat, Carbonsäureanhydrid, Carbonsäure, Carbonsäureester, Lacton, Carbonsäurechlorid, Keton, Aldehyd, 1,3-Diketon, 1,3-Ketoester, 1,3-Ketoamid, Cyanat, Thiocyanat, Isocyanat, Isothiocyanat, (Meth)acrylat, (Meth)acrylamid, (Meth)acrylnitril, Maleat, Maleamid, Maleimid, Fumarat, Fumaramid, Itaconat, Itaconamid, Crotonat und Crotonamid steht,
Q' für eine zwei- oder dreiwertige Verbindungseinheit entstanden aus der Reaktion von Q mit HX steht,
Y für N oder X steht, wobei X für O oder S oder NR³ steht, wobei R³ für einen Wasserstoff-Rest oder für einen Alkyl- oder Cycloalkyl- oder Aralkyl-Rest mit 1 bis 8 C-Atomen, welcher gegebenenfalls eine tertiäre Aminogruppe oder eine Guanidin-Gruppe enthält, steht,
A für einen zweiwertigen Kohlenwasserstoff-Rest mit 2 bis 30 C-Atomen, welcher gegebenenfalls ungesättigte Anteile und gegebenenfalls Ether-Sauerstoff oder sekundären oder tertiären Amin-Stickstoff enthält, steht,
wobei A auch zusammen mit R³ für einen dreiwertigen Kohlenwasserstoff-Rest mit 5 bis 10 C-Atomen, welcher gegebenenfalls einen tertiären Amin-Stickstoff enthält, stehen kann, und
Z für eine über ein Stickstoffatom gebundene Guanidingruppe steht, welche kein Stickstoffatom enthält, welches direkt an einen aromatischen Ring gebunden oder Teil eines heteroaromatischen Ringsystems, wie zum Beispiel Imidazol oder Pyrimidin, ist,
für die Vernetzung einer härtbaren Zusammensetzung.

Im vorliegenden Dokument bezeichnet der Begriff "Silangruppe" eine an einen organischen Rest oder an einen Polyorganosiloxan-Rest gebundene Silylgruppe mit einer bis drei, insbesondere zwei oder drei, hydrolysierbaren Substituenten am Silicium-Atom. Besonders gebräuchliche hydrolysierbare Substituenten sind Alkoxy-Reste. Diese Silangruppen werden auch als "Alkoxysilangruppen" bezeichnet. Silangruppen können auch in partiell oder vollständig hydrolysierter Form vorhanden sein.
Als "Hydroxysilan", "Isocyanatosilan", "Aminosilan" bzw. "Mercaptosilan" werden Organoalkoxysilane bezeichnet, die am organischen Rest zusätzlich zur Silangruppe eine oder mehrere Hydroxyl-, Isocyanato-, Amino- bzw. Mercaptogruppen aufweisen.
Als "primäre Aminogruppe" bzw. "primärer Amin-Stickstoff" wird eine NH₂-Gruppe bzw. deren Stickstoffatom bezeichnet, die bzw. das an einen organischen Rest gebunden ist, und als "sekundäre Aminogruppe" bzw. "sekundärer Amin-Stickstoff" wird eine NH-Gruppe bzw. deren Stickstoffatom bezeichnet, die bzw. das an zwei organische Reste, welche auch gemeinsam Teil eines Rings sein können, gebunden ist, und als "tertiäre Aminogruppe" bzw. "tertiärer Amin-Stickstoff" wird eine N-Gruppe bzw. deren Stickstoffatom bezeichnet, die bzw. das an drei organische Reste, welche auch zu zweit oder zu dritt Teil eines oder mehrerer Ringe sein können, gebunden ist.
Mit "Poly" beginnende Substanznamen wie Polyol oder Polyisocyanat bezeichnen Substanzen, die formal zwei oder mehr der in ihrem Namen vorkommenden funktionellen Gruppen pro Molekül enthalten.
Der Begriff "organisches Polymer" umfasst ein Kollektiv von chemisch einheitlichen, sich aber in Bezug auf Polymerisationsgrad, Molmasse und Kettenlänge unterscheidenden Makromolekülen, das durch eine Polyreaktion (Polymerisation, Polyaddition, Polykondensation) hergestellt wurde und im PolymerRückgrat mehrheitlich Kohlenstoffatome aufweist, sowie Umsetzungsprodukte eines solchen Kollektivs von Makromolekülen. Polymere mit einem Polyorganosiloxan-Rückgrat (gemeinhin als "Silicone" bezeichnet) stellen keine organischen Polymere im Sinne des vorliegenden Dokuments dar.
Der Begriff "Silangruppen-haltiger Polyether" umfasst auch Silangruppen-haltige organische Polymere, welche zusätzlich zu Polyether-Einheiten auch Urethangruppen, Harnstoffgruppen oder Thiourethangruppen enthalten können. Solche Silangruppen-haltige Polyether können auch als "Silangruppen-haltige Polyurethane" bezeichnet werden.
Unter "Molekulargewicht" versteht man im vorliegenden Dokument die molare Masse (in Gramm pro Mol) eines Moleküls oder eines Teils eines Moleküls, auch als "Rest" bezeichnet. Als "mittleres Molekulargewicht" wird das Zahlenmittel Mₙ einer oligomeren oder polymeren Mischung von Molekülen oder Resten bezeichnet, welches üblicherweise mittels Gelpermeationschromatographie (GPC) gegen Polystyrol als Standard bestimmt wird.
Als "lagerstabil" oder "lagerfähig" wird eine Substanz oder eine Zusammensetzung bezeichnet, wenn sie bei Raumtemperatur in einem geeigneten Gebinde während längerer Zeit, typischerweise mindestens 3 Monaten bis zu 6 Monaten und mehr, aufbewahrt werden kann, ohne dass sie sich in ihren Anwendungs- oder Gebrauchseigenschaften, insbesondere der Viskosität und der Vernetzungsgeschwindigkeit, durch die Lagerung in einem für ihren Gebrauch relevanten Ausmass verändert.
Eine gestrichelte Linie in den Formeln in diesem Dokument stellt jeweils die Bindung zwischen einem Substituenten und dem zugehörigen Molekülrest dar. Als "Raumtemperatur" wird eine Temperatur von ca. 23 °C bezeichnet.

Für den Fall, dass L für eine kovalente Bindung steht, weist der Katalysator der Formel (I) entweder die Formel Q-Q'-Y-A-Z oder die Formel Z-A-Y-Q'-Q'-Y-A-Z auf.

Bevorzugt weist A 2 bis 20 C-Atome auf.
Besonders bevorzugt ist A entweder ausgewählt aus der Gruppe bestehend aus 1,2-Ethylen, 1,2-Propylen, 1,3-Propylen, 1,3-Pentylen, 1,1-Dimethyl-1,2-ethyl, 1,5-Pentylen, 2-Methyl-1,5-pentylen, 1,6-Hexylen, 2,2(4),4-Trimethyl-1,6-hexamethylen, 1,8-Octylen, 1,10-Decylen, 1,12-Dodecylen, (1,5,5-Trimethylcyclohexan-1-yl)methan-1,3, 1,3-Cyclohexylen-bis(methylen), 1,4-Cyclohexylen-bis(methylen), 1,3-Phenylen-bis(methylen), 2- und/oder 4-Methyl-1,3-cyclohexylen, N-Methyl-4-aza-1,7-heptylen, 3-Oxa-1,5-pentylen, 3,6-Dioxa-1,8-octylen, 4,7-Dioxa-1,10-decylen und einem Polyoxypropylen-Rest mit einem mittleren Molekulargewicht im Bereich von etwa 200 bis 250 g/mol, oder A steht zusammen mit R³ einschliesslich des Stickstoffatoms von X für einen Rest ausgewählt aus der Gruppe bestehend aus Piperidin-4-ylmethyl, 2-(Piperidin-4-yl)ethyl und 2-Piperazinoethyl.

Für den Fall, dass A zusammen mit R³ einschliesslich des Stickstoffatoms von X für Piperidin-4-ylmethyl bzw. 2-(Piperidin-4-yl)ethyl bzw. 2-Piperazinoethyl steht, weist der Katalysator der Formel (I) die Formel 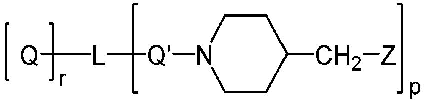 bzw. 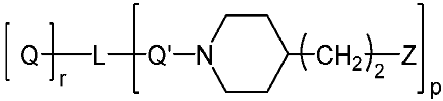 bzw. 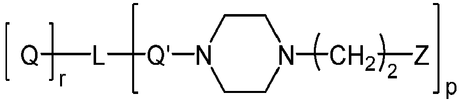 auf.

Y steht bevorzugt für N oder O oder NR³.
R³ steht bevorzugt für einen Wasserstoff-Rest.
R³ steht weiterhin bevorzugt für einen Alkyl- oder Cycloalkyl-Rest mit 1 bis 8 C-Atomen.
R³ steht weiterhin bevorzugt für einen Rest der Formel ---R⁷-Z, wobei R⁷ für einen gegebenenfalls verzweigten Alkylen-Rest mit 2 bis 6 C-Atomen, insbesondere für 1,2-Ethylen, 1,3-Propylen oder 1,6-Hexylen, steht und Z die genannten Bedeutungen aufweist.
R³ steht weiterhin bevorzugt für einen N,N-Dimethylaminopropyl-Rest.
R³ steht weiterhin bevorzugt zusammen mit A und unter Einbezug des Stickstoffatoms von X für Piperidin-4-ylmethyl oder 2-(Piperidin-4-yl)ethyl oder 2-Piperazinoethyl.

Z steht bevorzugt für 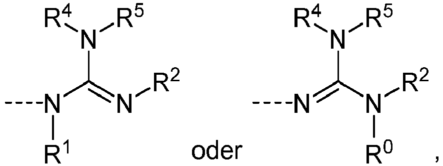 wobei
R⁰ und R¹ unabhängig voneinander für einen Wasserstoff-Rest oder für einen Alkyl- oder Cycloalkyl- oder Aralkyl-Rest mit 1 bis 8 C-Atomen stehen,
R² für einen Wasserstoff-Rest oder für einen Alkyl-, Cycloalkyl- oder Aralkyl-Rest mit 1 bis 18 C-Atomen, welcher gegebenenfalls Ether-Sauerstoff oder tertiären Amin-Stickstoff enthält, steht,
R⁴ und R⁵ unabhängig voneinander jeweils für einen Wasserstoff-Rest oder für einen Alkyl-, Cycloalkyl- oder Aralkyl-Rest mit 1 bis 18 C-Atomen, welcher gegebenenfalls einen Ether-Sauerstoff oder einen tertiären Amin-Stickstoff enthält, stehen,
   wobei
R² und R⁰ auch zusammen für einen Alkylen-Rest mit 3 bis 6 C-Atomen, welcher gegebenenfalls einen Ether-Sauerstoff oder einen tertiären Amin-Stickstoff enthält, stehen können,
R⁴ und R⁵ auch zusammen für einen Alkylen-Rest mit 4 bis 7 C-Atomen, welcher gegebenenfalls einen Ether-Sauerstoff oder einen tertiären Amin-Stickstoff enthält, stehen können, und
R² und R⁵ auch zusammen für einen Alkylen-Rest mit 2 bis 12 C-Atomen stehen können.
R¹ und R⁰ stehen bevorzugt unabhängig voneinander jeweils für einen Wasserstoff-Rest oder für einen Alkyl-Rest mit 1 bis 4 C-Atomen, insbesondere für einen Wasserstoff-Rest.
R² steht bevorzugt für einen Wasserstoff-Rest oder für einen Alkyl-, Cycloalkyl- oder Aralkyl-Rest mit 1 bis 12 C-Atomen, insbesondere 1 bis 8 C-Atomen, welcher gegebenenfalls Ether-Sauerstoff oder tertiären Amin-Stickstoff enthält. R⁴ und R⁵ stehen bevorzugt unabhängig voneinander jeweils für einen Wasserstoff-Rest oder für einen Alkyl-, Cycloalkyl- oder Aralkyl-Rest mit 1 bis 12 C-Atomen, welcher gegebenenfalls ein Sauerstoffatom oder ein Stickstoffatom enthält.
Weiterhin bevorzugt stehen R⁴ und R⁵ zusammen für einen Alkylen-Rest mit 4 bis 7 C-Atomen, welcher gegebenenfalls ein Sauerstoffatom oder ein Stickstoffatom enthält.

R¹ und R⁰ stehen bevorzugt unabhängig voneinander jeweils für einen Wasserstoff-Rest oder für einen Alkyl-Rest mit 1 bis 4 C-Atomen, insbesondere für einen Wasserstoff-Rest.
R² steht bevorzugt für einen Wasserstoff-Rest oder für einen Alkyl-, Cycloalkyl- oder Aralkyl-Rest mit 1 bis 12 C-Atomen, insbesondere 1 bis 8 C-Atomen, welcher gegebenenfalls Ether-Sauerstoff oder tertiären Amin-Stickstoff enthält. R⁴ und R⁵ stehen bevorzugt unabhängig voneinander jeweils für einen Wasserstoff-Rest oder für einen Alkyl-, Cycloalkyl- oder Aralkyl-Rest mit 1 bis 12 C-Atomen, welcher gegebenenfalls ein Sauerstoffatom oder ein Stickstoffatom enthält.

Weiterhin bevorzugt stehen R⁴ und R⁵ zusammen für einen Alkylen-Rest mit 4 bis 7 C-Atomen, welcher gegebenenfalls ein Sauerstoffatom oder ein Stickstoffatom enthält.

R⁴ steht besonders bevorzugt für einen Wasserstoff-Rest.
R⁵ steht besonders bevorzugt für einen Alkyl-, Cycloalkyl- oder Aralkyl-Rest mit 1 bis 12 C-Atomen, insbesondere 1 bis 8 C-Atomen, welcher gegebenenfalls ein Ether-Sauerstoff oder tertiären Amin-Stickstoff enthält.

Besonders bevorzugt steht Z für 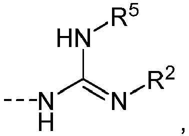 wobei R² und R⁵ die bereits genannten Bedeutungen aufweisen.
Besonders bvorzugt stehen R² und R⁵ unabhängig voneinander jeweils für Ethyl, Isopropyl, tert.Butyl, 3-(Dimethylamino)propyl oder Cyclohexyl, insbesondere für Isopropyl oder Cyclohexyl.
Ein solcher Katalysator der Formel (I) weist als R³ bevorzugt einen Wasserstoff-Rest oder einen Alkyl- oder Cycloalkyl-Rest mit 1 bis 8 C-Atomen oder einen Rest der Formel 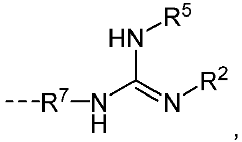 wobei R⁷, R² und R⁵ die bereits genannten Bedeutungen aufweisen, oder einen N,N-Dimethylaminopropyl-Rest auf.

Q steht bevorzugt für Epoxid, Carbonat, Carbonsäureanhydrid, Carbonsäureester, Lacton, Keton, Aldehyd, 1,3-Diketon, 1,3-Ketoester, 1,3-Ketoamid, Cyanat, Isocyanat, Acrylat, Methacrylat, Acrylamid, Methacrylamid, Maleat, Maleimid, Fumarat oder Itaconat.
Q steht besonders bevorzugt für Epoxid, Carbonat, Carbonsäureester, Lacton, Keton, Aldehyd, 1,3-Diketon, 1,3-Ketoester, Isocyanat, Acrylat oder Methacrylat.
Die Epoxid-Gruppe liegt bevorzugt als Glycidyl-Gruppe vor, insbesondere als Glycidyloxy-Gruppe.

Die Aziridin-Gruppe stellt bevorzugt eine N-Aziridinyl-Gruppe dar.

Q' ist bevorzugt ausgewählt aus der Gruppe bestehend aus 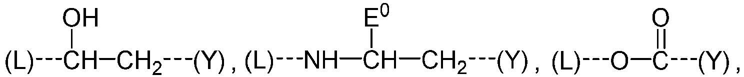 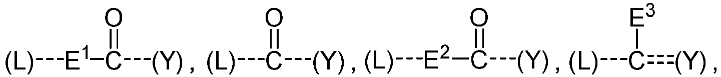 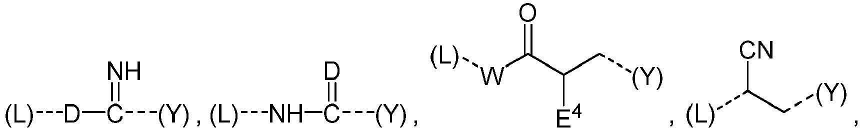 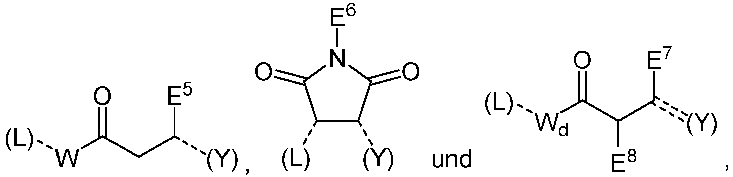 wobei
D für O oder S steht,
W für O oder NR⁶ steht, wobei R⁶ für einen Wasserstoff-Rest oder für einen einwertigen Kohlenwasserstoffrest mit 1 bis 8 C-Atomen steht,
E⁰ für einen Wasserstoff-Rest oder für einen Methyl-Rest steht,
E¹ für einen Carboxyl-substituierten Alkylen-, Alkendiyl- oder Phenylen-Rest mit 2 bis 8 C-Atomen steht,
E² für einen Hydroxyalkylen-Rest mit 2 bis 5 C-Atomen oder für einen über O gebundenen Hydroxyalkylenoxy-Rest mit 2 oder 3 C-Atomen steht,
E³ für einen Wasserstoff-Rest oder für einen einwertigen Kohlenwasserstoff-rest mit 1 bis 6 C-Atomen, welcher gegebenenfalls Heteroatome in Form von Ether-, Ester-, Amino- oder Amid-Gruppen aufweist, oder zusammen mit L für einen gegebenenfalls substituierten 1,4-Butylen- oder 1,5-Pentylen-Rest steht, E⁴ für einen Wasserstoff- oder Methyl- oder Alkoxycarbonylmethyl-Rest mit 2 bis 9 C-Atomen steht,
E⁵ für einen Alkoxycarbonyl-Rest mit 1 bis 8 C-Atomen oder für einen Methyl-Rest steht,
E⁶ für einen Wasserstoff-Rest oder für einen Alkyl-Rest mit 1 bis 8 C-Atomen steht,
E⁷ für einen einwertigen Kohlenwasserstoffrest mit 1 bis 6 C-Atomen steht,
E⁸ für einen Wasserstoff-Rest oder für einen einwertigen Kohlenwasserstoff-rest mit 1 bis 6 C-Atomen steht, und
d für 0 oder 1 steht.

Die in Klammern stehenden Buchstaben (L) und (Y) stellen dabei jeweils die Anbindung von Q' an L und Y dar.

Bevorzugt steht D für O.
Bevorzugt steht W für O.
Bevorzugt steht E¹ für 2-Carboxy-1,2-ethylen, 2-Carboxy-1,2-ethendiyl, 3-Carboxy-1,3-propylen, 2-Carboxy-1,2-cyclohexylen oder 2-Carboxy-1,2-phenylen.
Bevorzugt steht E² für 1-Hydroxy-1,2-ethylen, 1-Hydroxy-1,3-propylen, 1-Hydroxy-1,4-butylen, 1-Hydroxy-1,5-pentylen, 2-Hydroxy-1,2-ethylenoxy oder 3-Hydroxy-1,3-propylenoxy.
Bevorzugt steht E³ für einen Wasserstoff-Rest oder für einen Methyl-Rest. Bevorzugt steht E⁴ für einen Wasserstoff-Rest oder für Methyl, Methoxycarbonylmethyl, Ethoxycarbonylmethyl oder Butoxycarbonylmethyl, insbesondere für einen Wasserstoff-Rest oder für einen Methyl-Rest.
Bevorzugt steht E⁵ für Methoxycarbonyl, Ethoxycarbonyl oder Butoxycarbonyl oder für Methyl.
Bevorzugt steht E⁶ für einen Wasserstoff-Rest oder Methyl, Ethyl oder Butyl. Bevorzugt steht E⁷ für Methyl.
Bevorzugt steht E⁸ für einen Wasserstoff-Rest.

Bevorzugt steht p für 1 oder 2 oder 3.
Bevorzugt steht r für 0.
Bevorzugt steht (p+r) für 1 oder 2 oder 3.
Besonders bevorzugt stehen p für 1 oder 2 oder 3 und r für 0.

Die bevorzugten Katalysatoren der Formel (I) sind aus gut zugänglichen Ausgangsstoffen in einem einfachen Verfahren herstellbar.

Der Katalysator der Formel (I) kann auch in tautomerer Form vorliegen. Alle möglichen Tautomerformen dieser Katalysatoren werden im Rahmen der vorliegenden Erfindung als gleichwertig angesehen.
Weiterhin kann der Katalysator der Formel (I) in protonierter Form vorliegen. Ebenfalls kann der Katalysator der Formel (I) in komplexierter Form vorliegen, insbesondere mit Kationen von Zink, Eisen oder Molybdän.

In einer bevorzugten Ausführungsform der Erfindung steht Q für eine Epoxid-Gruppe. In diesem Fall steht Y bevorzugt für S oder NR³, insbesondere für NR³, Q' steht für eine Verbindungseinheit der Formel 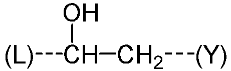 und L steht bevorzugt für einen (p+r)-wertigen Kohlenwasserstoffrest mit einem Molekulargewicht im Bereich von 15 bis 1'500 g/mol, welcher insbesondere (p+r) Ether-Sauerstoffe und gegebenenfalls eine Alkoxysilangruppe enthält. Insbesondere steht L dabei für einen Rest ausgewählt aus der Gruppe bestehend aus 2-Ethylhexylglycidylether, C₈- bis C₁₀-Alkylglycidylether, C₁₂- bis C₁₄-Alkylglycidylether, Kresylglycidylether, tert.Butylphenylglycidylether, Cardanol-Glycidylether, 1,4-Butandioldiglycidylether, 1,6-Hexandioldiglycidylether, Neopentylglykoldiglycidylether, Polypropylenglykoldiglycidylether mit einem mittleren Molekulargewicht im Bereich von 280 bis 1'500 g/mol, Bisphenol-A-Digylcidylether, Bisphenol-F-Digylcidylether, 3-Glycidoxypropyltrimethoxysilan und 3-Glycidoxypropyltriethoxysilan jeweils nach Entfernung der Epoxidgruppen.

In einer weiteren bevorzugten Ausführungsform der Erfindung steht Q für eine cyclische Carbonat-Gruppe. In diesem Fall stehen insbesondere r für 0 und p für 1, Y steht für X und Q' steht für eine Verbindungseinheit der Formel 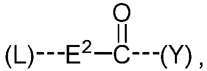 bei welcher E² für einen über O gebundenen Hydroxyalkylenoxy-Rest mit 2 oder 3 C-Atomen steht, und L steht insbesondere für einen Wasserstoff-Rest oder für einen linearen Alkylrest mit 1 bis 12 C-Atomen.

Besonders bevorzugt stehen dabei E² für 1-Hydroxy-1,2-ethylenoxy und L für einen Methyl-Rest.

In einer weiteren bevorzugten Ausführungsform der Erfindung steht Q für eine Carbonsäureester-Gruppe. In diesem Fall steht Y steht für X, Q' steht für eine Verbindungseinheit der Formel 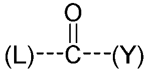 und L steht bevorzugt entweder für einen Wasserstoff-Rest oder für eine kovalente Bindung oder für einen (p+r)-wertigen Kohlenwasserstoffrest mit einem mittleren Molekulargewicht im Bereich von 14 bis 500 g/mol, welcher gegebenenfalls ungesättigte Anteile und gegebenenfalls Ether-Sauerstoff aufweist.
Insbesondere stehen dabei entweder r für 0, p für 1 und L für einen Rest ausgewählt aus 1-Pentyl, 3-Heptyl, 1-Undecyl und Phenyl, oder (p+r) steht für 2 und L steht für einen Rest ausgewählt aus 1,4-Butylen, 1,2-Phenylen, 1,3-Phenylen und 1,4-Phenylen.

In einer weiteren bevorzugten Ausführungsform der Erfindung steht Q für eine Lacton-Gruppe. In diesem Fall stehen insbesondere r für 0 und p für 1, Y steht für X und Q' steht für eine Verbindungseinheit der Formel 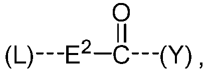 bei welcher E² für einen Hydroxyalkylen-Rest mit 2 bis 5 C-Atomen steht, und L steht insbesondere für einen Wasserstoff-Rest oder für einen linearen Alkyl-rest mit 1 bis 12 C-Atomen.
Insbesondere stehen dabei E² und L zusammen für 3-Hydroxypropyl, 3-Hydroxy-3-methylpropyl, 4-Hydroxybutyl, 4-Hydroxy-4-methylbutyl oder 5-Hydroxypentyl.

In einer weiteren bevorzugten Ausführungsform der Erfindung steht Q für eine Keton-Gruppe. In diesem Fall stehen insbesondere r für 0, p für 1, Y für N und Q' für eine Verbindungseinheit der Formel 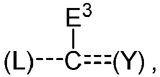 bei welcher entweder E³ für einen einwertigen Kohlenwasserstoffrest mit 1 bis 6 C-Atomen und L für einen einwertigen Kohlenwasserstoff-Rest mit 1 bis 20 C-Atome stehen, oder E³ und L zusammen für einen 1,4-Butylen- oder 1,5-Pentylen-Rest stehen. Insbesondere steht E³ für einen Methyl-Rest und L ist ausgewählt aus Methyl, Ethyl, Isopropyl, Isobutyl und Phenyl, oder E³ und L stehen zusammen für 1,4-Butylen oder 1,5-Pentylen.

In einer weiteren bevorzugten Ausführungsform der Erfindung steht Q für eine Aldehyd-Gruppe. In diesem Fall stehen insbesondere r für 0, p für 1, Y für N und Q' für eine Verbindungseinheit der Formel 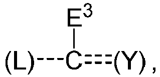 bei welcher E³ für einen Wasserstoff-Rest und L für einen einwertigen Kohlenwasserstoff-Rest mit 1 bis 20 C-Atomen, welcher gegebenenfalls Sauerstoff oder Stickstoff in Form von Estergruppen, Amidgruppen, Ethergruppen oder tertiären Aminogruppen enthält, stehen.
Insbesondere steht E³ für einen Rest ausgewählt aus 2-Propyl, 3-Heptyl, 1-Undecyl, Phenyl, 3-Acetyloxy-2-methylprop-2-yl, 3-Lauroyloxy-2-methylprop-2-yl und 3-(N-Morpholino)-2-methylprop-2-yl.

In einer weiteren bevorzugten Ausführungsform der Erfindung steht Q für eine 1,3-Diketon-Gruppe. In diesem Fall stehen insbesondere r für 0, p für 1, Y für N und Q' für eine Verbindungseinheit der Formel 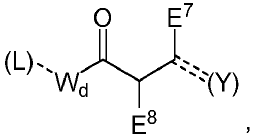 bei welcher d für 0, E⁷ und L jeweils für einen Methyl-Rest und E⁸ für einen Wasserstoff-Rest stehen.

In einer weiteren bevorzugten Ausführungsform der Erfindung steht Q für eine 1,3-Ketoester- oder eine 1,3-Ketoamid-Gruppe. In diesem Fall stehen insbesondere r für 0 und p für 1 oder (p+r) für 2, Y für N und Q' steht für eine Verbindungseinheit der Formel 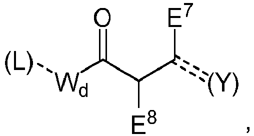 bei welcher d für 1 steht.

Im Fall von r für 0, p für 1 und W für O stehen insbesondere E⁷ für Methyl, E⁸ für Wasserstoff und L für Methyl oder Ethyl oder Isopropyl oder tert.Butyl.
Im Fall von (p+r) für 2 und W für O stehen insbesondere E⁷ für Methyl, E⁸ für Wasserstoff und L ist ausgewählt aus 1,2-Ethylen, 1,2-Propylen, 3-Oxa-1,5-pentylen, 5-Methyl-4-oxa-2,6-hexylen, 1,3-Propylen, 1,4-Butylen, 1,5-Pentylen, 3-Methyl-1,5-pentylen, 1,6-Hexylen und 2,2(4),4-Trimethyl-1,6-hexylen.
Im Fall von r für 0, p für 1 und W für NR⁶ stehen insbesondere E⁷ für Methyl, E⁸ für Wasserstoff und R⁶ und L jeweils für Methyl oder Ethyl oder Butyl oder 2-Ethylhexyl oder 2-Methoxyethyl.

In einer weiteren bevorzugten Ausführungsform der Erfindung steht Q für eine Isocyanat-Gruppe. In diesem Fall steht Y für X und Q' steht für eine Verbindungseinheit der Formel 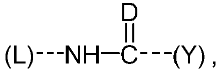 bei welcher D für O steht, und L steht bevorzugt für einen (p+r)-wertigen Kohlenwasserstoffrest mit einem mittleren Molekulargewicht im Bereich von 14 bis 20'000 g/mol, welcher gegebenenfalls Sauerstoff oder Stickstoff oder Silicium in Form von Ether-, Ester-, Urethan-, Harnstoff-, Uretdion-, Isocyanurat-, Biuret-, Allophanat-, Uretonimin-, Iminooxadiazindion-, Oxadiazintrion- oder Alkoxysilan-Gruppen, aufweist. Insbesondere steht L für einen Rest ausgewählt aus Butyl, Hexyl, Lauryl, Stearyl, Cyclohexyl, Phenyl, 3-Trimethoxysilylpropyl, 3-Triethoxysilylpropyl und Isocyanatgruppen-haltigen Polyurethanpolymeren aus der Umsetzung von Polyolen mit Diisocyanaten mit einem mittleren Molekulargewicht im Bereich von 500 bis 20'000 g/mol nach Entfernung von einer oder mehreren Isocyanatgruppen.

In einer weiteren bevorzugten Ausführungsform der Erfindung steht Q für eine Acrylat- oder Methacrylat-Gruppe. In diesem Fall steht Y für X, X für NR³ und Q' steht für eine Verbindungseinheit der Formel 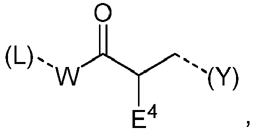 bei welcher W für O und E⁴ für einen Wasserstoff- oder einen Methyl-Rest steht, und L steht bevorzugt für einen (p+r)-wertigen Kohlenwasserstoffrest mit einem mittleren Molekulargewicht im Bereich von 28 bis 20'000 g/mol, welcher gegebenenfalls Sauerstoff oder Stickstoff oder Silicium in Form von Ether-, Ester, Urethan-, Harnstoff-, Uretdion-, Isocyanurat-, Biuret-, Allophanat-, Uretonimin-, Iminooxadiazindion-, Oxadiazintrion- oder Alkoxysilan-Gruppen enthält.
L steht dabei insbesondere für einen Rest ausgewählt aus Butyl, 2-Ethylhexyl, Trimethoxysilylpropyl, Triethoxysilylpropyl, 1,2-Ethylen, 3,6,9-Trioxa-1,11-undecylen, 2,5-Dimethyl-3,6-dioxa-1,8-nonylen, einem Polyoxyethylen-Rest mit einem Molekulargewicht im Bereich von 200 bis 2'000 g/mol, einem Polyoxypropylen-Rest mit einem Molekulargewicht im Bereich von 200 bis 2'000 g/mol, 1,4-Butylen, 1,6-Hexylen, 2,2-Dimethyl-1,3-propylen, Trimethylolpropan nach Entfernung der drei Hydroxylgruppen und (Meth)acrylat-Gruppen aufweisenden Polyurethanpolymeren mit einem mittleren Molekulargewicht im Bereich von 500 bis 20'000 g/mol, insbesondere aus der Umsetzung von Hydroxy-funktionellen (Meth)acrylaten wie insbesondere 2-Hydroxyethylacrylat mit Isocyanatgruppen-haltigen Polyurethanpolymeren.

Ein geeigneter Katalysator der Formel (I) wird insbesondere erhalten durch die Umsetzung von
- mindestens einem Guanidin der Formel (II),

   HX-A-Z (II)

   wobei X, A und Z die genannten Bedeutungen aufweisen,
- mit mindestens einer funktionellen Verbindung, welche mindestens eine Reaktivgruppe ausgewählt aus Epoxid, Aziridin, Carbonat, Carbonsäureanhydrid, Carbonsäure, Carbonsäureester, Lacton, Carbonsäurechlorid, Keton, Aldehyd, 1,3-Diketon, 1,3-Ketoester, 1,3-Ketoamid, Cyanat, Thiocyanat, Isocyanat, Isothiocyanat, (Meth)acrylat, (Meth)acrylamid, (Meth)-acrylnitril, Maleat, Maleamid, Maleimid, Fumarat, Fumaramid, Itaconat, Itaconamid, Crotonat und Crotonamid aufweist.

Das Reaktionsprodukt aus dieser Umsetzung wird bevorzugt ohne Aufarbeitung oder Reinigung als Katalysator für die Vernetzung einer härtbaren Zusammensetzung verwendet. Falls bei der Umsetzung aufgrund von Kondensationsreaktionen Abspalter entstehen, werden diese bevorzugt entfernt, insbesondere durch Destillation, gegebenenfalls unter Vakuum.

Bei der Umsetzung des Guanidins der Formel (II) mit einer funktionellen Verbindung, welche mindestens eine Keton-, Aldehyd-, 1,3-Diketon-, 1,3-Ketoester -oder 1,3-Ketoamid-Gruppe aufweist, weist das Guanidin als HX-Gruppe geeigneterweise eine NH₂-Gruppe auf. Dabei entsteht in einer Kondensationsreaktion unter Freisetzung von Wasser ein Katalysator der Formel (I), bei welchem die Verbindungseinheit Q' dreiwertig ist und Y für N steht.

Ein weiterer Gegenstand der Erfindung ist somit ein Verfahren zur Herstellung des Katalysators der Formel (I), wobei
- mindestens ein Guanidin der Formel (II),

   HX-A-Z (II)

   wobei X, A und Z die genannten Bedeutungen aufweisen,
- mit mindestens einer funktionellen Verbindung, welche mindestens eine Reaktivgruppe ausgewählt aus Epoxid, Aziridin, Carbonat, Carbonsäureanhydrid, Carbonsäure, Carbonsäureester, Lacton, Carbonsäurechlorid, Keton, Aldehyd, 1,3-Diketon, 1,3-Ketoester, 1,3-Ketoamid, Cyanat, Thiocyanat, Isocyanat, Isothiocyanat, (Meth)acrylat, (Meth)acrylamid, (Meth)-acrylnitril, Maleat, Maleamid, Maleimid, Fumarat, Fumaramid, Itaconat, Itaconamid, Crotonat und Crotonamid aufweist,
umgesetzt wird.

Als Reaktivgruppe bevorzugt ist Epoxid, Carbonat, Carbonsäureanhydrid, Carbonsäureester, Lacton, Keton, Aldehyd, 1,3-Diketon, 1,3-Ketoester, 1,3-Ketoamid, Cyanat, Isocyanat, Acrylat, Methacrylat, Acrylamid, Methacrylamid, Maleat, Maleimid, Fumarat oder Itaconat.
Besonders bevorzugt ist Epoxid, Carbonat, Carbonsäureester, Lacton, Keton, Aldehyd, 1,3-Diketon, 1,3-Ketoester, Isocyanat, Acrylat oder Methacrylat.

Verbindungen mit den bevorzugten Reaktivgruppen sind besonders einfach zugänglich und ermöglichen in einem besonders einfachen Verfahren stabile Umsetzungsprodukte mit einer hohen katalytischen Aktivität.

Als Epoxidgruppe bevorzugt ist eine Glycidyl-Gruppe, insbesondere eine Glycidyloxy-Gruppe. Eine solche funktionelle Verbindung lässt sich besonders einfach mit einem Guanidin der Formel (II) umsetzen.
Als Aziridingruppe bevorzugt ist eine N-Aziridinyl-Gruppe. Eine solche funktionelle Verbindung ist einfach verfügbar und lässt sich besonders gut mit einem Guanidin der Formel (II) umsetzen.

Die funktionelle Verbindung weist bevorzugt eine bis sechs der genannten Reaktivgruppen auf, besonders bevorzugt eine oder zwei oder drei der genannten Reaktivgruppen.
Weist die funktionelle Verbindung mehr als eine Reaktivgruppe auf, sind diese bevorzugt gleich, wie beispielsweise in Diepoxiden oder Triacrylaten. Es ist aber auch möglich, dass die funktionelle Verbindung verschiedene Reaktivgruppen aufweist.

Als funktionelle Verbindung geeignet sind insbesondere im Handel erhältliche Substanzen.

Als funktionelle Verbindung mit mindestens einer Epoxidgruppe geeignet sind insbesondere
- aliphatische Monoepoxide, bevorzugt Propylenoxid, Butylenoxid, Hexylenoxid, Allylglycidylether, Butylglycidylether, Hexylglycidylether, 2-Ethylhexylglycidylether, sowie Glycidylether von Fettalkoholen, wie insbesondere C₈-bis C₁₀-Alkylglycidylether oder C₁₂- bis C₁₄-Alkylglycidylether, sowie Epoxysilane wie 3-Glycidoxypropyltrimethoxysilan, 3-Glycidoxypropyltriethoxysilan, 3-Glycidoxypropyldimethoxymethylsilan oder 3-Glycidoxypropyldiethoxymethylsilan;
- aromatische Monoepoxide, bevorzugt Styroloxid oder Glycidylether von Phenol, Kresol, tert.Butylphenol oder Cardanol;
- aliphatische oder cycloaliphatische, Ethergruppen-haltige Polyepoxide, bevorzugt Glycidylether von Glykolen wie Ethylenglykol, Propylenglykol, Butylenglykol, Hexandiol, Octandiol, Polypropylenglykolen, Dimethylolcyclohexan, Neopentylglykol, Rizinusöl, Trimethylolpropan, Trimethylolethan, Pentaerythrol, Glycerin, alkoxyliertes Glycerin oder alkoxyliertes Trimethylolpropan, sowie kernhydrierte Bisphenol-A-, -F- oder -A/F-Flüssigharze; oder
- aromatische Polyepoxide, bevorzugt Diglycidylether von Bisphenol-A, Bisphenol-F oder Bisphenol-A/F oder Novolak-Glycidylether, insbesondere in Form von sogenannten Flüssigharzen, wie sie im Handel beispielsweise von Dow, Huntsman oder Hexion erhältlich sind.

Als funktionelle Verbindung mit mindestens einer Aziridingruppe geeignet sind N-Alkylaziridine, insbesondere Michael-Addukte von Aziridin oder 2-Methylaziridin, bevorzugt Methyl 3-(aziridin-1-yl)propanoat, Methyl 3-(2-methylaziridin-1-yl)propanoat, Butyl 3-(aziridin-1-yl)propanoat, Butyl 3-(2-methylaziridin-1-yl)-propanoat, 1,1,1-Trimethylolpropan tris(3-(aziridin-1-yl)propanoat), 1,1,1-Trimethylolpropan tris(3-(2-methylaziridin-1-yl)propanoat), Pentaerythritol tetrakis(3-(aziridin-1-yl)propanoat) oder Pentaerythritol tetrakis(3-(2-methylaziridin-1-yl)-propanoat).

Als funktionelle Verbindung mit mindestens einer Carbonatgruppe geeignet sind Dialkylcarbonate, bevorzugt Dimethylcarbonat, sowie insbesondere cyclische Carbonate, bevorzugt Ethylencarbonat, 1,2-Propylencarbonat, 4-Ethyl-1,3-dioxolan-2-on, 1,3-Dioxan-2-on oder 5,5-Diethyl-1,3-dioxan-2-on, oder durch Insertion von CO₂ in die Epoxidgruppe(n) der oben genannten funktionellen Verbindungen mit mindestens einer Epoxidgruppe erhältliche cyclische Carbonate. Besonders bevorzugt ist 1,2-Propylencarbonat.

Als funktionelle Verbindung mit mindestens einer Carbonsäureanhydrid-Gruppe geeignet sind Anhydride von Monocarbonsäuren, bevorzugt Essigsäureanhydrid, sowie insbesondere cyclische Anhydride von Dicarbonsäuren oder Polycarbonsäuren, bevorzugt Bernsteinsäureanhydrid, Maleinsäureanhydrid, Hexahydrophthalsäureanhydrid, Methylhexahydrophthalsäureanhydrid, Tetrahydrophthalsäureanhydrid, Phthalsäureanhydrid, Methylphthalsäureanhydrid, Trimellitsäureanhydrid, Pyromellitsäuredianhydrid oder 4,4'-[(lsopropyliden)-bis(p-phenylenoxy)]diphthalsäuredianhydrid.

Als funktionelle Verbindung mit mindestens einer Carbonsäureester-Gruppe geeignet sind insbesondere
- Alkylester von aliphatischen, cycloaliphatischen und arylaliphatischen Monocarbonsäuren, bevorzugt von Ameisensäure, Essigsäure, Propionsäure, Buttersäure, Isobuttersäure, Valeriansäure, Capronsäure, 2-Ethyl-capronsäure, Oenanthsäure, Caprylsäure, Pelargonsäure, Caprinsäure, Undecansäure, Laurinsäure, Tridecansäure, Myristinsäure, Pentadecansäure, Palmitinsäure, Margarinsäure, Stearinsäure, Nonadecansäure, Arachinsäure, Palmitoleinsäure, Ölsäure, Erucasäure, Cyclohexancarbonsäure oder Phenylessigsäure, sowie Gemische aus gesättigten und/oder einfach und/oder mehrfach ungesättigten Fettsäuren, wie sie bei der Verseifung natürlicher Fette und Öle pflanzlichen oder tierischen Ursprungs erhalten werden;
- Alkylester von aromatischen Monocarbonsäuren, bevorzugt von Benzoesäure, Toluylsäure, Salicylsäure, Anissäure oder Naphthoesäure;
- Dialkylester von aliphatischen und cycloaliphatischen Dicarbonsäuren (sogenannte dibasische Ester), bevorzugt von Oxalsäure, Malonsäure, Bernsteinsäure, Glutarsäure, Adipinsäure, Pimelinsäure, Korksäure, Azelainsäure, Sebacinsäure, 1,12-Dodecandisäure, Hexahydrophthalsäure, Hexahydroisophthalsäure, Methylhexahydrophthalsäure, Hexahydroterephthalsäure, Dimerfettsäuren, 3,6,9-Trioxaundecandisäure oder von ähnlichen Derivaten von Polyethylenglykol;
- Dialkylester von aromatischen Dicarbonsäuren, bevorzugt von Phthalsäure, Isophthalsäure oder Terephthalsäure;
- Ester von tri- oder mehrfunktionellen Carbonsäuren, bevorzugt von Citronensäure oder Trimellithsäure.
Besonders bevorzugt sind dabei die Methyl- oder die Ethylester.

Als funktionelle Verbindung mit mindestens einer Lactongruppe geeignet sind β-, γ-, δ- oder ε-Lactone, bevorzugt β-Butyrolacton, γ-Butyrolacton, γ-Valerolacton, γ-Caprolacton, γ-Heptalacton, γ-Octalacton, γ-Nonalacton, γ-Decalacton, γ-Undecalacton, γ-Dodecalacton, δ-Valerolacton, δ-Caprolacton, δ-Heptalacton, δ-Octalacton, δ-Nonalacton, δ-Decalacton, δ-Undecalacton, δ-Dodecalacton, ε-Caprolacton, ε-Heptalacton, ε-Octalacton, ε-Nonalacton, ε-Decalacton, ε-Undecalacton oder ε-Dodecalacton. Insbesondere geeignet sind γ-, δ- oder ε-Lactone, bevorzugt γ-Butyrolacton, γ-Valerolacton, γ-Caprolacton, γ-Octalacton, γ-Nonalacton, γ-Decalacton, γ-Undecalacton, γ-Dodecalacton, δ-Valerolacton, δ-Caprolacton, δ-Nonalacton, δ-Decalacton, δ-Undecalacton, δ-Dodecalacton, ε-Caprolacton, ε-Decalacton, ε-Undecalacton oder ε-Dodecalacton. Besonders bevorzugt sind γ-Butyrolacton, γ-Valerolacton, δ-Valerolacton, δ-Caprolacton oder ε-Caprolacton.

Als funktionelle Verbindung mit mindestens einer Ketogruppe geeignet sind insbesondere
- aliphatische, cycloaliphatische oder aromatische Ketone, bevorzugt Aceton, Methylethylketon, Methylpropylketon, Methylisopropylketon, Methylisobutylketon, Methylpentylketon, Methylisopentylketon, Diethylketon, Dipropylketon, Diisopropylketon, Dibutylketon, Diisobutylketon, Cyclopentanon, Cyclohexanon, Acetophenon, Propiophenon oder Benzophenon;
- Diketone, bevorzugt 2,3-Butandion, 2,3-Pentandion, 2,3-Hexandion, 3,4-Hexandion, 2,5-Hexandion, 3,4-Dimethyl-2,5-hexandion, 1,2-Dibenzoylethan, 1,4-Bis-(2-furyl)-1,4-butandion, 2-(2-Oxopropyl)cyclopentanon, 1,2-Diacetylbenzol, 1,3-Diacetylbenzol, 1,4-Diacetylbenzol, 1,2-Diacetylcyclohexan, 1,3-Diacetylcyclohexan oder 1,4-Diacetylcyclohexan;
- Triketone, bevorzugt 1,3,5-Triacetylbenzol oder 1,3,5-Triacetylcyclohexan.

Als funktionelle Verbindung mit mindestens einer Aldehydgruppe geeignet sind insbesondere
- aliphatische, cycloaliphatische oder aromatische Aldehyde, bevorzugt Formaldehyd, Acetaldehyd, Propanal, 2-Methylpropanal, 2,2-Dimethylpropanal, 2,2-Dimethyl-3-phenylpropanal, Butanal, 2-Methylbutanal, 2-Ethylbutanal, Pentanal, 2-Methylpentanal, 3-Methylpentanal, 4-Methylpentanal, 2,3-Dimethylpentanal, Hexanal, 2-Ethylhexanal, Heptanal, Octanal, Nonanal, Decanal, Undecanal, 2-Methylundecanal, Dodecanal, Methoxyacetaldehyd, Cyclopropancarboxaldehyd, Cyclopentancarboxaldehyd, Cyclohexancarboxaldehyd, Diphenylacetaldehyd, Benzaldehyd, die isomeren Tolualdehyde, 4-Ethyl- oder 4-Propyl- oder 4-Isopropyl oder 4-Butyl-benzaldehyd, 2,4-Dimethylbenzaldehyd, 2,4,5-Trimethylbenzaldehyd, 4-Acetoxybenzaldehyd, 4-Anisaldehyd, 4-Ethoxybenzaldehyd, die isomeren Di- oder Trialkoxybenzaldehyde, 2-, 3- oder 4-Nitrobenzaldehyd, 2-, 3- oder 4-Formylpyridin, Furfural, 2-Thiophencarbaldehyd, 1- oder 2-Naphthylaldehyd, 3- oder 4-Phenyloxybenzaldehyd, Chinolin-2-carbaldehyd oder dessen 3-, 4-, 5-, 6-, 7- oder 8-Stellungsisomere, Anthracen-9-carbaldehyd, Zimtaldehyd oder substituierte Zimtaldehyde;
- Hydroxylgruppen aufweisende Aldehyde, bevorzugt 2,2-Dimethyl-3-hydroxypropanal, 3-Bis(2-hydroxyethyl)amino-2,2-dimethylpropanal, 3-Bis(2-hydroxypropyl)amino-2,2-dimethylpropanal, die isomeren Salicylaldehyde oder Vanillin;
- Estergruppen aufweisende Aldehyde, bevorzugt 2,2-Dimethyl-3-formoxypropanal, 2,2-Dimethyl-3-acetoxypropanal, 2,2-Dimethyl-3-isobutoyloxypropanal, 2,2-Dimethyl-3-caproyloxypropanal, 2,2-Dimethyl-3-(2-ethylhexoyloxy)-propanal, 2,2-Dimethyl-3-capryloyloxypropanal, 2,2-Dimethyl-3-caprinoyloxypropanal, 2,2-Dimethyl-3-lauroyloxypropanal, 2,2-Dimethyl-3-myristoyloxypropanal, 2,2-Dimethyl-3-palmitoyloxypropanal, 2,2-Dimethyl-3-stearoyloxypropanal, 2,2-Dimethyl-3-oleyloxypropanal, 2,2-Dimethyl-3-benzoyloxypropanal oder Glyoxylsäuremethyl- oder -ethylester;
- Ethergruppen aufweisende Aldehyde, bevorzugt 2,2-Dimethyl-3-phenoxypropanal, 3-Cyclohexyloxy-2,2-dimethylpropanal, 2,2-Dimethyl-3-(2-ethylhexyloxy)-propanal, 2,2-Dimethyl-3-lauroxy-propanal oder 2,2-Dimethyl-3-stearoxypropanal;
- tertiäre Aminogruppen aufweisende Aldehyde, bevorzugt 2,2-Dimethyl-3-(N,N-dimethylamino)-propanal, 2,2-Dimethyl-3-(N,N-diethylamino)-propanal, 2,2-Dimethyl-3-(N,N-dibutylamino)-propanal, 2,2-Dimethyl-3-(N-pyrrolidino)-propanal, 2,2-Dimethyl-3-(N-piperidino)-propanal, 2,2-Dimethyl-3-(N-morpholino)-propanal, 3-(N-(2,6-dimethylmorpholino))-propanal, 3-Bis(methoxyethyl)amino-2,2-dimethylpropanal oder 3-Bis(2-hydroxypropyl)amino-2,2-dimethylpropanal;
- Dialdehyde, bevorzugt Glyoxal, Glutaraldehyd, ortho-Phthalaldehyd, Isophthalaldehyd, Terephthalaldehyd, Naphthalindicarboxaldehyd, Anthracendicarboxaldehyd, 2,5-Furandicarbaldehyd, 2,5-Thiophendicarbaldehyd, Cyclopentandicarbaldehyd, 1,2-Cyclohexandicarbaldehyd, 1,3-Cyclohexandicarbaldehyd, 1,4-Cyclohexandicarbaldehyd, 2(3),5(6)-Diformyl-bicyclo[2.2.1]-heptan (Norbornandicarbaldehyd), 3(4),8(9)-Diformyl-tricyclo[5.2.1.02,6]decan (Tricyclodecandicarbaldehyd oder TCD-Dialdehyd), 2,5-Tetrahydrofurandicarbaldehyd, 2,5-Tetrahydrothiophendicarbaldehyd, 1,3-Bis(4,4-dimethyl-5-oxo-2-pentyl)benzol, 1,4-Bis(4,4-dimethyl-5-oxo-2-pentyl)benzol, 3-(3-Oxopropyl)cyclohexancarbaldehyd, 4-(3-Oxopropyl)cyclohexancarbaldehyd, 3-(1-Formylethyl)cyclohexancarbaldehyd, 4-(1-Formylethyl)cyclohexancarbaldehyd, N,N'-Bis(2,2-dimethyl-3-oxopropyl)piperazin, N,N'-Bis(2,2-diethyl-3-oxopropyl)piperazin, N,N'-Bis(2-methyl-2-propyl-3-oxopropyl)piperazin oder N,N'-Bis(2-butyl-2-ethyl-3-oxopropyl)piperazin;
- Trialdehyde, bevorzugt 1,3,5-Benzoltricarbaldehyd oder 1,3,5-Cyclohexantricarbaldehyd.

Als funktionelle Verbindung mit mindestens einer 1,3-Diketogruppe bevorzugt sind 2,4-Pentandion, 3,5-Heptandion, 6-Methyl-3,5-heptandion, 2,2,6,6-Tetramethyl-3,5-heptandion, 2,2,4,6,6-Pentamethyl-3,5-heptandion, 1,3-Diphenyl-1,3-propandion, 3-Phenyl-2,4-pentandion, 2-Acetylcyclopentanon oder 2-Acetylcyclohexanon.

Als funktionelle Verbindung mit mindestens einer 1,3-Ketoestergruppe bevorzugt sind Methylacetoacetat, Ethylacetoacetat, Propylacetoacetat, Isopropylacetoacetat, Butylacetoacetat, tert.Butylacetoacetat, Ethyl-3-oxo-valerat oder Ethyl-3-oxo-hexanoat, oder mehrfunktionelle Acetoacetate aus der mehrfachen Veresterung von Polyolen, bevorzugt Ethylenglykol-bis(acetoacetat), Propylenglykol-bis(acetoacetat), Diethylenglykol-bis(acetoacetat), Dipropylenglykol-bis-(acetoacetat), 1,3-Propandiol-bis(acetoacetat), 1,4-Butandiol-bis(acetoacetat), 1,5-Pentandiol-bis(acetoacetat), 3-Methyl-1,5-pentandiol-bis(acetoacetat), 1,6-Hexandiol-bis(acetoacetat), 2,2(4),4-Trimethyl-1,6-hexandiol-bis(acetoacetat), Poly(oxyalkylen)-bis(acetoacetate), 1,3-Cyclohexandimethanol-bis(acetoacetat), 1,4-Cyclohexandiol-bis(acetoacetat), Glycerin-tris(acetoacetat) oder Trimethylolpropan-tris(acetoacetat).

Als funktionelle Verbindung mit mindestens einer 1,3-Ketoamidgruppe bevorzugt sind N,N-Diethyl-3-oxo-butanamid, N,N-Dibutyl-3-oxo-butanamid, N,N-Bis(2-ethylhexyl)-3-oxo-butanamid, N,N-Bis(2-methoxyethyl)-3-oxo-butanamid, N,N-Dibutyl-3-oxo-heptanamid, N,N-Bis(2-methoxyethyl)-3-oxo-heptanamid, N,N-Bis(2-ethylhexyl)-2-oxo-cyclopentancarboxamid, N,N-Dibutyl-3-oxo-3-phenylpropanamid oder N,N-Bis(2-methoxyethyl)-3-oxo-3-phenylpropanamid, oder mehrfunktionelle Ketoamide aus der mehrfachen Amidierung von Polyetheraminen mit Diketen oder einem 1,3-Ketoester.

Als funktionelle Verbindung mit mindestens einer Cyanatgruppe geeignet sind insbesondere Cyansäureester von Phenolen oder Polyphenolen, bevorzugt Phenylcyanat, 2,2-Bis(4-cyanatophenyl)propan (Bisphenol A-Dicyanat), Bis(4-cyanatophenyl)methan, Bis(4-cyanato-3,5-dimethylphenyl)methan, 1,1-Bis(4-cyanatophenyl)ethan, Bis(4-cyanatophenyl)dicyclopentadien, m-Phenylendicyanat, p-Phenylendicyanat, 4,4'-Dicyanatodiphenylsulfon, 1,3,5-Tricyanatobenzol, Novolak-Cyanate, Cyanate von Ester-modifizierten Bisphenolen, insbesondere von Caprolacton-modifizierten Bisphenolen, sowie Cyanatgruppen aufweisende Polymere der genannten Polycyanate mit Polyolen, insbesondere Polyether- oder Polyesterpolyolen.

Als funktionelle Verbindung mit mindestens einer Isocyanatgruppe geeignet sind insbesondere
- aliphatische oder cycloaliphatische oder aromatische Monoisocyanate, bevorzugt Butylisocyanat, Hexylisocyanat, Laurylisocyanat, Stearylisocyanat, Cyclohexylisocyanat, Allylisocyanat oder Phenylisocyanat, sowie 3-Isocyanatopropyltrimethoxysilan, 3-Isocyanatopropyltriethoxysilan, 3-Isocyanatopropyldimethoxymethylsilan oder 3-Isocyanatopropyldiethoxymethylsilan;
- aliphatische oder cycloaliphatische Di- oder Triisocyanate, bevorzugt 1,4-Tetramethylendiisocyanat, 2-Methylpentamethylen-1,5-diisocyanat, 1,6-Hexamethylendiisocyanat (HDI), 2,2,4- und/oder 2,4,4-Trimethyl-1,6-hexamethylendiisocyanat (TMDI), 1,10-Decamethylendiisocyanat, 1,12-Dodecamethylendiisocyanat, Lysin- oder Lysinesterdiisocyanat, Cyclohexan-1,3- oder -1,4-diisocyanat, 1-Methyl-2,4- und/oder -2,6-diisocyanatocyclohexan (H₆TDI), 1-Isocyanato-3,3,5-trimethyl-5-isocyanatomethyl-cyclohexan (IPDI), Perhydro-2,4'- und/oder -4,4'-diphenylmethandiisocyanat (H₁₂MDI), 1,3- oder 1,4-Bis-(isocyanatomethyl)cyclohexan, m- oder p-Xylylendiisocyanat (m- oder p-XDI), Tetramethyl-1,3- oder -1,4-xylylendiisocyanat (m- oder p-TMXDI), Bis-(1-Isocyanato-1-methylethyl)naphthalin, Dimer- oder Trimerfettsäureisocyanate wie insbesondere 3,6-Bis-(9-isocyanatononyl)-4,5-di-(1-heptenyl)cyclohexen (Dimeryldiisocyanat); besonders bevorzugt HDI oder IPDI;
- aromatische Di- oder Triisocyanate, bevorzugt 2,4- und/oder 2,6-Toluylendiisocyanat (TDI), 4,4'- und/oder 2,4'- und/oder 2,2'-Diphenylmethandiisocyanat (MDI), Gemische aus MDI und MDI-Homologen (polymeres MDI oder PMDI), 1,3- oder 1,4-Phenylendiisocyanat, 2,3,5,6-Tetramethyl-1,4-diisocyanatobenzol, Naphthalin-1,5-diisocyanat (NDI), 3,3'-Dimethyl-4,4'-diisocyanatodiphenyl (TODI), Dianisidindiisocyanat (DADI), 1,3,5-Tris-(isocyanatomethyl)benzol, Tris-(4-isocyanatophenyl)methan oder Tris-(4-isocyanatophenyl)thiophosphat; besonders bevorzugt MDI oder TDI;
- Oligomere und Derivate der genannten Di- und Triisocyanate, bevorzugt abgeleitet von HDI, IPDI, MDI oder TDI. Davon insbesondere geeignet sind im Handel erhältliche Typen, bevorzugt HDI-Biurete, HDI-Isocyanurate, HDI-Uretdione, HDI-Iminooxadiazindione, HDI-Allophanate, IPDI-Isocyanurate, TDI-Oligomere oder gemischte Isocyanurate auf Basis TDI/HDI, oder bei Raumtemperatur flüssige Formen von MDI (sogenanntes "modifiziertes MDI"), welche Gemische von MDI mit MDI-Derivaten, wie insbesondere MDI-Carbodiimiden oder MDI-Uretoniminen oder MDI-Urethanen darstellen, Gemische aus MDI und MDI-Homologen (polymeres MDI oder PMDI). Solche oligomeren Polyisocyanate stellen in der Praxis üblicherweise Gemische von Substanzen mit unterschiedlichen Oligomerisierungsgraden und/oder chemischen Strukturen dar. Vorzugsweise weisen sie eine mittlere NCO-Funktionalität von 2.1 bis 4.0 auf.
- Isocyanatgruppen-haltige Polyurethanpolymere aus der Umsetzung von Polyolen, insbesondere Polyetherpolyolen, mit Polyisocyanaten, wie nachfolgend beschrieben.
Bevorzugt weist die funktionelle Verbindung mit mindestens einer Isocyanat-gruppe aliphatische Isocyanatgruppen auf, insbesondere abgeleitet von IPDI oder HDI. Diese Isocyanatgruppen weisen eine moderate Reaktivität auf, was die Umsetzung mit einem Guanidin der Formel (II) erleichtert.

Ein geeignetes Isocyanatgruppen-haltiges Polyurethanpolymer wird bevorzugt erhalten aus der Umsetzung von Polyolen mit einer überstöchiometrischen Menge an Polyisocyanaten, insbesondere Diisocyanaten. Die Umsetzung wird bevorzugt unter Feuchtigkeitsausschluss bei einer Temperatur im Bereich von 50 °C bis 100 °C durchgeführt, gegebenenfalls in Anwesenheit geeigneter Katalysatoren. Der Überschuss an Polyisocyanat wird bevorzugt so gewählt, dass im Polyurethanpolymer nach der Umsetzung aller Hydroxylgruppen ein Gehalt an freien Isocyanatgruppen im Bereich von 0.5 bis 20 Gewichts-%, bevorzugt 0.5 bis 10 Gewichts-%, besonders bevorzugt 0.5 bis 5 Gewichts-%, bezogen auf das gesamte Polymer, verbleibt.
Ein geeignetes Isocyanatgruppen-haltiges Polyurethanpolymer weist bevorzugt ein mittleres Molekulargewicht im Bereich von 500 bis 20'000 g/mol auf. Geeignete Polyisocyanate sind insbesondere Diisocyanate, bevorzugt 1,6-Hexamethylendiisocyanat (HDI), 1-Isocyanato-3,3,5-trimethyl-5-isocyanatomethylcyclohexan (Isophorondiisocyanat oder IPDI), 2,4- und/oder 2,6-Toluylendiisocyanat (TDI) oder 4,4'-, 2,4'- und/oder 2,2'-Diphenylmethandiisocyanat (MDI). Geeignete Polyole sind insbesondere Polyetherpolyole, bevorzugt Polyoxyalkylenpolyole, welche Polymerisationsprodukte von Ethylenoxid oder 1,2-Propylenoxid oder 1,2- oder 2,3-Butylenoxid oder Oxetan oder Tetrahydrofuran oder Mischungen davon sind, eventuell polymerisiert mit Hilfe eines Startermoleküls mit zwei oder mehreren aktiven Wasserstoffatomen; Polyesterpolyole, bevorzugt Produkte aus der Polykondensation von Diolen oder Triolen mit Lactonen oder Dicarbonsäuren oder deren Ester oder Anydriden; Polycarbonatpolyole, OH-endständige Blockcopolymere mit mindestens zwei unterschiedlichen Blöcken mit Polyether-, Polyester- oder Polycarbonat-Einheiten; Polyacrylat- oder Polymethacrylat-Polyole; Polyhydroxy-funktionelle Fette oder Öle, insbesondere natürliche Fette oder Öle; oder Polykohlenwasserstoffpolyole wie beispielsweise Polyhydroxy-funktionelle Polyolefine.
Weiterhin geeignete Polyole sind niedrigmolekulare zwei- oder mehrwertige Alkohole, bevorzugt 1,2-Ethandiol, 1,2-Propandiol, Neopentylglykol, Dibromoneopentylglykol, Diethylenglykol, Triethylenglykol, die isomeren Dipropylenglykole oder Tripropylenglykole, die isomeren Butanediole, Pentandiole, Hexandiole, Heptandiol, Octandiole, Nonandiole, Decandiole, Undecandiol, 1,3- oder 1,4-Cyclohexandimethanol, hydriertes Bisphenol A, Dimerfettsäurealkohole, 1,1,1-Trimethylolethan, 1,1,1-Trimethylolpropan, Glycerin, Pentaerythritol, Zuckeralkohole oder Zucker, oder niedrigmolekulare Alkoxylierungsprodukte der genannten zwei- oder mehrwertige Alkohole.
Geeignet sind insbesondere auch Mischungen der genannten Polyole. Geeignet sind insbesondere Diole oder Triole oder Mischungen davon.

Als funktionelle Verbindung mit mindestens einer Isothiocyanatgruppe geeignet sind insbesondere Methylisothiocyanat, Ethylisothiocyanat, Propylisothiocyanat, Isopropylisothiocyanat, n-Butylisothiocyanat, 2-Isothiocyanatobutan, Cyclohexylisothiocyanat, n-Octylisothiocyanat, Allylisothiocyanat, Phenylisothiocyanat, o- oder m- oder p-Tolylisothiocyanat, 1,2- oder 1,3- oder 1,4-Phenylendiisothiocyanat oder 2,4- und/oder 2,6-Toluylendiisothiocyanat.

Als funktionelle Verbindung mit mindestens einer Acrylat- oder Methacrylat-gruppe geeignet sind insbesondere
- Acrylsäureester oder Methacrylsäureester, bevorzugt Methyl (meth)acrylat, Ethyl (meth)acrylat, Butyl (meth)acrylat, 2-Ethylhexyl (meth)acrylat, Lauryl (meth)acrylat, Stearyl (meth)acrylat, Cyclohexyl (meth)acrylat, Tetrahydrofuryl (meth)acrylat, Isobornyl (meth)acrylat, 2-Phenoxyethyl (meth)acrylat, 2-(2-Phenoxyethoxy)ethyl (meth)acrylat, 2-(4-Nonylphenoxy)ethyl (meth)acrylat, oder 3-(Meth)acryloxypropyltrimethoxysilan, 3-(Meth)acryloxypropyltriethoxysilan, 3-Meth)acryloxypropyldimethoxymethylsilan oder 3-(Meth)acryloxypropyldiethoxymethylsilan;
- zwei- oder mehrwertige Acrylate oder Methacrylate von aliphatischen Polyethern, Polyestern, Novolaken, Phenolen, aliphatischen oder cycloaliphatischen Alkoholen, Glykolen, Polyesterglykolen oder mono- oder polyalkoxylierten Derivaten der vorgenannten Verbindungen, bevorzugt Ethylenglykoldi(meth)acrylat, Tetraethylenglykol-di(meth)acrylat, Tripropylenglykol-di-(meth)acrylat, Polyethylenglykol-di(meth)acrylat, Polypropylenglykol-di-(meth)acrylat, 1,4-Butandiol-di(meth)acrylat, 1,6-Hexandiol-di(meth)acrylat, Neopentylglykol-di(meth)acrylat, Trimethylolpropan-tri(meth)acrylat, Pentaerythritol-tetra(meth)acrylat, Dipentaerythritol-tetra(meth)acrylat, Dipentaery-thritol-penta(meth)acrylat, Dipentaerythritol-hexa(meth)acrylat; zwei- oder mehrwertige acryl- oder methacrylfunktionelle Polybutadiene, Polyisoprene oder Block-copolymere davon; Addukte aus zwei- oder mehrwertigen Epoxiden wie die bereits genannten mit Acrylsäure oder Methacrylsäure; zwei- oder mehrwertige Polyurethanacrylate oder -methacrylate, insbesondere Umsetzungsprodukte aus Isocyanatgruppen-haltigen Polyurethanpolymeren mit 2-Hydroxyethylacrylat; Tris-(2-hydroxyethyl)isocyanurat-tri(meth)acrylat oder Tris-(2-hydroxyethyl)cyanurat-tri(meth)acrylat;
- zwei- oder mehr Acrylat- oder Methacrylat-Gruppen aufweisende Polyurethanpolymere, insbesondere mit einem mittleren Molekulargewicht im Bereich von 500 bis 20'000 g/mol, insbesondere aus der Umsetzung von Hydroxy-funktionellen (Meth)acrylaten, wie insbesondere 2-Hydroxyethylacrylat, mit Isocyanatgruppen-haltigen Polyurethanpolymeren.

Als funktionelle Verbindung mit mindestens einer Acrylamid- oder Methacrylamidgruppe geeignet sind insbesondere
- Acrylamid, Methacrylamid oder N-substituierte Acrylamide oder Methacrylamide, bevorzugt N,N-Dimethylacrylamid, N,N-Diethylacrylamid,N-Methyl-acrylamid, N-Ethylacrylamid, N-Propylacrylamid, N-Isopropylacrylamid, N-Butylacrylamid, N-tert.Butylacrylamid, N,N-Dimethylaminopropylacrylamid, N-Butoxymethylacrylamid, N-Isobutoxymethylacrylamid oder Diacetonacrylamid;
- zwei- oder mehrwertige Acrylamide oder Methacrylamide, bevorzugt N,N'-Methylen-bis(acrylamid), N,N'-Ethylen-bis(acrylamid) oder N,N',N"-Tris-((meth)acryloyl)perhydrotriazin.

Als funktionelle Verbindung mit mindestens einer Acrylnitril- oder Methacrylnitrilgruppe geeignet sind insbesondere Acrylnitril oder Methacrylnitril. Bevorzugt ist Acrylnitril.
Als funktionelle Verbindung mit mindestens einer Maleatgruppe geeignet sind insbesondere Dialkylmaleate, bevorzugt Maleinsäuredimethylester, Maleinsäurediethylester oder Maleinsäuredibutylester.
Als funktionelle Verbindung mit mindestens einer Fumaratgruppe geeignet sind insbesondere Dialkylfumarate, bevorzugt Fumarsäuredimethylester, Fumarsäurediethylester oder Fumarsäuredibutylester.
Als funktionelle Verbindung mit mindestens einer Maleimidgruppe geeignet sind insbesondere Maleimid sowie N-Alkylmaleimide, bevorzugt N-Methylmaleimid, N-Ethylmaleimid, N-Butylmaleimid, N-Hexylmaleimid oder 1,1-(1,6-Hexylen)-bis(1H-pyrrol-2,5-dion).
Als funktionelle Verbindung mit mindestens einer Itaconatgruppe geeignet sind insbesondere Dialkylitaconate, bevorzugt Itaconsäuredimethylester, Itaconsäurediethylester, Itaconsäuredibutylester oder Itaconsäuredihexylester.

Besonders bevorzugt ist die funktionelle Verbindung ausgewählt aus der Gruppe bestehend aus 2-Ethylhexylglycidylether, C₈- bis C₁₀-Alkylglycidylether, C₁₂-bis C₁₄-Alkylglycidylether, Kresylglycidylether, tert.Butylphenylglycidylether, Cardanol-Glycidylether, 1,4-Butandioldiglycidylether, 1,6-Hexandioldiglycidylether, Neopentylglykoldiglycidylether, Polypropylenglykoldiglycidylether mit einem mittleren Molekulargewicht im Bereich von 280 bis 1000 g/mol, Bisphenol-A-Digylcidylether, Bisphenol-F-Digylcidylether, 3-Glycidoxypropyltrimethoxysilan, 3-Glycidoxypropyltriethoxysilan, 1,2-Propylencarbonat, Bernsteinsäureanhydrid, Maleinsäureanhydrid, Hexahydrophthalsäureanhydrid, Phthalsäureanhydrid, Capronsäuremethylester, Capronsäureethylester, 2-Ethylcapronsäuremethylester, 2-Ethylcapronsäureethylester, Laurinsäuremethylester, Laurinsäureethylester, Benzoesäuremethylester, Benzoesäureethylester, Adipinsäuredimethylester, Adipinsäurediethylester, Phthalsäuredimethylester, Phthalsäurediethylester, Isophthalsäuredimethylester, Isophthalsäurediethylester, Terephthalsäuredimethylester, Terephthalsäurediethylester, γ-Butyrolacton, γ-Valerolacton, δ-Valerolacton, δ-Caprolacton, ε-Caprolacton, Aceton, Methylethylketon, Methylisopropylketon, Methylisobutylketon, Cyclopentanon, Cyclohexanon, Acetophenon, 2-Methylpropanal, 2-Ethylhexanal, Dodecanal, Benzaldehyd, 2,2-Dimethyl-3-acetoxypropanal, 2,2-Dimethyl-3-lauroyloxypropanal, 2,2-Dimethyl-3-(N-morpholino)-propanal, 3-Bis(methoxyethyl)amino-2,2-dimethylpropanal, Terephthalaldehyd, 2,4-Pentandion, Methylacetoacetat, Ethylacetoacetat, Isopropylacetoacetat, tert.Butylacetoacetat, Ethylenglykolbis(acetoacetat), Propylenglykol-bis(acetoacetat), Diethylenglykol-bis(aceto-acetat), Dipropylenglykol-bis(acetoacetat), 1,3-Propandiol-bis(acetoacetat), 1,4-Butandiol-bis(acetoacetat), 1,5-Pentandiol-bis(acetoacetat), 3-Methyl-1,5-pentandiol-bis(acetoacetat), 1,6-Hexandiol-bis(acetoacetat), 2,2(4),4-Trimethyl-1,6-hexandiol-bis(acetoacetat), N,N-Dimethyl-3-oxo-butanamid, N,N-Diethyl-3-oxo-butanamid, N,N-Dibutyl-3-oxo-butanamid, N,N-Bis(2-ethylhexyl)-3-oxo-butanamid, N,N-Bis(2-methoxyethyl)-3-oxo-butanamid, Butylisocyanat, Hexylisocyanat, Laurylisocyanat, Stearylisocyanat, Cyclohexylisocyanat, Phenylisocyanat, 3-Isocyanatopropyltrimethoxysilan, 3-Isocyanatopropyltriethoxysilan, Isocyanatgruppen-haltige Polyurethanpolymeren mit einem mittleren Molekulargewicht im Bereich von 500 bis 20'000 g/mol aus der Umsetzung von Polyolen mit Diisocyanaten, Methylisothiocyanat, Ethylisothiocyanat, Isopropylisothiocyanat, Butylisothiocyanat, Cyclohexylisothiocyanat, Phenylisothiocyanat, Butyl(meth)acrylat, 2-Ethylhexyl(meth)acrylat, (Meth)acryloxypropyltrimethoxysilan, (Meth)acryloxypropyltriethoxysilan, Ethylenglykol-di(meth)acrylat, Tetraethylenglykol-di(meth)acrylat, Tripropylenglykol-di(meth)acrylat, Polyethylenglykol-di(meth)acrylat mit einem mittleren Molekulargewicht im Bereich von 200 bis 2'000 g/mol, Polypropylenglykol-di(meth)acrylat mit einem mittleren Molekulargewicht im Bereich von 200 bis 2'000 g/mol, 1,4-Butandiol-di(meth)acrylat, 1,6-Hexandiol-di(meth)acrylat, Neopentylglykol-di(meth)acrylat, Trimethylolpropan-tri(meth)acrylat, (Meth)acrylat-Gruppen aufweisenden Polyurethanpolymeren mit einem mittleren Molekulargewicht im Bereich von 500 bis 20'000 g/mol, insbesondere aus der Umsetzung von hydroxy-funktionellen (Meth)acrylaten mit Isocyanatgruppen-haltigen Polyurthanpolymeren, Acrylnitril, Maleinsäurediethylester, Fumarsäurediethylester, N-Ethylmaleimid und Itaconsäurediethylester.

Ein Katalysator der Formel (I) aus der Umsetzung von mindestens einem Guanidin der Formel (II) mit mindestens einer der beschriebenen funktionellen Verbindungen wird bevorzugt dadurch erhalten, dass die HX-Gruppen ungefähr stöchiometrisch in Bezug auf die Reaktivgruppen der funktionellen Verbindung eingesetzt werden, wodurch der erhaltene Katalysator weitgehend frei von den genannten Reaktivgruppen ist. Die Umsetzung kann aber auch unter- oder überstöchiometrisch geführt werden. Insbesondere bei Einsatz einer funktionellen Verbindung, welche mehr als eine der genannten Reaktivgruppen enthält, kann eine unterstöchiometrische Umsetzung der Reaktivgruppen vorteilhaft sein, da der Katalysator der Formel (I) aufgrund der enthaltenen Reaktivgruppen eine zusätzliche Funktionalität aufweist, die beispielsweise zu dessen Einbindung in die Polymermatrix oder dessen Verankerung auf ein Substrat oder einen Füllstoff dienen kann.

Eine 1,3-Diketon- oder 1,3-Ketoester- oder 1,3-Ketoamid- oder (Meth)acrylat- oder Maleat- oder Fumarat- oder Itaconat-Gruppe kann mit HX-Gruppen zweimal reagieren. Wird die Umsetzung stöchiometrisch so durchgeführt, dass pro solcher Gruppe nur eine HX-Gruppe vorhanden ist, so wird diese bevorzugt an die Ketogruppe kondensiert bzw. an die Doppelbindung addiert. Ist hingegen mehr als eine HX-Gruppe vorhanden, so kann zusätzlich eine Umesterung bzw. Amidierung erfolgen. Bevorzugt wird eine solche Gruppe nur einmal umgesetzt.

Die Umsetzung erfolgt insbesondere unter Bedingungen, wie sie für Reaktionen zwischen den an der jeweiligen Umsetzung beteiligten Reaktivgruppen typischerweise verwendet werden, bevorzugt bei einer Temperatur im Bereich von 0 °C bis 160 °C. Die Umsetzung kann unter Einsatz eines Lösemittels oder bevorzugt lösemittelfrei erfolgen. Gegebenenfalls können Hilfsstoffe wie beispielsweise Katalysatoren, Initiatoren, Trocknungsmittel oder Stabilisatoren mitverwendet werden.

Ein geeignetes Guanidin der Formel (II) für die Umsetzung mit mindestens einer funktionellen Verbindung wird insbesondere erhalten aus der Umsetzung von
- mindestens einem Amin der Formel (III),

   HX'-A'-NHR¹ (III)

   wobei
   X' für O oder S oder NR⁸ steht und R⁸ für einen Wasserstoff-Rest oder für einen Alkyl- oder Cycloalkyl- oder Aralkyl-Rest mit 1 bis 8 C-Atomen, welcher gegebenenfalls eine primäre oder sekundäre oder tertiäre Aminogruppe enthält, steht,
   A' für A steht,
   wobei A' auch zusammen mit R⁸ für einen dreiwertigen Kohlenwasserstoff-Rest mit 5 bis 10 C-Atomen, welcher gegebenenfalls einen tertiären Amin-Stickstoff enthält, stehen kann,
   und A und R¹ die bereits genannten Bedeutungen aufweisen,
- mit mindestens einem Reagens zur Einführung von Guanidin-Gruppen ausgewählt aus der Gruppe bestehend aus Cyanamiden, Carbodiimiden, Harnstoffen, O-Alkylisoharnstoffen, Thioharnstoffen, S-Alkylisothioharnstoffen, Aminoiminomethansulfonsäuren, Guanylpyrazolen und Guanidinen.

X' steht bevorzugt für O oder NR⁸.
R⁸ steht bevorzugt für einen Wasserstoff-Rest.
R⁸ steht weiterhin bevorzugt für einen Alkyl- oder Cycloalkyl-Rest mit 1 bis 8 C-Atomen.
R⁸ steht weiterhin bevorzugt für einen Rest der Formel ---R⁷-NH₂, wobei R⁷ die bereits genannten Bedeutungen aufweist.
R⁸ steht weiterhin bevorzugt für einen N,N-Dimethylaminopropyl-Rest.
R⁸ steht weiterhin bevorzugt zusammen mit A' und unter Einbezug des Stickstoffatoms für Piperidin-4-ylmethyl oder 2-(Piperidin-4-yl)ethyl oder 2-Piperazinoethyl.

Der Umsetzung wird bevorzugt bei erhöhter Temperatur, gegebenenfalls unter erhöhtem Druck und gegebenenfalls in Gegenwart eines Katalysators, durchgeführt, wobei aus dem Reagens freigesetzte Abspalter während oder nach der Umsetzung bevorzugt entfernt werden, insbesondere mittels Destillation, gegebenenfalls unter Vakuum.
Bevorzugt wird das Verhältnis zwischen dem Amin der Formel (III) und dem Reagens so gewählt, dass das Reagens bei der Umsetzung vollständig umgesetzt wird.

Als Reagens zur Einführung von Guanidin-Gruppen bevorzugt sind Cyanamide oder Carbodiimide.
Als Reagens zur Einführung von Guanidin-Gruppen besonders bevorzugt ist ein Carbodiimid der Formel R⁵-N=C=N-R², wobei R² und R⁵ die beschriebenen Bedeutungen aufweisen.
Besonders geeignet ist N,N'-Diisopropylcarbodiimid (DIC), N,N'-Di-tert.butylcarbodiimid, N,N'-Dicyclohexylcarbodiimid (DCC) oder N-Ethyl-N'-(3-dimethylaminopropyl)carbodiimid (EDC), insbesondere N,N'-Diisopropylcarbodiimid (DIC) oder N,N'-Dicyclohexylcarbodiimid (DCC). Diese Reagentien sind einfach verfügbar und lassen sich gut zu Guanidinen umsetzen.

Als Amin der Formel (III) geeignet sind insbesondere
- aliphatische oder cycloaliphatische Hydroxylamine, insbesondere 2-Aminoethanol, 2-Methylaminoethanol (2-Amino-1-propanol), 1-Amino-2-propanol, 3-Amino-1-propanol, 4-Amino-1-butanol, 4-Amino-2-butanol, 2-Amino-2-methylpropanol, 5-Amino-1-pentanol, 6-Amino-1-hexanol, 7-Amino-1-heptanol, 8-Amino-1-octanol, 10-Amino-1-decanol, 12-Amino-1-dodecanol, 4-(2-Aminoethyl)-2-hydroxyethylbenzol, 3-Aminomethyl-3,5,5-trimethyl-cyclohexanol, eine primäre Aminogruppe tragende Derivate von Glykolen wie Diethylenglykol, Dipropylenglykol, Dibutylenglykol oder höheren Oligomeren oder Polymere dieser Glykole, insbesondere 2-(2-Aminoethoxy)ethanol, 2-(2-(2-Aminoethoxy)ethoxy)ethanol, α-(2-Hydroxymethylethyl)-ω-(2-aminomethylethoxy)poly(oxy(methyl-1,2-ethandiyl)), eine Hydroxylgruppe und eine primäre Aminogruppe tragende Derivate von polyalkoxylierten drei- oder höherwertigen Alkoholen, Produkte aus der einfachen Cyanoethylierung und anschliessenden Hydrierung von Glykolen, insbesondere 3-(2-Hydroxyethoxy)propylamin, 3-(2-(2-Hydroxyethoxy)ethoxy)propylamin oder 3-(6-Hydroxyhexyloxy)propylamin, sowie weiterhin Hydroxylamine mit einer primären und einer sekundären Aminogruppe wie insbesondere N-(2-Aminoethyl)-2-aminoethanol oder N-(3-Aminopropyl)-2-aminoethanol;
- aliphatische Mercaptoamine, insbesondere 2-Aminoethanthiol (Cysteamin), 3-Aminopropanthiol, 4-Amino-1-butanthiol, 6-Amino-1-hexanthiol, 8-Amino-1-octanthiol, 10-Amino-1-decanthiol oder 12-Amino-1-dodecanthiol;
- aliphatische, cycloaliphatische oder arylaliphatische primäre Diamine, insbesondere Ethylendiamin, 1,2- und 1,3-Propandiamin, 2-Methyl-1,2-propandiamin, 2,2-Dimethyl-1,3-propandiamin, 1,3- und 1,4-Butandiamin, 1,3-Pentandiamin (DAMP), 1,5-Pentandiamin, 1,5-Diamino-2-methylpentan (MPMD), 2-Butyl-2-ethyl-1,5-pentandiamin (C11-Neodiamin), 1,6-Hexandiamin, 2,5-Dimethyl-1,6-hexandiamin, 2,2,4- und 2,4,4-Trimethylhexamethylendiamin (TMD), 1,7-Heptandiamin, 1,8-Octandiamin, 1,9-Nonandiamin, 1,10-Decandiamin, 1,11-Undecandiamin, 1,12-Dodecandiamin, 1,2-, 1,3- oder 1,4-Diaminocyclohexan, Bis(4-aminocyclohexyl)methan, Bis(4-amino-3-methylcyclohexyl)methan, Bis(4-amino-3-ethylcyclohexyl)methan, Bis(4-amino-3,5-dimethylcyclohexyl)methan, Bis(4-amino-3-ethyl-5-methylcyclohexyl)methan, 1-Amino-3-aminomethyl-3,5,5-trimethylcyclohexan (Isophorondiamin oder IPD), 2- und/oder 4-Methyl-1,3-diaminocyclohexan, 1,3- oder 1,4-Bis-(aminomethyl)cyclohexan, 2,5(2,6)-Bis(aminomethyl)bicyclo[2.2.1]heptan (NBDA), 3(4),8(9)-Bis(aminomethyl)tricyclo[5.2.1.0^{2,6}]decan, 1,4-Diamino-2,2,6-trimethylcyclohexan (TMCDA), 1,8-Menthandiamin, 3,9-Bis(3-aminopropyl)-2,4,8,10-tetraoxaspiro[5.5]undecan, 1,3-Bis(aminomethyl)benzol, 1,4-Bis(aminomethyl)benzol, 4-Aminomethyl-1,8-octandiamin, oder Produkte aus der doppelten Cyanoethylierung und nachfolgenden Reduktion von Fettaminen, welche abgeleitet sind von natürlichen Fettsäuren, wie N,N-Bis(3-aminopropyl)dodecylamin oder N,N-Bis(3-aminopropyl)talgalkylamin, erhältlich als Triameen® Y12D oder Triameen® YT (von Akzo Nobel);
- Ethergruppenhaltige aliphatische oder cycloaliphatische primäre Diamine, insbesondere Bis(2-aminoethyl)ether, 3,6-Dioxaoctan-1,8-diamin, 4,7-Dioxadecan-1,10-diamin, 4,7-Dioxadecan-2,9-diamin, 4,9-Dioxadodecan-1,12-diamin, 5,8-Dioxadodecan-3,10-diamin, 4,7,10-Trioxatridecan-1,13-diamin, cycloaliphatische ethergruppenhaltige Diamine aus der Propoxylierung und nachfolgenden Aminierung von 1,4-Dimethylolcyclohexan, erhältlich insbesondere als Jeffamine® RFD-270 (von Huntsman), Polyoxyalkylenamine mit einem mittleren Molekulargewicht im Bereich von 200 bis 500 g/mol, wie sie im Handel beispielsweise unter den Handelsnamen Jeffamine® (von Huntsman), Polyetheramine (von BASF) und PC Amine® (von Nitroil) erhältlich sind, dadurch gekennzeichnet, dass sie 2-Aminopropyl- oder 2-Aminobutyl-Endgruppen tragen, insbesondere Jeffamine® D-230, Jeffamine® D-400, Jeffamine® XTJ-582, Jeffamine® HK-511 oder Jeffamine® XTJ-566 (alle von Huntsman), oder dazu analoge Typen von BASF und Nitroil;
- Diamine mit einer primären und einer sekundären Aminogruppe, insbesondere N-Methyl-1,2-ethandiamin, N-Ethyl-1,2-ethandiamin, N-Butyl-1,2-ethan-diamin, N-Hexyl-1,2-ethandiamin, N-(2-Ethylhexyl)-1,2-ethandiamin, N-Cyclohexyl-1,2-ethandiamin, 4-Aminomethylpiperidin, 4-((2-Amino)ethyl)piperidin, N-Methyl-1,3-propandiamin, N-Ethyl-1,3-propandiamin, N-Butyl-1,3-propandiamin, N-Hexyl-1,3-propandiamin, N-(2-Ethylhexyl)-1,3-propandiamin, N-Dodecyl-1,3-propandiamin, N-Cyclohexyl-1,3-propandiamin, 3-Methyl-amino-1-pentylamin, 3-Ethylamino-1-pentylamin, 3-Butylamino-1-pentyl-amin, 3-Hexylamino-1-pentylamin, 3-(2-Ethylhexyl)amino-1-pentylamin, 3-Dodecylamino-1-pentylamin, 3-Cyclohexylamino-1-pentylamin oder 3-aminopropylierte Fettamine wie insbesondere N-Cocoalkyl-1,3-propandiamin, N-Oleyl-1,3-propandiamin, N-Soyaalkyl-1,3-propandiamin, N-Talgalkyl-1,3-propandiamin oder N-(C₁₆₋₂₂-Alkyl)-1,3-propandiamin, wie sie beispielsweise unter dem Handelsnamen Duomeen® (von Akzo Nobel) erhältlich sind;
- Polyalkylenamine und weitere Polyamine mit primären und sekundären Aminogruppen und/oder tertiären Aminogruppen,, insbesondere Diethylentriamin (DETA), Triethylentetramin (TETA), Tetraethylenpentamin (TEPA), Pentaethylenhexamin (PEHA), Polyethylenpolyamin mit 5 bis 7 Ethylenamin-Einheiten (sogenanntes "higher ethylenepolyamine", HEPA), Dipropylentriamin (DPTA), N-(2-Aminoethyl)-1,3-propandiamin (N3-Amin), N,N'-Bis-(3-aminopropyl)ethylendiamin (N4-Amin), Bis-hexamethylentriamin (BHMT), N3-(3-Aminopentyl)-1,3-pentandiamin, N5-(3-Aminopropyl)-2-methyl-1,5-pentandiamin, N5-(3-Amino-1-ethylpropyl)-2-methyl-1,5-pentandiamin, N-(2-Aminoethyl)piperazin, N-(2-Aminopropyl)piperazin, N¹-((3-Dimethylamino)-propyl)-1,3-diaminopropan, N-Methyl-N'-(2-aminoethyl)-1,2-ethandiamin, N-Methyl-N'-(3-aminopropyl)-1,2-ethandiamin, N-Methyl-N'-(2-aminoethyl)-1,3-propandiamin oder N-Methyl-N'-(3-aminopropyl)-1,3-propandiamin.

Bevorzugt ist das Amin der Formel (III) ausgewählt aus der Gruppe bestehend aus Ethylendiamin, 1,2-Propandiamin, 1,3-Propandiamin, N-Methyl-1,2-ethan-diamin, N-Ethyl-1,2-ethandiamin, N-Butyl-1,2-ethandiamin, N-Hexyl-1,2-ethan-diamin, N-(2-Ethylhexyl)-1,2-ethandiamin, N-Cyclohexyl-1,2-ethandiamin, N-Methyl-1,3-propandiamin, N-Ethyl-1,3-propandiamin, N-Butyl-1,3-propandiamin, N-Hexyl-1,3-propandiamin, N-(2-Ethylhexyl)-1,3-propandiamin, N-Cyclohexyl-1,3-propandiamin, 1,3-Pentandiamin (DAMP), 1,5-Diamino-2-methylpentan (MPMD), 1,6-Hexandiamin, 2,2,4- und 2,4,4-Trimethylhexamethylendiamin (TMD), 1,8-Octandiamin, 1,10-Decandiamin, 1,12-Dodecandiamin, 1-Amino-3-aminomethyl-3,5,5-trimethylcyclohexan (Isophorondiamin oder IPD), 1,3-Bis(aminomethyl)cyclohexan, 1,4-Bis(aminomethyl)-cyclohexan, 1,3-Bis(aminomethyl)benzol, 2- und/oder 4-Methyl-1,3-diaminocyclohexan, 4-Aminomethylpiperidin, 4-((2-Amino)ethyl)piperidin, N-(2-Aminoethyl)piperazin, Diethylentriamin, Dipropylentriamin, N-(2-Aminoethyl)-1,3-propandiamin (N3-Amin), Bis(hexamethylen)triamin (BHMT), N'-Methyl-N'-(3-aminopropyl)-1,3-propandiamin, N¹-((3-Dimethylamino)propyl)-1,3-diaminopropan, Bis-(2-aminoethyl)ether, 3,6-Dioxaoctan-1,8-diamin, 4,7-Dioxadecan-1,10-diamin, 2-Aminoethanol, 1-Amino-2-propanol, 3-Amino-1-propanol, 2-Amino-2-methylpropanol, 5-Amino-1-pentanol, 6-Amino-1-hexanol, 3-Aminomethyl-3,5,5-trimethylcyclohexanol, 2-(2-Aminoethoxy)ethanol, 2-(2-(2-Aminoethoxy)ethoxy)ethanol und Polyoxypropylendiaminen mit einem mittleren Molekulargewicht im Bereich von etwa 220 bis 250 g/mol wie insbesondere Jeffamine® D-230.

Davon bevorzugt sind Amine mit einer sekundären und zwei primären Aminogruppen, also insbesondere Diethylentriamin, Dipropylentriamin, N-(2-Aminoethyl)-1,3-propandiamin (N3-Amin) oder Bis(hexamethylen)triamin (BHMT). Diese Amine lassen sich zu Guanidinen der Formel (II) mit zwei Guanidingruppen umsetzen, wobei X für NR³ und R³ für Rest der Formel ---R⁷-Z steht. Damit sind Katalysatoren der Formel (I) mit besonders hoher Aktivität erhältlich.

Davon bevorzugt sind weiterhin Amine mit einer primären und einer sekundären und gegebenenfalls einer tertiären Aminogruppe, also insbesondere N-Methyl-1,3-propandiamin, N-Ethyl-1,3-propandiamin, N-Butyl-1,2-ethandiamin, N-Hexyl-1,2-ethandiamin, N-(2-Ethylhexyl)-1,2-ethandiamin, N-Cyclohexyl-1,2-ethandiaminN-Methyl-1,3-propandiamin, N-Ethyl-1,3-propandiamin, N-Butyl-1,3-propandiamin, N-Hexyl-1,3-propandiamin, N-(2-Ethylhexyl)-1,3-propandiamin, N-Cyclohexyl-1,3-propandiamin, 4-Aminomethylpiperidin, 4-((2-Amino)ethyl)piperidin, N-(2-Aminoethyl)piperazin oder N¹-((3-Dimethylamino)propyl)-1,3-diaminopropan. Diese Amine lassen sich besonders selektiv zu Guanidinen der Formel (II) umsetzen, welche ihrereseits besonders einfach zu Katalysatoren der Formel (I) weiter umgesetzt werden können. Gegebenenfalls vorhandene tertiäre Aminogruppe sind insofern vorteilhaft, als dass sie die katalytische Aktivität weiter erhöhen können.

Das Guanidin der Formel (II) für die Umsetzung mit mindestens einer funktionellen Verbindung ist bevorzugt ausgewählt aus der Gruppe bestehend aus 1-(2-Aminoethyl)-2,3-diisopropylguanidin, 1-(2-Aminoethyl)-2,3-dicyclohexylguanidin,1-(2-Aminopropyl)-2,3-diisopropylguanidin, 1-(2-Aminopropyl)-2,3-dicyclohexylguanidin, 1-(3-Aminopropyl)-2,3-diisopropylguanidin, 1-(3-Aminopropyl)-2,3-dicyclohexylguanidin, 1-(2-Methylaminoethyl)-2,3-diisopropylguanidin, 1-(2-Methylaminoethyl)-2,3-dicyclohexylguanidin, 1-(2-Ethylaminoethyl)-2,3-diisopropylguanidin, 1-(2-Ethylaminoethyl)-2,3-dicyclohexylguanidin, 1-(2-Cyclohexylaminoethyl)-2,3-diisopropylguanidin, 1-(2-Cyclohexylaminoethyl)-2,3-dicyclohexylguanidin, 1-(3-Methylaminopropyl)-2,3-diisopropylguanidin, 1-(3-Methylaminopropyl)-2,3-dicyclohexylguanidin, 1-(3-Ethylaminopropyl)-2,3-diisopropylguanidin, 1-(3-Ethylaminopropyl)-2,3-dicyclohexylguanidin, 1-(3-Cyclohexylaminopropyl)-2,3-diisopropylguanidin, 1-(3-Cyclohexylaminopropyl)-2,3-dicyclohexylguanidin, 1-(3-Aminopentyl)-2,3-diisopropylguanidin, 1-(3-Aminopentyl)-2,3-dicyclohexylguanidin, 1-(5-Amino-4-methylpentyl)-2,3-diisopropylguanidin, 1-(5-Amino-4-methylpentyl)-2,3-dicyclohexylguanidin, 1-(5-Amino-2-methylpentyl)-2,3-diisopropylguanidin, 1-(5-Amino-2-methylpentyl)-2,3-dicyclo-hexylguanidin, 1-(6-Aminohexyl)-2,3-diisopropylguanidin, 1-(6-Aminohexyl)-2,3-dicyclohexylguanidin, 1-(6-Amino-2,2(4),4-trimethyl-hexyl)-2,3-diisopropyl-guanidin, 1-(6-Amino-2,2(4),4-trimethylhexyl)-2,3-dicyclohexylguanidin, 1-(6-Amino-3,3(5),5-trimethylhexyl)-2,3-diisopropylguanidin, 1-(6-Amino-3,3(5),5-trimethylhexyl)-2,3-dicyclohexylguanidin, 1-(8-Aminooctyl)-2,3-diisopropylguanidin, 1-(8-Aminooctyl)-2,3-dicyclohexylguanidin, 1-(12-Aminododecyl)-2,3-diisopropylguanidin, 1-(12-Aminododecyl)-2,3-dicyclohexylguanidin, 1-(3-Aminomethyl-3,5,5-trimethylcyclohexyl)-2,3-diisopropylguanidin, 1-(3-Aminomethyl-3,5,5-trimethylcyclohexyl)-2,3-dicyclohexylguanidin, 1-(3-Amino-1,5,5-trimethyl-cyclohexylmethyl)-2,3-diisopropylguanidin, 1-(3-Amino-1,5,5-trimethylcyclohexylmethyl)-2,3-dicyclohexylguanidin, 1-(3-Aminomethylcyclohexyl)-2,3-diisopropylguanidin, 1-(3-Aminomethylcyclohexyl)-2,3-dicyclohexylguanidin, 1-(3-Aminomethylbenzyl)-2,3-diisopropylguanidin, 1-(3-Aminomethylbenzyl)-2,3-dicyclo-hexylguanidin, 1-(3-Amino-2(4)-methylcyclohexyl)-2,3-diisopropylguanidin, 1-(3-Amino-2(4)-methylcyclohexyl)-2,3-dicyclohexylguanidin, 1,1'-(3-Aza-1,5-pentylen)bis(2,3-diisopropylguanidin), 1-(Piperidin-4-ylmethyl)-2,3-diisopropyl-guanidin, 1-(Piperidin-4-ylmethyl)-2,3-dicyclohexylguanidin, 1-(2-(Piperidin-4-yl)ethyl)-2,3-diisopropylguanidin, 1-(2-(Piperidin-4-yl)ethyl)-2,3-dicyclohexyl-guanidin, 1-(2-Piperazinoethyl)-2,3-diisopropylguanidin, 1-(2-Piperazinoethyl)-2,3-dicyclohexylguanidin, 1,1'-(3-Aza-1,5-pentylen)bis(2,3-dicyclohexylguanidin), 1,1'-(4-Aza-1,7-heptylen)bis(2,3-diisopropylguanidin), 1,1'-(4-Aza-1,7-heptylen)bis(2,3-dicyclohexylguanidin), 1,1'-(3-Aza-1,6-hexylen)bis(2,3-diisopropylguanidin), 1,1'-(3-Aza-1,6-hexylen)bis(2,3-dicyclohexylguanidin), 1,1'-(7-Aza-1,13-tridecylen)bis(2,3-diisopropylguanidin), 1,1'-(7-Aza-1,13-tridecylen)-bis(2,3-dicyclohexylguanidin), 1-(3-((3-aminopropyl)(methyl)amino)propyl)-2,3-diisopropylguanidin, 1-(3-((3-aminopropyl)(methyl)amino)propyl)-2,3-dicyclo-hexylguanidin, 1-(3-((3-Dimethylaminopropyl)annino)propyl)-2,3-diisopropylguanidin, 1-(3-((3-Dimethylaminopropyl)amino)propyl)-2,3-dicyclohexylguanidin, 1-(5-Amino-3-oxapentyl)-2,3-diisopropylguanidin, 1-(5-Amino-3-oxapentyl)-2,3-dicyclohexylguanidin, 1-(8-Amino-3,6-dioxadoctyl)-2,3-diisopropylguanidin, 1-(8-Amino-3,6-dioxadoctyl)-2,3-dicyclohexylguanidin, 1-(10-Amino-4,7-dioxadecyl)-2,3-diisopropylguanidin, 1-(10-Amino-4,7-dioxadecyl)-2,3-dicyclo-hexylguanidin, 1-(2-Hydroxyethyl)-2,3-diisopropylguanidin, 1-(2-Hydroxyethyl)-2,3-dicyclohexylguanidin, 1-(2-Hydroxypropyl)-2,3-diisopropylguanidin, 1-(2-Hydroxypropyl)-2,3-dicyclohexylguanidin, 1-(3-Hydroxypropyl)-2,3-diisopropylguanidin, 1-(3-Hydroxypropyl)-2,3-dicyclohexylguanidin, 1-(2-Hydroxy-1,1-dimethylethyl)-2,3-diisopropylguanidin, 1-(2-Hydroxy-1,1-dimethylethyl)-2,3-di-cyclohexylguanidin, 1-(5-Hydroxypentyl)-2,3-diisopropylguanidin, 1-(5-Hydroxy-pentyl)-2,3-dicyclohexylguanidin, 1-(6-Hydroxyhexyl)-2,3-diisopropylguanidin, 1-(6-Hydroxyhexyl)-2,3-dicyclohexylguanidin, 1-(3-Hydroxy-1,5,5-trimethyl-cyclohexylmethyl)-2,3-diisopropylguanidin, 1-(3-Hydroxy-1,5,5-trimethylcyclo-hexylmethyl)-2,3-dicyclohexylguanidin, 1-(2-(2-Hydroxyethoxy)ethyl)-2,3-diisopropylguanidin, 1-(2-(2-Hydroxyethoxy)ethyl)-2,3-dicyclohexylguanidin, 1-((2-(2-Hydroxyethoxy)ethoxy)ethyl)-2,3-diisopropylguanidin, 1-((2-(2-Hydroxy-ethoxy)ethoxy)ethyl)-2,3-dicyclohexylguanidin, 1-(ω-2-Aminopropyl-polyoxypropylen)-2,3-diisopropylguanidin mit einem Molekulargewicht im Bereich von etwa 320 bis 400 g/mol und 1-(ω-2-Aminopropyl-polyoxypropylen)-2,3-dicyclo-hexylguanidin mit einem Molekulargewicht im Bereich von etwa 400 bis 500 g/mol.

Davon bevorzugt sind die Guanidine mit einer primären oder sekundären Aminogruppe. Sie lassen sich besonders leicht mit funktionellen Verbindungen umsetzten und ermöglichen Katalysatoren der Formel (I) mit besonders hoher Aktivität.
Davon bevorzugt sind weiterhin die Guanidine mit einer Hydroxylgruppe oder einer sekundären Aminogruppe. Sie lassen sich besonders rein herstellen. Bevorzugt sind weiterhin Guanidine mit einer tertiären Aminogruppe. Sie ermöglichen Katalysatoren der Formel (I) mit besonders hoher Aktivität.

Ein Katalysator der Formel (I) kann auch erhalten werden, indem in einem ersten Schritt ein Amin der Formel (III) mit einer der beschriebenen funktionellen Verbindungen unter Reaktion mindestens einer deren Reaktivgruppen mit der HX-Gruppe zu einem Amin-Zwischenprodukt umgesetzt wird, und dieses in einem zweiten Schritt mit dem Reagens zur Einführung von Guanidin-Gruppen zu einem Katalysator der Formel (I) umgesetzt wird.
Diese Herstellung ist insbesondere geeignet für Amine, bei welchen X für NH₂ steht, also für Polyamine, welche zwei primäre Aminogruppen aufweisen.
Der erste Schritt wird dabei bevorzugt so geführt, dass die primären Aminogruppen des Amins gegenüber den Reaktivgruppen der funktionellen Verbindung im stöchiometrischen Überschuss vorhanden sind, so dass ein Amin-Zwischenprodukt mit einer primären Aminogruppe entsteht.
Die Reaktionsbedingungen für die an den jeweiligen Umsetzungen beteiligten Reagentien sind dabei bevorzugt dieselben wie bereits beschrieben.

In einem bevorzugten Katalysator der Formel (I) steht p für p¹, r für 0, Y für X², Q' für 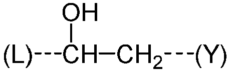 und L für L¹. Dieser weist somit die Formel (Ia) auf, 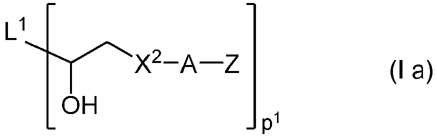 wobei
p¹ für eine ganze Zahl von 1 bis 4 steht,
L¹ für einen p¹-wertigen Kohlenwasserstoffrest mit einem mittleren Molekulargewicht im Bereich von 15 bis 1'500 g/mol, welcher insbesondere eine oder mehrere Ethergruppen und gegebenenfalls eine Alkoxysilangruppe aufweist, steht,
X² für S oder NR³ steht,
und A, R³ und Z die beschriebenen Bedeutungen aufweisen.
Bevorzugt steht p¹ für 1 oder 2 oder 3, besonders bevorzugt für 1 oder 2. Bevorzugt steht X² für NR³.
Ein Katalxsator der Formel (Ia) wird insbesondere erhalten aus der Umsetzung von mindestens einem Guanidin der Formel (II), bei welchem X für X² steht, mit mindestens einer der beschriebenen funktionellen Verbindungen mit mindestens einer Epoxidgruppe. Die Umsetzung wird bevorzugt bei einer Temperatur im Bereich von 20 °C bis 140 °C, insbesondere 40 °C bis 120 °C, durchgeführt.

In einem weiteren bevorzugten Katalysator der Formel (I) steht p für p², r für 0, Y für X¹, Q' für 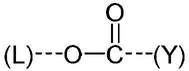 und L für L². Dieser weist somit die Formel (Ib) auf, 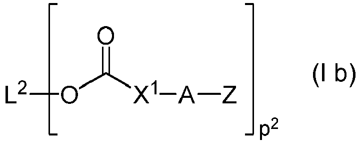 wobei
p² für 1 oder 2 steht,
X¹ für O oder NR³ steht,
L² für einen Alkylrest mit 1 bis 18 C-Atomen steht,
und A, R³ und Z die beschriebenen Bedeutungen aufweisen.
Bevorzugt steht p² für 1.
Bevorzugt weist L² ein Molekulargewicht im Bereich von 45 bis 185 g/mol auf. Bevorzugt steht L² für einen Hydroxyalkyl-Rest mit 2 bis 8 C-Atomen. Besonders bevorzugt steht L² für 2-Hydroxyethyl, 2-Hydroxypropyl oder 3-Hydroxypropyl, insbesondere für 2-Hydroxypropyl.
Ein Katalysator der Formel (Ib) wird insbesondere erhalten aus der Umsetzung von mindestens einem Guanidin der Formel (II), bei welchem X für O oder NR³ steht, mit mindestens einem Dialkylcarbonat. Die Umsetzung wird bevorzugt bei einer Temperatur im Bereich von 20 °C bis 140 °C, insbesondere 40 °C bis 120 °C, durchgeführt und freigesetzte Alkohole oder Phenole werden bevorzugt während oder nach der Umsetzung entfernt, insbesondere durch Destillation unter Vakuum.

In einem weiteren bevorzugten Katalysator der Formel (I) steht p für p³, r für 0, Y für X¹, Q' für 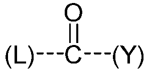 und L für L³. Dieser weist somit die Formel (Ic) auf, 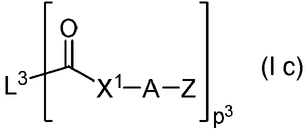 wobei
p³ für 1 oder 2 oder 3 steht,
X¹ für O oder NR³ steht,
L³ für eine kovalente Bindung oder für einen Wasserstoff-Rest oder für einen p³-wertigen Kohlenwasserstoffrest mit einem mittleren Molekulargewicht im Bereich von 14 bis 500 g/mol, welcher gegebenenfalls ungesättigte Anteile und gegebenenfalls Ethergruppen oder Estergruppen aufweist, steht,
und A, R³ und Z die beschriebenen Bedeutungen aufweisen.
Bevorzugt steht p³ für 1 oder 2, insbesondere für 1.
Ein Katalysator der Formel (Ic) wird insbesondere erhalten aus der Umsetzung von mindestens einem Guanidin der Formel (II), bei welchem X für O oder für NR³ steht, mit mindestens einer der beschriebenen funktionellen Verbindungen mit mindestens einer Carbonsäureester-Gruppe. Die Umsetzung wird bevorzugt bei einer Temperatur im Bereich von 20 °C bis 160 °C, insbesondere 40 °C bis 140 °C, durchgeführt. Die bei der Umsetzung entstehenden Abspalter in Form von Alkoholen oder Phenolen werden bevorzugt während oder nach der Umsetzung entfernt, insbesondere durch Destillation unter Vakuum.

In einem weiteren bevorzugten Katalysator der Formel (I) steht p für 1, r für 0, Y für X¹, Q' für 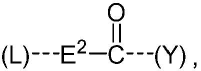 E² für CH(OH)-(CH₂)_{b} oder für CH(OH)-(CH₂)_{c}O und L für L⁴. Dieser weist somit die Formel (Id) oder (Ie) auf, 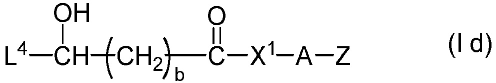 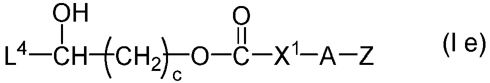 wobei
X¹ für O oder NR³ steht,
b für eine ganze Zahl im Bereich von 1 bis 4 steht,
c für 1 oder 2 steht,
L⁴ für einen Wasserstoff-Rest oder für einen linearen Alkylrest mit 1 bis 12 C-Atomen steht,
und A, R³ und Z die beschriebenen Bedeutungen aufweisen.
Bevorzugt steht b für 2 oder 3 oder 4.
Bevorzugt steht c für 1.
Im Fall von Formel (Id) steht L⁴ bevorzugt für einen Wasserstoff-Rest oder für einen linearen Alkylrest mit 1 bis 8 C-Atomen, insbesondere für einen Wasserstoff-Rest oder für einen Methylrest.
Im Fall von Formel (Ie) steht L⁴ bevorzugt für einen Wasserstoff-Rest oder für einen Methyl-Rest, insbesondere für einen Methyl-Rest.
Ein Katalysator der Formel (Id) wird insbesondere erhalten aus der Umsetzung von mindestens einem Guanidin der Formel (II), bei welchem X für O oder NR³ steht, mit mindestens einem der beschriebenen Lactone.
Ein Katalysator der Formel (Ie) wird insbesondere erhalten aus der Umsetzung von mindestens einem Guanidin der Formel (II), bei welchem X für O oder NR³ steht, mit mindestens einem der beschriebenen cyclischen Carbonate.
Die Umsetzung wird bevorzugt bei einer Temperatur im Bereich von 20 °C bis 140 °C, insbesondere 40 °C bis 120 °C, durchgeführt.

In einem weiteren bevorzugten Katalysator der Formel (I) steht p für p⁵, r für 0, Y für N, Q' für 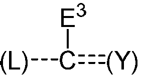 und L für L⁵. Dieser weist somit die Formel (If) auf, 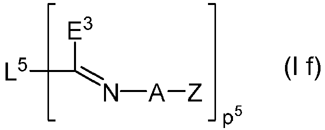 wobei
p⁵ für 1 oder 2 oder 3 steht,
L⁵ für einen einwertigen Kohlenwasserstoff-Rest mit 1 bis 20 C-Atomen steht, welcher gegebenenfalls Heteroatome, insbesondere Sauerstoff oder Stickstoff in Form von Estergruppen, Amidgruppen, Ethergruppen oder tertiären Aminogruppen, enthält, oder zusammen mit E³ für einen gegebenenfalls substituierten 1,4-Butylen- oder 1,5-Pentylen-Rest steht,
und A und Z die beschriebenen Bedeutungen aufweisen.
Bevorzugt steht p⁵ für 1 oder 2, insbesondere für 1.
Bevorzugt steht E³ für einen Wasserstoff-Rest oder für einen Methyl-Rest.
Für den Fall, dass E³ für einen Wasserstoff-Rest steht, steht L⁵ bevorzugt für Prop-2-yl, Hept-3-yl, Undec-1-yl, Phenyl, 1,1-Dimethyl-2-acetoxyeth-1-yl, 1,1-Dimethyl-2-lauroyloxyeth-1-yl, 1,1-Dimethyl-2-(N-morpholino)eth-1-yl, 2-Bis-(methoxyethyl)amino-1,1-dimethyleth-1-yl oder 2-Bis(2-hydroxypropyl)amino-1,1-dimethyleth-1-yl.
Für den Fall, dass E³ für einen Methyl-Rest steht, steht L⁵ bevorzugt für Methyl, Ethyl, Prop-2-yl, 2-Methylprop-1-yl, Benzyl.
Ein Katalysator der Formel (If) wird insbesondere erhalten aus der Umsetzung von mindestens einem Guanidin der Formel (II), bei welchem X für NH steht, mit mindestens einer der beschriebenen funktionellen Verbindungen mit mindestens einer Keton- oder Aldehydgruppe. Die Umsetzung wird bevorzugt bei einer Temperatur im Bereich von 0 °C bis 120 °C, insbesondere 20 °C bis 100 °C, durchgeführt. Das bei der Umsetzung entstehende Wasser wird bevorzugt während oder nach der Umsetzung entfernt, insbesondere durch Azeotropdestillation oder Destillation unter Vakuum.

In einem weiteren bevorzugten Katalysator der Formel (I) steht p für p⁶, r für 0, Y für X¹, Q' für 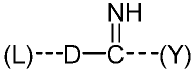 und L für L⁶. Dieser weist somit die Formel (Ig) auf, 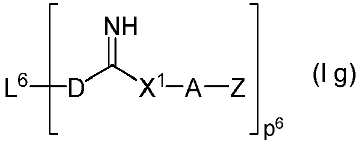 wobei
p⁶ für eine ganze Zahl von 1 bis 3 steht,
X¹ für O oder NR³ steht,
L⁶ für einen p⁶-wertigen Kohlenwasserstoffrest mit einem mittleren Molekulargewicht im Bereich von 77 bis 5'000 g/mol, welcher gegebenenfalls Heteroatome, insbesondere Sauerstoff in der Form von Ethergruppen oder Estergruppen, oder Fluoratome aufweist, steht,
und D, A, R³ und Z die beschriebenen Bedeutungen aufweisen.
Bevorzugt steht p⁶ für 2 oder 3, insbesondere für 2.
Ein Katalysator der Formel (Ig) wird insbesondere erhalten aus der Umsetzung von mindestens einem Guanidin der Formel (II), bei welchem X für O oder für NR³ steht, mit mindestens einer der beschriebenen funktionellen Verbindungen mit mindestens einer Cyanat- oder Thiocyanatgruppe. Die Umsetzung wird bevorzugt bei einer Temperatur im Bereich von 20 °C bis 150 °C durchgeführt.

In einem weiteren bevorzugten Katalysator der Formel (I) steht p für p⁷, r für 0, Y für X¹, Q' für 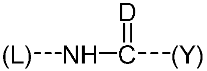 und L für L⁷. Dieser weist somit die Formel (Ih) auf, 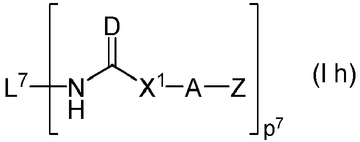 wobei
p⁷ für eine ganze Zahl von 1 bis 4 steht,
X¹ für O oder NR³ steht,
L⁷ für einen p⁷-wertigen Kohlenwasserstoffrest mit einem mittleren Molekulargewicht im Bereich von 15 bis 20'000 g/mol, welcher gegebenenfalls Heteroatome, insbesondere in der Form von Ethergruppen, Estergruppen, Amidgruppen, Urethangruppen, Isocyanuratgruppen, Cyanuratgruppen, Isocyanatgruppen oder Alkoxysilangruppen aufweist, steht,
und D, A, R³ und Z die beschriebenen Bedeutungen aufweisen.
Bevorzugt steht p⁷ für 1 oder 2 oder 3.
Ein Katalysator der Formel (Ih) wird insbesondere erhalten aus der Umsetzung von mindestens einem Guanidin der Formel (II), bei welchem X für O oder für NR³ steht, mit mindestens einer der beschriebenen funktionellen Verbindungen mit mindestens einer Isocyanat- oder Isothiocyanatgruppe. Die Umsetzung wird bevorzugt bei einer Temperatur im Bereich von 0 °C bis 120 °C durchgeführt. Für den Fall, dass X für NR³ steht, ist eine Temperatur im Bereich von 0 °C bis 80 °C bevorzugt. Für den Fall, dass X für O steht, ist eine Temperatur im Bereich von 40 °C bis 120 °C bevorzugt.

In einem weiteren bevorzugten Katalysator der Formel (I) steht p für p⁸, r für 0, Y für NR³, Q' für 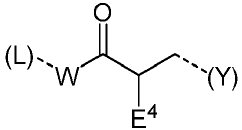 und L für L⁸. Dieser weist somit die Formel (Ii) auf, 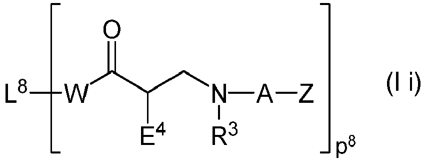 wobei
p⁸ für eine ganze Zahl von 1 bis 3 steht,
L⁸ für einen Wasserstoff-Rest oder einen p⁸-wertigen Kohlenwasserstoffrest mit einem mittleren Molekulargewicht im Bereich von 14 bis 20'000 g/mol, welcher gegebenenfalls Heteroatome, insbesondere in Form von Ethergruppen, Estergruppen, Urethangruppen, Isocyanuratgruppen, Cyanuratgruppen oder Alkoxysilangruppen aufweist, steht,
und W, E⁴, A, R³ und Z die beschriebenen Bedeutungen aufweisen. Bevorzugt steht p⁸ für 1 oder 2, insbesondere für 1.
Ein Katalysator der Formel (Ii) wird insbesondere erhalten aus der Umsetzung von mindestens einem Guanidin der Formel (II), bei welchem X für NR³ steht, mit mindestens einer der beschriebenen funktionellen Verbindungen mit mindestens einer Acrylat-, Methacrylat-, Acrylamid-, Methacrylamid-, Fumarat-, Fumaramid-, Itaconat- oder Itaconamid-Gruppe. Die Umsetzung wird bevorzugt bei einer Temperatur im Bereich von 0 °C bis 140 °C, insbesondere 20 °C bis 120 °C, durchgeführt.

In einem weiteren bevorzugten Katalysator der Formel (I) steht p für p⁹, r für 0, Y für N, Q' für 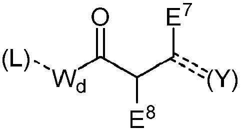 und L für L⁹. Dieser weist somit die Formel (Ij) auf, 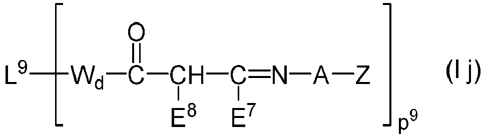 wobei
p⁹ für 1 oder 2 oder 3 steht,
L⁹ für einen p⁹-wertigen Kohlenwasserstoffrest mit einem mittleren Molekulargewicht im Bereich von 15 bis 5'000 g/mol, welcher gegebenenfalls Heteroatome, insbesondere in der Form von Ethergruppen, Estergruppen, aufweist, steht,
und d, W, E⁷, E⁸, A und Z die beschriebenen Bedeutungen aufweisen. Bevorzugt steht p⁹ für 1 oder 2, insbesondere für 1.
Ein Katalysator der Formel (Ij) wird insbesondere erhalten aus der Umsetzung von mindestens einem Guanidin der Formel (II), bei welchem X für NH steht, mit mindestens einer der beschriebenen funktionellen Verbindungen mit mindestens einer 1,3-Diketon- oder 1,3-Ketoester- oder 1,3-Ketoamid-Gruppe. Die Umsetzung wird bevorzugt bei einer Temperatur im Bereich von 0 °C bis 120 °C, insbesondere 20 °C bis 100 °C, durchgeführt. Das bei der Umsetzung entstehende Wasser wird bevorzugt während oder nach der Umsetzung entfernt, insbesondere durch Azeotropdestillation oder Destillation unter Vakuum.

Der Katalysator der Formel (I) wird verwendet für die Vernetzung einer härtbaren Zusammensetzung, wo er die Vernetzung bzw. Aushärtung der Zusammensetzung beschleunigt.

Als härtbare Zusammensetzung geeignet ist insbesondere
- eine Epoxidharz-Zusammensetzung, insbesondere heisshärtende, über Dicyandiamid oder Carbonsäuren oder Carbonsäureanhydride vernetzende Systeme, wie sie beispielsweise in Klebstoffen, Beschichtungen und Giessharzen eingesetzt werden;
- oder eine Polyurethan-Zusammensetzung, insbesondere zweikomponentige, durch Reaktion von Polyolen mit Isocyanaten vernetzende Systeme, wie sie beispielsweise für Klebstoffe, Beläge, Vergussmassen, Dichtfugen, Formkörper oder Blockschäume eingesetzt werden, sowie einkomponentige Systeme mit blockierten Isocyanatgruppen oder blockierten Aminogruppen, wie sie beispielsweise in Pulverlacken, Coil Coatings, Elektrotauchlacken und Flüssiglacken eingesetzt werden;
- oder eine Epoxidharz-Polyurethan-Zusammensetzung;
- oder eine Cyanatesterharz-Zusammensetzung;
- oder eine Silangruppen-haltige Zusammensetzung.

Besonders vorteilhaft ist die Verwendung zur Vernetzung von Silangruppen-haltigen Zusammensetzungen, insbesondere von Zusammensetzungen auf Basis von Silangruppen-haltigen Polymeren. Zusammensetzungen auf Basis von Silangruppen-haltigen Polymeren härten auch bei relativ geringer Katalysator-Konzentration rasch aus und neigen nicht zu migrationsbedingten Fehlern wie Separation, Ausschwitzen oder Substratverschmutzung.

Das Silangruppen-haltige Polymer ist dabei insbesondere ausgewählt aus der Gruppe bestehend aus Polyorganosiloxanen mit endständigen Silangruppen und Silangruppen-haltigen organischen Polymeren, wie sie im Folgenden genauer beschrieben werden.

Ein Polyorganosiloxan mit endständigen Silangruppen weist den Vorteil auf, dass es im ausgehärteten Zustand besonders wasser- und lichtbeständig ist und besonders weichelastische Eigenschaften ermöglicht.
Ein Silangruppen-haltiges organisches Polymer weist den Vorteil auf, dass es besonders gute Haftungseigenschaften auf einer Vielzahl von Untergründen aufweist und besonders kostengünstig ist.

Ein weiterer Gegenstand der Erfindung ist somit eine härtbare Zusammensetzung enthaltend mindestens einen Katalysator der Formel (I).

Bevorzugt stellt die härtbare Zusammensetzung einen Klebstoff oder einen Dichtstoff oder eine Beschichtung dar.

Bevorzugt enthält die härtbare Zusammensetzung weiterhin mindestens ein Silangruppen-haltiges Polymer.
Eine solche Zusammensetzung verfügt typischerweise über eine gute Lagerfähigkeit ohne Separationsneigung, erlaubt aufgrund der geringen Toxizität und geringen Flüchtigkeit des Katalysators der Formel (I) eine niedrige Gefahreneinstufung und ermöglicht emissions- und geruchsarme Produkte, welche rasch aushärten und dabei ein mechanisch hochwertiges und beständiges Material bilden. Besonders vorteilhaft ist dabei der Umstand, dass dieses Material kaum zu migrationsbedingten Fehlern wie Ausschwitzen oder Substratverschmutzung neigt, im Gegensatz zu Zusammensetzungen mit Katalysatoren nach dem Stand der Technik, wie beispielsweise DBU oder TMG. Zusammensetzungen enthaltend solche aus dem Stand der Technik bekannten Katalysatoren neigen zu Migrationseffekten, was sich vor der Aushärtung durch Separation und nach der Aushärtung durch klebrige und/oder schmierige Oberflächen und/oder Substratverschmutzungen äussern kann. Besonders letztere Effekte sind äusserst unerwünscht, da klebrige und schmierige Oberflächen schnell verschmutzen und schlecht überstreichbar sind, und Substratverunreinigungen zu bleibenden Verfärbungen führen können.

Das Silangruppen-haltige Polymer ist in einer bevorzugten Ausführungsform ein Polyorganosiloxan mit endständigen Silangruppen.
Ein bevorzugtes Polyorganosiloxan mit endständigen Silangruppen weist die Formel (IV) auf, 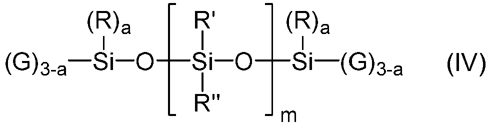 wobei
R, R' und R" unabhängig voneinander jeweils für einen einwertigen Kohlenwasserstoff-Rest mit 1 bis 12 C-Atomen stehen;
G für einen Hydroxyl-Rest oder für einen Alkoxy-, Acetoxy-, Ketoximato-, Amido- oder Enoxy-Rest mit 1 bis 13 C-Atomen steht;
a für 0, 1 oder 2 steht; und
m für eine ganze Zahl im Bereich von 50 bis etwa 2'500 steht.

R steht bevorzugt für Methyl, Vinyl oder Phenyl.
R' und R" stehen bevorzugt unabhängig voneinander jeweils für einen Alkylrest mit 1 bis 5, bevorzugt 1 bis 3 C-Atomen, insbesondere für Methyl.
G steht bevorzugt für einen Hydroxyl-Rest oder für einen Alkoxy- oder Ketoximato-Rest mit 1 bis 6 C-Atomen, insbesondere für einen Hydroxyl-, Methoxy-, Ethoxy-, Methylethylketoximato- oder Methylisobutylketoximato-Rest. Besonders bevorzugt steht G für einen Hydroxylrest.
a steht bevorzugt für 0 oder 1, insbesondere für 0.
Weiterhin ist m bevorzugt so gewählt, dass das Polyorganosiloxan der Formel (IV) bei Raumtemperatur eine Viskosität im Bereich von 100 bis 500'000 mPa·s, insbesondere von 1000 bis 100'000 mPa·s, aufweist.

Polyorganosiloxane der Formel (IV) sind gut handhabbar und vernetzen mit Feuchtigkeit und/oder Silanvernetzern zu festen Silicon-Polymeren mit elastischen Eigenschaften.
Geeignete kommerziell erhältliche Polyorganosiloxane der Formel (IV) sind beispielsweise erhältlich von Wacker, Momentive Performance Material, GE Advanced Materials, Dow Corning, Bayer oder Shin Etsu.

Bevorzugt enthält die Zusammensetzung zusätzlich zum Polyorganosiloxan mit endständigen Silangruppen einen Silanvernetzer, insbesondere ein Silan der Formel (V),

(R"')_{q}-Si-(G')_{4-q} (V)

wobei
R'" für einen einwertigen Kohlenwasserstoff-Rest mit 1 bis 12 C-Atomen steht, G' für einen Hydroxyl-Rest oder für einen Alkoxy-, Acetoxy-, Ketoximato-, Amido- oder Enoxy-Rest mit 1 bis 13 C-Atomen steht; und
q für einen Wert von 0, 1 oder 2, insbesondere für 0 oder 1, steht.

Besonders geeignete Silane der Formel (V) sind Methyltrimethoxysilan, Ethyltrimethoxysilan, Propytrimethoxysilan, Vinyltrimethoxysilan, Methyltriethoxysilan, Vinyltriethoxysilan, Phenyltriethoxysilan, Tetramethoxysilan, Tetraethoxysilan, Methyltris(methylethylketoximo)silan, Vinyltris(methylethylketoximo)silan und Methyltris(isobutylketoximo)silan.

Das Silangruppen-haltige Polymer ist in einer weiteren bevorzugten Ausführungsform ein Silangruppen-haltiges organisches Polymer, insbesondere ein Polyolefin, Polyester, Polyamid, Poly(meth)acrylat oder Polyether oder eine Mischform dieser Polymere, welches jeweils eine oder bevorzugt mehrere Silangruppen trägt. Die Silangruppen können seitlich in der Kette oder endständig stehen und sind über ein C-Atom an das organische Polymer gebunden. Besonders bevorzugt ist das Silangruppen-haltige organische Polymer ein Silangruppen-haltiges Polyolefin oder ein Silangruppen-haltiger Polyester oder ein Silangruppen-haltiges Poly(meth)acrylat oder ein Silangruppen-haltiger Polyether oder eine Mischform dieser Polymere.
Am meisten bevorzugt ist das Silangruppen-haltige organische Polymer ein Silangruppen-haltiger Polyether.

Das Silangruppen-haltige organische Polymer weist als Silangruppen bevorzugt Alkoxysilangruppen auf, insbesondere Alkoxysilangruppen der Formel (VI), 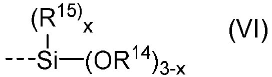 wobei
R¹⁴ für einen linearen oder verzweigten, einwertigen Kohlenwasserstoffrest mit 1 bis 5 C-Atomen, insbesondere für Methyl oder für Ethyl oder für Isopropyl, steht;
R¹⁵ für einen linearen oder verzweigten, einwertigen Kohlenwasserstoffrest mit 1 bis 8 C-Atomen, insbesondere für Methyl oder für Ethyl, steht; und x für einen Wert von 0 oder 1 oder 2, bevorzugt für 0 oder 1, insbesondere für 0, steht.
Besonders bevorzugt steht R¹⁴ für Methyl oder für Ethyl.
Für bestimmte Anwendungen steht der Rest R¹⁴ bevorzugt für eine Ethylgruppe, da in diesem Fall bei der Aushärtung der Zusammensetzung ökologisch und toxikologisch harmloses Ethanol freigesetzt wird.
Besonders bevorzugt sind Trimethoxysilangruppen, Dimethoxymethylsilangruppen oder Triethoxysilangruppen.
Dabei weisen Methoxysilangruppen den Vorteil auf, dass sie besonders reaktiv sind, und Ethoxysilangruppen weisen den Vorteil auf, dass sie toxikologisch vorteilhaft und besonders lagerstabil sind.

Das Silangruppen-haltige organische Polymer weist im Mittel bevorzugt 1.3 bis 4, insbesondere 1.5 bis 3, besonders bevorzugt 1.7 bis 2.8, Silangruppen pro Molekül auf. Die Silangruppen sind bevorzugt endständig.
Das Silangruppen-haltige organische Polymer weist bevorzugt ein mittleres Molekulargewicht, bestimmt mittels GPC gegenüber Polystyrol-Standard, im Bereich von 1'000 bis 30'000 g/mol, insbesondere von 2'000 bis 20'000 g/mol, auf. Das Silangruppen-haltige organische Polymer weist bevorzugt ein Silan-Equivalentgewicht von 300 bis 25'000 g/Eq, insbesondere von 500 bis 15'000 g/Eq, auf.

Das Silangruppen-haltige organische Polymer kann bei Raumtemperatur fest oder flüssig vorliegen. Bevorzugt ist es bei Raumtemperatur flüssig.

Meist bevorzugt handelt es sich beim Silangruppen-haltigen organischen Polymer um einen bei Raumtemperatur flüssigen Silangruppen-haltigen Polyether, wobei die Silangruppen insbesondere Dialkoxysilangruppen und/oder Trialkoxysilangruppen, besonders bevorzugt Trimethoxysilangruppen oder Triethoxysilangruppen, sind.

Verfahren zur Herstellung von Silangruppen-haltigen Polyethern sind dem Fachmann bekannt.
In einem bevorzugten Verfahren sind Silangruppen-haltige Polyether erhältlich aus der Umsetzung von Allylgruppen-haltigen Polyethern mit Hydrosilanen, gegebenenfalls unter Kettenverlängerung mit beispielsweise Diisocyanaten.
In einem weiteren bevorzugten Verfahren sind Silangruppen-haltige Polyether erhältlich aus der Copolymerisation von Alkylenoxiden und Epoxysilanen, gegebenenfalls unter Kettenverlängerung mit beispielsweise Diisocyanaten.
In einem weiteren bevorzugten Verfahren sind Silangruppen-haltige Polyether erhältlich aus der Umsetzung von Polyetherpolyolen mit Isocyanatosilanen, gegebenenfalls unter Kettenverlängerung mit Diisocyanaten.
In einem weiteren bevorzugten Verfahren sind Silangruppen-haltige Polyether erhältlich aus der Umsetzung von Isocyanatgruppen-haltigen Polyethern, insbesondere NCO-terminierten Urethan-Polyethern aus der Umsetzung von Polyetherpolyolen mit einer überstöchiometischen Menge an Polyisocyanaten, mit Aminosilanen, Hydroxysilanen oder Mercaptosilanen. Silangruppen-haltige Polyether aus diesem Verfahren sind besonders bevorzugt. Dieses Verfahren ermöglicht den Einsatz einer Vielzahl von kommerziell gut verfügbaren, kostengünstigen Ausgangsmaterialien, womit unterschiedliche Polymereigenschaften erhalten werden können, beispielsweise eine hohe Dehnbarkeit, eine hohe Festigkeit, ein niedriges Elastizitätsmodul, ein tiefer Glasübergangspunkt oder eine hohe Witterungsbeständigkeit.

Besonders bevorzugt ist der Silangruppen-haltige Polyether erhältlich aus der Umsetzung von NCO-terminierten Urethan-Polyethern mit Aminosilanen oder Hydroxysilanen. Geeignete NCO-terminierte Urethan-Polyether sind erhältlich aus der Umsetzung von Polyetherpolyolen, insbesondere Polyoxyalkylendiolen oder Polyoxyalkylentriolen, bevorzugt Polyoxypropylendiolen oder Polyoxypropylentriolen, mit einer überstöchiometrischen Menge an Polyisocyanaten, insbesondere Diisocyanaten.
Bevorzugt wird die Umsetzung zwischen dem Polyisocyanat und dem Polyetherpolyol unter Feuchtigkeitsausschluss bei einer Temperatur von 50 °C bis 160 °C durchgeführt, gegebenenfalls in Anwesenheit geeigneter Katalysatoren, wobei das Polyisocyanat so dosiert ist, dass dessen Isocyanatgruppen im Verhältnis zu den Hydroxylgruppen des Polyols im stöchiometrischen Überschuss vorhanden sind. Insbesondere wird der Überschuss an Polyisocyanat so gewählt, dass im resultierenden Urethan-Polyether nach der Umsetzung aller Hydroxylgruppen ein Gehalt an freien Isocyanatgruppen von 0.1 bis 5 Gewichts-%, bevorzugt 0.2 bis 4 Gewichts-%, besonders bevorzugt 0.3 bis 3 Gewichts-%, bezogen auf das gesamte Polymer, verbleibt.
Bevorzugte Diisocyanate sind ausgewählt aus der Gruppe bestehend aus 1,6-Hexamethylendiisocyanat (HDI), 1-Isocyanato-3,3,5-trimethyl-5-isocyanatomethyl-cyclohexan (=Isophorondiisocyanat oder IPDI), 2,4- und 2,6-Toluylendiisocyanat und beliebige Gemische dieser Isomeren (TDI) und 4,4'-, 2,4'- und 2,2'-Diphenylmethandiisocyanat und beliebige Gemische dieser Isomeren (MDI). Besonders bevorzugt sind IPDI oder TDI. Meist bevorzugt ist IPDI. Damit werden Silangruppen-haltige Polyether mit besonders guter Lichtechtheit erhalten.

Speziell geeignet als Polyetherpolyole sind Polyoxyalkylendiole oder Polyoxyalkylentriole mit einem Ungesättigtheitsgrad tiefer als 0.02 mEq/g, insbesondere tiefer als 0.01 mEq/g, und einem mittleren Molekulargewicht im Bereich von 400 bis 25'000 g/mol, insbesondere 1000 bis 20'000 g/mol.
Neben Polyetherpolyolen können anteilig auch andere Polyole eingesetzt werden, insbesondere Polyacrylatpolyole, sowie niedrigmolekulare Diole oder Triole.

Geeignete Aminosilane für die Umsetzung mit einem NCO-terminierten Urethan-Polyether sind primäre und sekundäre Aminosilane. Bevorzugt sind 3-Aminopropyltrimethoxysilan, 3-Aminopropyldimethoxymethylsilan, 4-Aminobutyl-trimethoxysilan, 4-Amino-3-methylbutyl-trimethoxysilan, 4-Amino-3,3-dimethylbutyl-trimethoxysilan, N-Butyl-3-aminopropyltrimethoxysilan, N-Phenyl-3-aminopropyltrimethoxysilan, Addukte aus primären Aminosilanen wie 3-Aminopropyltrimethoxysilan, 3-Aminopropyldimethoxymethylsilan oder N-(2-Aminoethyl)-3-aminopropyltrimethoxysilan und Michael-Akzeptoren wie Acrylnitril, (Meth)acrylsäureestern, (Meth)acrylsäureamiden, Maleinsäure- oder Fumarsäurediestern, Citraconsäurediestern oder Itaconsäurediestern, insbesondere N-(3-Trimethoxysilylpropyl)amino-bernsteinsäuredimethyl- oder -diethylester. Ebenfalls geeignet sind Analoga der genannten Aminosilane mit Ethoxy- oder Isopropoxygruppen anstelle der Methoxygruppen am Silicium.

Geeignete Hydroxysilane für die Umsetzung mit einem NCO-terminierten Urethan-Polyether sind insbesondere erhältlich aus der Addition von Aminosilanen an Lactone oder an cyclische Carbonate oder an Lactide.
Dafür geeignete Aminosilane sind insbesondere 3-Aminopropyl-trimethoxysilan, 3-Aminopropyl-triethoxysilan, 4-Aminobutyl-trimethoxysilan, 4-Aminobutyl-triethoxysilan, 4-Amino-3-methylbutyl-trimethoxysilan, 4-Amino-3-methylbutyl-triethoxysilan, 4-Amino-3,3-dimethylbutyl-trimethoxysilan, 4-Amino-3,3-dimethylbutyl-triethoxysilan, 2-Aminoethyl-trimethoxysilan oder 2-Aminoethyltriethoxysilan. Besonders bevorzugt sind 3-Aminopropyl-trimethoxysilan, 3-Aminopropyl-triethoxysilan, 4-Amino-3,3-dimethylbutyl-trimethoxysilan oder 4-Amino-3,3-dimethylbutyl-triethoxysilan.
Als Lactone geeignet sind insbesondere γ-Valerolacton, γ-Octalacton, δ-Decalacton, und ε-Decalacton, insbesondere γ-Valerolacton.
Als cyclische Carbonate eignen sich insbesondere 4,5-Dimethyl-1,3-dioxolan-2-on, 4,4-Dimethyl-1,3-dioxolan-2-on, 4-Ethyl-1,3-dioxolan-2-on, 4-Methyl-1,3-dioxolan-2-on oder 4-(Phenoxymethyl)-1,3-dioxolan-2-on.
Als Lactide eignen sich insbesondere 1,4-Dioxan-2,5-dion (Lactid aus 2-Hydroxyessigsäure, auch "Glycolid" genannt), 3,6-Dimethyl-1,4-dioxan-2,5-dion (Lactid aus Milchsäure, auch "Lactid" genannt) und 3,6-Diphenyl-1,4-dioxan-2,5-dion (Lactid aus Mandelsäure).
Bevorzugte Hydroxysilane, welche auf diese Weise erhalten werden, sind N-(3-Triethoxysilylpropyl)-2-hydroxypropanamid, N-(3-Trimethoxysilylpropyl)-2-hydroxypropanamid, N-(3-Triethoxysilylpropyl)-4-hydroxypentanamid, N-(3-Triethoxysilylpropyl)-4-hydroxyoctanamid, N-(3-Triethoxysilylpropyl)-5-hydroxydecanamid und N-(3-Triethoxysilylpropyl)-2-hydroxypropylcarbamat.

Weiterhin sind geeignete Hydroxysilane auch erhältlich aus der Addition von Aminosilanen an Epoxide oder aus der Addition von Aminen an Epoxysilane. Bevorzugte Hydroxysilane, welche auf diese Weise erhalten werden, sind 2-Morpholino-4(5)-(2-trimethoxysilylethyl)cyclohexan-1-ol, 2-Morpholino-4(5)-(2-triethoxysilylethyl)cyclohexan-1-ol oder 1-Morpholino-3-(3-(triethoxysilyl)propoxy)propan-2-ol.

Als Silangruppen-haltige Polyether sind auch kommerziell erhältliche Produkte geeignet, insbesondere die Folgenden: MS Polymer™ (von Kaneka Corp.; insbesondere die Typen S203H, S303H, S227, S810, MA903 und S943); MS Polymer™ bzw. Silyl™ (von Kaneka Corp.; insbesondere die Typen SAT010, SAT030, SAT200, SAX350, SAX400, SAX725, MAX450, MAX951); Excestar® (von Asahi Glass Co. Ltd.; insbesondere die Typen S2410, S2420, S3430, S3630); SPUR+* (von Momentive Performance Materials; insbesondere die Typen 1010LM, 1015LM, 1050MM); Vorasil™ (von Dow Chemical Co.; insbesondere die Typen 602 und 604); Desmoseal® (von Bayer MaterialScience AG; insbesondere die Typen S XP 2458, S XP 2636, S XP 2749, S XP 2774 und S XP 2821), TEGOPAC® (von Evonik Industries AG; insbesondere die Typen Seal 100, Bond 150, Bond 250), Polymer ST (von Hanse Chemie AG/Evonik Industries AG, insbesondere die Typen 47, 48, 61, 61LV, 77, 80, 81); Geniosil® STP (von Wacker Chemie AG; insbesondere die Typen E10, E15, E30, E35).

Besonders bevorzugte Silangruppen-haltige organische Polymere weisen Endgruppen der Formel (VII) auf, 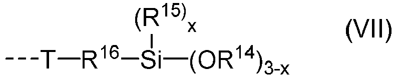 wobei
R¹⁶ für einen linearen oder verzweigten, zweiwertigen Kohlenwasserstoffrest mit 1 bis 12 C-Atomen, welcher gegebenenfalls cyclische und/oder aromatische Anteile und gegebenenfalls ein oder mehrere Heteroatome, insbesondere ein oder mehrere Stickstoffatome, aufweist, steht;
T für einen zweiwertigen Rest ausgewählt aus -O-, -S-, -N(R¹⁷)-, -O-CO-N(R¹⁷)-, -N(R¹⁷)-CO-O- und -N(R¹⁷)-CO-N(R¹⁷)- steht,
   wobei R¹⁷ für einen Wasserstoff-Rest oder für einen linearen oder verzweigten Kohlenwasserstoff-Rest mit 1 bis 20 C-Atomen, welcher gegebenenfalls cyclische Anteile aufweist, und welcher gegebenenfalls eine Alkoxysilan-, Ether- oder Carbonsäureestergruppe aufweist, steht; und
R¹⁴, R¹⁵ und x die bereits genannten Bedeutungen aufweisen.
Bevorzugt steht R¹⁶ für 1,3-Propylen oder für 1,4-Butylen, wobei Butylen mit einer oder zwei Methylgruppen substituiert sein kann.
Besonders bevorzugt steht R¹⁶ für 1,3-Propylen.

Bevorzugt ist der Katalysator der Formel (I) in der Zusammensetzung in einer solchen Menge vorhanden, dass die Konzentration an Guanidin-Gruppen bezogen auf die Menge des Silangruppen-haltigen Polymers im Bereich von 0.05 bis 20 mmol/100 g Polymer, bevorzugt 0.1 bis 15 mmol/100 g Polymer, insbesondere 0.1 bis 10 mmol/100 g, liegt.
Eine solche Zusammensetzung weist eine gute Lagerfähigkeit und eine schnelle Aushärtung auf.

Zusätzlich zum Katalysator der Formel (I) kann die Zusammensetzung weitere Katalysatoren, insbesondere für die Vernetzung von Silangruppen, enthalten. Als weitere Katalysatoren geeignet sind insbesondere Metall-Verbindungen und/oder basische Stickstoff- oder Phosphorverbindungen.
Geeignete Metall-Verbindungen sind insbesondere Verbindungen von Zinn, Titan, Zirkonium, Aluminium oder Zink, insbesondere Diorganozinn(IV)-Verbindungen wie insbesondere Dibutylzinn(IV)-diacetat, Dibutylzinn(IV)-dilaurat, Dibutylzinn(IV)-dineodecanoat oder Dibutylzinn(IV)-bis(acetylacetonat) und Dioctylzinn(IV)-dilaurat, sowie Titan(IV)- oder Zirkonium(IV)- oder Aluminium(III)- oder Zink(II)-Komplexe mit insbesondere Alkoxy-, Carboxylat-, 1,3-Diketonat-, 1,3-Ketoesterat- oder 1,3-Ketoamidat-Liganden.

Geeignete basische Stickstoff- oder Phosphorverbindungen sind insbesondere Imidazole, Pyridine, Phosphazen-Basen oder bevorzugt Amine, Hexahydrotriazine, Biguanide, Amidine oder weitere Guanidine.
Geeignete Amine sind insbesondere Alkyl-, Cycloalkyl- oder Aralkylamine wie Triethylamin, Triisopropylamin, 1-Butylamin, 2-Butylamin, tert.Butylamin, 3-Methyl-1-butylamin, 3-Methyl-2-butylamin, Dibutylamin, Tributylamin, Hexylamin, Dihexylamin, Cyclohexylamin, Dicyclohexylamin, Dimethylcyclohexylamin, Benzylamin, Dibenzylamin, Dimethylbenzylamin, Octylamin, 2-Ethylhexylamin, Di-(2-ethylhexyl)amin, Laurylamin, N,N-Dimethyl-laurylamin, Stearylamin, N,N-Dimethyl-stearylamin; von natürlichen Fettsäuregemischen abgeleitete Fettamine, wie insbesondere Cocoalkylamin, N,N-Dimethyl-cocoalkylamin, C₁₆₋₂₂-Alkylamin, N,N-Dimethyl-C₁₆₋₂₂-alkylamin, Soyaalkylamin, N,N-Dimethyl-soyaalkylamin, Oleylamin, N,N-Dimethyl-oleylamin, Talgalkylamin oder N,N-Dimethyl-talgalkylamin, erhältlich beispielsweise unter den Handelsnamen Armeen® (von Akzo Nobel) oder Rofamin® (von Ecogreen Oleochemicals); aliphatische, cycloaliphatische oder araliphatische Diamine wie Ethylendiamin, Butandiamin, Hexamethylendiamin, Dodecandiamin, Neopentandiamin, 2-Methyl-pentamethylendiamin (MPMD), 2,2(4),4-Trimethylhexamethylendiamin (TMD), Isophorondiamin (IPD), 2,5(2,6)-Bis-(aminomethyl)-bicyclo[2.2.1]heptan (NBDA), 1,3-Xylylendiamin (MXDA), N,N'-Di(tert.butyl)ethylendiamin, N,N,N',N'-Tetramethyl-ethylendiamin, N,N,N',N'-Tetramethyl-propylendiamin, N,N,N',N'-Tetramethyl-hexamethylendiamin, 3-Dimethylaminopropylamin, 3-(Methylamino)propylamin, 3-(Cyclohexylamino)propylamin, Piperazin, N-Methylpiperazin, N,N'-Dimethylpiperazin, 1,4-Diazabicyclo[2.2.2]octan, Fettpolyamine wie N-Cocoalkyl-1,3-propandiamin, N-Oleyl-1,3-propandiamin, N-Soyaalkyl-1,3-propandiamin, N-Talgalkyl-1,3-propandiamin oder N-(C₁₆₋₂₂-Alkyl)-1,3-propandiamin, erhältlich beispielsweise unter dem Handelsnamen Duomeen® (von Akzo Nobel); Polyalkylenamine wie Diethylentriamin, Dipropylentriamin, Triethylentetramin (TETA), Tetraethylenpentamin (TEPA), Pentamethylenhexamin (PEHA), 3-(2-Aminoethyl)aminopropylamin, N,N'-Bis(3-aminopropyl)ethylendiamin, N-(3-Aminopropyl)-N-methylpropandiamin, Bis(3-dimethylaminopropyl)amin, N-(3-Dimethylaminopropyl)-1,3-propylendiamin, N-(2-Aminoethyl)piperazin (N-AEP), N-(2-Aminopropyl)piperazin, N,N'-Di-(2-aminoethyl)piperazin, 1-Methyl-4-(2-dimethylaminoethyl)piperazin, N,N,N',N",N"-Pentamethyldiethylentriamin, N,N,N',N",N"-Pentamethyldipropylentriamin, Polyethylenimine, erhältlich beispielsweise unter den Handelsnamen Lupasol® (von BASF) und Epomin® (von Nippon Shokubai); Etheramine, wie insbesondere 2-Methoxyethylamin, 2-Ethoxyethylamin, 3-Methoxypropylamin, 3-Ethoxypropylamin, 3-(2-Ethylhexyloxy)propylamin, 3-(2-Methoxyethoxy)propylamin, 2(4)-Methoxyphenylethylamin, Morpholin, N-Methylmorpholin, N-Ethylmorpholin, 2-Aminoethylmorpholin, Bis(2-aminoethyl)ether, Bis(dimethylaminoethyl)-ether, Bis(dimorpholinoethyl)ether, N,N,N'-Trimethyl-N'-hydroxyethylbis(2-aminoethyl)ether, 3,6-Dioxaoctan-1,8-diamin, 4,7-Dioxadecan-1,10-diamin, 4,7-Dioxadecan-2,9-diamin, 4,9-Dioxadodecan-1,12-diamin, 5,8-Dioxadodecan-3,10-diamin, 4,7,10-Trioxatridecan-1,13-diamin oder 2-Aminopropyl-terminierte Glykole, wie sie beispielsweise unter dem Handelsnamen Jeffamin® (von Huntsman) erhältlich sind; Aminoalkohole, wie insbesondere Ethanolamin, Isopropanolamin, Diethanolamin, Diisopopanolamin, Triethanolamin, Triisopropanolamin, N-Butylethanolamin, Diglykolamin, N,N-Diethylethanolamin, N-Methyldiethanolamin, N-Methyldiisopropylamin, N,N,N'-Trimethylaminoethylethanolamin, N-(3-Dimethylaminopropyl)-N,N-diisopropanolamin, N,N-Bis(3-dimethylaminopropyl)-N-isopropanolamin, 2-(2-Dimethylaminoethoxy)ethanolamin oder Addukte aus Mono- und Polyaminen mit Epoxiden oder Diepoxiden; Phenolgruppen-haltige Amine, wie insbesondere Kondensationsprodukte aus Phenolen, Aldehyden und Aminen (sogenannte Mannich-Basen und Phenalkamine) wie insbesondere 2-(Dimethylaminomethyl)phenol, 2,4,6-Tris(dimethylaminomethyl)phenol oder Polymere aus Phenol, Formaldehyd und N,N-Dimethyl-1,3-propandiamin sowie im Handel unter den Markennamen Cardolite® (von Cardolite), Aradur® (von Huntsman) und Beckopox® (von Cytec) erhältliche Phenalkamine; Amidgruppen-haltige Polyamine, sogenannte Polyamidoamine, wie sie im Handel erhältlich sind, beispielsweise unter den Markennamen Versamid® (von Cognis), Aradur® (von Huntsman), Euretek® (von Huntsman) oder Beckopox® (von Cytec); oder Aminosilane, wie insbesondere 3-Aminopropyltrimethoxysilan, 3-Aminopropyldimethoxymethylsilan, N-(2-Aminoethyl)-3-aminopropyltrimethoxysilan, N-(2-Aminoethyl)-3-aminopropylmethyldimethoxysilan, N-(2-Aminoethyl)-N'-[3-(trimethoxysilyl)propyl]ethylendiamin oder deren Analoga mit Ethoxy- anstelle der Methoxygruppen am Silicium.
Geeignete Hexahydrotriazine sind insbesondere 1,3,5-Hexahydrotriazin oder 1,3,5-Tris(3-(dimethylamino)propyl)-hexahydrotriazin.
Geeignete Biguanide sind insbesondere Biguanid, 1-Butylbiguanid, 1,1-Dimethylbiguanid, 1-Butylbiguanid, 1-Phenylbiguanid oder 1-(o-Tolyl)biguanid (OTBG).
Geeignete Amidine sind insbesondere 1,8-Diazabicyclo[5.4.0]undec-7-en (DBU), 1,5-Diazabicyclo[4.3.0]non-5-en (DBN), 6-Dibutylamino-1,8-diazabicyclo[5.4.0]undec-7-en, 6-Dibutylamino-1,8-diazabicyclo[5.4.0]undec-7-en, N,N'-Di-n-hexylacetamidin (DHA), 2-Methyl-1,4,5,6-tetrahydropyrimidin, 1,2-Dimethyl-1,4,5,6-tetrahydropyrimidin, 2,5,5-Trimethyl-1,4,5,6-tetrahydropyrimidin, N-(3-Trimethoxysilylpropyl)-4,5-dihydroimidazol oder N-(3-Triethoxysilylpropyl)-4,5-dihydroimidazol.
Geeignete weitere Guanidine sind insbesondere 1-Butylguanidin, 1,1-Dimethylguanidin, 1,3-Dimethylguanidin, 1,1,3,3-Tetramethylguanidin (TMG), 2-(3-(Trimethoxysilyl)propyl)-1,1,3,3-tetramethylguanidin, 2-(3-(Methyldimethoxysilyl)-propyl)-1,1,3,3-tetramethylguanidin, 2-(3-(Triethoxysilyl)propyl)-1,1,3,3-tetramethylguanidin, 1,5,7-Triazabicyclo[4.4.0]dec-5-en (TBD), 7-Methyl-1,5,7-triazabicyclo[4.4.0]dec-5-en, 7-Cyclohexyl-1,5,7-triazabicyclo[4.4.0]dec-5-en, 1-Phenylguanidin, 1-(o-Tolyl)guanidin (OTG), 1,3-Diphenylguanidin, 1,3-Di(o-tolyl)guanidin oder 2-Guanidinobenzimidazol.

Weiterhin kann die Zusammensetzung als Co-Katalysator eine Säure enthalten, insbesondere eine Carbonsäure. Bevorzugt sind aliphatische Carbonsäuren wie Ameisensäure, Laurinsäure, Stearinsäure, Isostearinsäure, Ölsäure, 2-Ethyl-2,5-dimethylcapronsäure, 2-Ethylhexansäure, Neodecansäure, Fettsäuregemische aus der Verseifung von natürlichen Fetten und Ölen oder Di- und Polycarbonsäuren, insbesondere Poly(meth)acrylsäuren.

Die Zusammensetzung ist in einer bevorzugten Ausführungsform im Wesentlichen frei von Organozinn-Verbindungen. Organozinn-freie Zusammensetzungen sind hinsichtlich Gesundheitsschutz und Umweltschutz vorteilhaft. Insbesondere beträgt der Zinngehalt der härtbaren Zusammensetzung weniger als 0.1 Gew.-%, insbesondere weniger als 0.05 Gew.-%.

Die Zusammensetzung enthält in einer weiteren bevorzugten Ausführungsform eine Kombination aus mindestens einem Katalysator der Formel (I) und mindestens einer Organozinn-Verbindung, insbesondere einer Diorganozinn(IV)-Verbindung wie die vorgängig genannten. Eine solche Zusammensetzung weist bereits bei einem geringen Zinn-Gehalt eine hohe Aushärtungsgeschwindigkeit auf, was aus toxikologischen und ökologischen Gründen vorteilhaft ist.

In einer Ausführungsform enthält die Zusammensetzung neben dem Katalysator der Formel (I) zusätzlich mindestens ein Organotitanat. Eine Kombination aus dem beschriebenen Katalysator und einem Organotitanat weist eine besonders hohe katalytische Aktivität auf. Dadurch wird eine schnelle Aushärtung einer solchen Zusammensetzung bei einer verhältnismässig geringen Einsatzmenge an Organotitanat ermöglicht.
Als Organotitanat geeignet sind insbesondere Titan(IV)-Komplexverbindungen. Bevorzugte Organoititanate sind insbesondere ausgewählt aus
- Titan(IV)-Komplexverbindungen mit zwei 1,3-Diketonat-Liganden, insbesondere 2,4-Pentandionat (=Acetylacetonat), und zwei Alkoholat-Liganden;
- Titan(IV)-Komplexverbindungen mit zwei 1,3-Ketoesterat-Liganden, insbesondere Ethylacetoacetat, und zwei Alkoholat-Liganden;
- Titan(IV)-Komplexverbindungen mit einem oder mehreren Aminoalkoholat-Liganden, insbesondere Triethanolamin oder 2-((2-Aminoethyl)amino)ethanol, und einem oder mehreren Alkoholat-Liganden;
- Titan(IV)-Komplexverbindungen mit vier Alkoholat-Liganden;
- sowie höherkondensierte Organotitanate, insbesondere oligomeres Titan(IV)-tetrabutanolat, auch als Polybutyltitanat bezeichnet;
wobei als Alkoholat-Liganden insbesondere Isobutoxy, n-Butoxy, Isopropoxy, Ethoxy und 2-Ethylhexoxy geeignet sind.

Insbesondere geeignet sind die kommerziell erhältlichen Typen Tyzor® AA, GBA, GBO, AA-75, AA-65, AA-105, DC, BEAT, BTP, TE, TnBT, KTM, TOT, TPT oder IBAY (alle von Dorf Ketal); Tytan PBT, TET, X85, TAA, ET, S2, S4 oder S6 (alle von Borica Company Ltd.) und Ken-React® KR® TTS, 7, 9QS, 12, 26S, 33DS, 38S, 39DS, 44, 134S, 138S, 133DS, 158FS oder LICA® 44 (alle von Kenrich Petrochemicals).
Ganz besonders geeignete Organotitanate sind ausgewählt aus Bis(ethylacetoacetato)diisobutoxy-titan(IV) (kommerziell erhältlich beispielsweise als Tyzor® IBAY von Dorf Ketal), Bis(ethylacetoacetato)diisopropoxy-titan(IV) (kommerziell erhältlich beispielsweise als Tyzor® DC von Dorf Ketal), Bis(acetylacetonato)diisopropoxy-titan(IV), Bis(acetylacetonato)diisobutoxy-titan(IV), Tris(oxyethyl)-amin-isopropoxy-titan(IV), Bis[tris(oxyethyl)amin]diisopropoxy-titan(IV), Bis(2-ethylhexan-1,3-dioxy)-titan(IV), Tris[2-((2-Aminoethyl)amino)ethoxy]ethoxy-titan(IV), Bis(neopentyl(diallyl)oxy)-diethoxy-titan(IV), Titan(IV)-tetrabutanolat, Tetra(2-ethylhexyloxy)titanat, Tetra(isopropoxy)titanat und Polybutyltitanat. Am meisten bevorzugt sind Bis(ethylacetoacetato)diisobutoxy-titan(IV) oder Bis(ethylacetoacetato)diisopropoxy-titan(IV).

Die Zusammensetzung kann weitere Bestandteile enthalten, insbesondere die folgenden Hilfs- und Zusatzmittel:
- Haftvermittler und/oder Vernetzer, insbesondere Aminosilane wie insbesondere 3-Aminopropyl-trimethoxysilan, 3-Aminopropyl-dimethoxymethylsilan, N-(2-Aminoethyl)-3-aminopropyl-trimethoxysilan, N-(2-Aminoethyl)-3-aminopropyl-dimethoxymethylsilan, N-(2-Aminoethyl)-N'-[3-(trimethoxysilyl)propyl]ethylendiamin oder deren Analoga mit Ethoxy- anstelle von Methoxygruppen, weiterhin N-Phenyl-, N-Cyclohexyl- oder N-Alkylaminosilane, Mercaptosilane, Epoxysilane, (Meth)acrylosilane, Anhydridosilane, Carbamatosilane, Alkylsilane oder Iminosilane, oligomere Formen dieser Silane, Addukte aus primären Aminosilanen mit Epoxysilanen oder (Meth)acrylosilanen oder Anhydridosilanen, aminofunktionelle Alkylsilsesquioxane, insbesondere aminofunktionelles Methylsilsesquioxan oder aminofunktionelles Propylsilsesquioxan. Insbesondere geeignet sind 3-Aminopropyltrimethoxysilan, 3-Aminopropyltriethoxysilan, N-(2-Aminoethyl)-3-aminopropyltrimethoxysilan, N-(2-Aminoethyl)-3-aminopropyl-triethoxysilan, 3-Glycidoxypropyltrimethoxysilan, 3-Glycidoxypropyltriethoxysilan oder 3-Ureidopropyltrimethoxysilan, oder oligomere Formen dieser Silane;
- Trocknungsmittel, insbesondere Tetraethoxysilan, Vinyltrimethoxysilan, Vinyltriethoxysilan oder Organoalkoxysilane, welche in α-Stellung zur Silangruppe eine funktionelle Gruppe aufweisen, insbesondere N-(Methyldimethoxysilylmethyl)-O-methyl-carbamat, (Methacryloxymethyl)silane, Methoxymethylsilane, Orthoameisensäureester, Calciumoxid oder Molekularsiebe, insbesondere Vinyltrimethoxysilan oder Vinyltriethoxysilan;
- Weichmacher, insbesondere Trialkylsilyl-terminierte Polydialkylsiloxane, vorzugsweise Trimethylsilyl-terminierte Polydimethylsiloxane, insbesondere mit Viskositäten im Bereich von 10 bis 1'000 mPa·s, oder entsprechende Verbindungen, bei denen einige der Methylgruppen ersetzt sind durch andere organische Gruppen, insbesondere Phenyl-, Vinyl- oder Trifluorpropylgruppen, sogenannte Reaktivweichmacher in Form von monofunktionellen, also einseitig reaktiven, Polysiloxanen, Carbonsäureester wie Phthalate, insbesondere Dioctylphthalat, Bis(2-ethylhexyl)phthalat, Bis(3-propylheptyl)-phthalat, Diisononylphthalat oder Diisodecylphthalat, Diester von ortho-Cyclohexandicarbonsäure, insbesondere Diisononyl-1,2-cyclohexandicarboxylat, Adipate, insbesondere Dioctyladipat, Bis(2-Ethylhexyl)adipat, Azelate, insbesondere Bis(2-ethylhexyl)acelat, Sebacate, insbesondere Bis(2-ethylhexyl)sebacat oder Diisononylsebacat, Polyole, insbesondere Polyoxyalkylenpolyole oder Polyesterpolyole, Glykolether, Glykolester, organische Phosphor- oder Sulfonsäureester, Sulfonsäureamide, Polybutene oder von natürlichen Fetten oder Ölen abgeleitete Fettsäuremethyl- oder -ethylester, auch "Biodiesel" genannt, wobei Siloxangruppen-haltige Weichmacher besonders geeignet sind für Silangruppen-haltige Polymere in Form von Polyorganosiloxanen;
- Lösemittel;
- anorganische oder organische Füllstoffe, insbesondere natürliche, gemahlene oder gefällte Calciumcarbonate, welche gegebenenfalls mit Fettsäuren, insbesondere Stearinsäure, beschichtet sind, Baryt (Schwerspat), Talke, Quarzmehle, Quarzsand, Dolomite, Wollastonite, Kaoline, calcinierte Kaoline, Mica (Kalium-Aluminium-Silikat), Molekularsiebe, Aluminiumoxide, Aluminiumhydroxide, Magnesiumhydroxid, Kieselsäuren inklusive hochdisperse Kieselsäuren aus Pyrolyseprozessen, industriell hergestellte Russe, Graphit, Metall-Pulver wie Aluminium, Kupfer, Eisen, Silber oder Stahl, PVC-Pulver oder Hohlkugeln;
- Fasern, insbesondere Glasfasern, Kohlefasern, Metallfasern, Keramikfasern oder Kunststofffasern wie Polyamidfasern oder Polyethylenfasern;
- Farbstoffe;
- Pigmente, insbesondere Titandioxid oder Eisenoxide;
- Rheologie-Modifizierer, insbesondere Verdickungsmittel, insbesondere Schichtsilikate wie Bentonite, Derivate von Rizinusöl, hydriertes Rizinusöl, Polyamide, Polyurethane, Harnstoffverbindungen, pyrogene Kieselsäuren, Celluloseether oder hydrophob modifizierte Polyoxyethylene;
- Stabilisatoren gegen Oxidation, Wärme, Licht oder UV-Strahlung;
- natürliche Harze, Fette oder Öle wie Kolophonium, Schellack, Leinöl, Rizinusöl oder Sojaöl;
- nicht-reaktive Polymere, wie insbesondere Homo- oder Copolymere von ungesättigten Monomeren, insbesondere aus der Gruppe umfassend Ethylen, Propylen, Butylen, Isobutylen, Isopren, Vinylacetat oder Alkyl(meth)acrylate, insbesondere Polyethylene (PE), Polypropylene (PP), Polyisobutylene, Ethylenvinylacetat-Copolymere (EVA) oder ataktische Poly-α-Olefine (APAO);
- flammhemmende Substanzen, insbesondere die bereits genannten Füllstoffe Aluminiumhydroxid und Magnesiumhydroxid, oder insbesondere organische Phosphorsäureester wie insbesondere Triethylphosphat, Trikresylphosphat, Triphenylphosphat, Diphenylkresylphosphat, Isodecyldiphenylphosphat, Tris(1,3-dichlor-2-propyl)phosphat, Tris(2-chlorethyl)phosphat, Tris(2-ethylhexyl)phosphat, Tris(chlorisopropyl)phosphat, Tris(chlorpropyl)-phosphat, isopropyliertes Triphenylphosphat, Mono-, Bis-oder Tris(isopropylphenyl)phosphate unterschiedlichen Isopropylierungsgrades, Resorcinol-bis(diphenylphosphat), Bisphenol-A-bis(diphenylphosphat) oder Ammoniumpolyphosphate;
- oberflächenaktive Substanzen, insbesondere Netzmittel, Verlaufsmittel, Entlüftungsmittel oder Entschäumer;
- Biozide, insbesondere Algizide, Fungizide oder das Pilzwachstum hemmende Substanzen;
sowie weitere üblicherweise in härtbaren Zusammensetzungen eingesetzte Substanzen. Es kann sinnvoll sein, gewisse Bestandteile vor dem Einmischen in die Zusammensetzung chemisch oder physikalisch zu trocknen.

In einer bevorzugten Ausführungsform enthält die Zusammensetzung mindestens ein Trocknungsmittel und mindestens einen Haftvermittler und/oder Vernetzer.
In einer bevorzugten Ausführungsform enthält die Zusammensetzung keine Phthalate als Weichmacher. Solche Zusammensetzungen sind toxikologisch vorteilhaft und weisen weniger Probleme mit Migrationseffekten auf.

Die Zusammensetzung wird vorzugsweise unter Ausschluss von Feuchtigkeit hergestellt und aufbewahrt. Typischerweise ist sie in einer geeigneten Verpackung oder Anordnung, wie insbesondere einer Flasche, einer Büchse, einem Beutel, einem Eimer, einem Fass oder einer Kartusche, unter Ausschluss von Feuchtigkeit lagerstabil.

Die Zusammensetzung kann in Form einer einkomponentigen oder in Form einer mehrkomponentigen, insbesondere zweikomponentigen, Zusammensetzung vorliegen.
Als "einkomponentig" wird im vorliegenden Dokument eine Zusammensetzung bezeichnet, bei welcher alle Bestandteile der Zusammensetzung vermischt im gleichen Gebinde gelagert werden und welche mit Feuchtigkeit härtbar ist. Als "zweikomponentig" wird im vorliegenden Dokument eine Zusammensetzung bezeichnet, bei welcher die Bestandteile der Zusammensetzung in zwei verschiedenen Komponenten vorliegen, welche in voneinander getrennten Gebinden gelagert werden. Erst kurz vor oder während der Applikation der Zusammensetzung werden die beiden Komponenten miteinander vermischt, worauf die vermischte Zusammensetzung aushärtet, gegebenenfalls unter Einwirkung von Feuchtigkeit.

Für den Fall, dass die Zusammensetzung ein Polyorganosiloxan mit endständigen Silangruppen enthält, ist sowohl eine einkomponentige Zusammensetzung, auch als RTV-1 bezeichnet, als auch eine zweikomponentige Zusammensetzung, auch als RTV-2 bezeichnet, bevorzugt. Im Fall einer RTV-2 Zusammensetzung ist das Polyorganosiloxan mit endständigen Silangruppen bevorzugt ein Bestandteil der ersten Komponente und ein Silanvernetzer, insbesondere ein Silan der Formel (V), ist bevorzugt ein Bestandteil der zweiten Komponente. Der Katalysator der Formel (I) kann dabei in der ersten und/oder in der zweiten Komponente enthalten sein.
Für den Fall, dass die Zusammensetzung ein Silangruppen-haltiges organisches Polymer enthält, ist die Zusammensetzung bevorzugt einkomponentig.

Eine zweite oder gegebenenfalls weitere Komponenten wird bzw. werden mit der ersten Komponente vor oder bei der Applikation vermischt, insbesondere über einen Statikmischer oder über einen dynamischen Mischer.
Die Zusammensetzung wird insbesondere umgebungswarm, bevorzugt in einem Temperaturbereich zwischen 0 °C und 45 °C, insbesondere 5 °C bis 35 °C, appliziert und härtet auch bei diesen Bedingungen aus.
Bei der Applikation beginnt die Vernetzungsreaktion der Silangruppen, gegebenenfalls unter Einfluss von Feuchtigkeit. Vorhandene Silangruppen können mit vorhandenen Silanolgruppen zu Siloxangruppen (Si-O-Si-Gruppen) kondensieren. Vorhandene Silangruppen können bei Kontakt mit Feuchtigkeit auch zu Silanolgruppen (Si-OH-Gruppen) hydrolysieren und durch nachfolgende Kondensationsreaktionen Siloxangruppen (Si-O-Si-Gruppen) bilden. Als Ergebnis dieser Reaktionen härtet die Zusammensetzung schliesslich aus. Der Katalysator der Formel (I) beschleunigt diese Aushärtung.
Falls für die Aushärtung Wasser benötigt wird, kann dieses entweder aus der Luft stammen (Luftfeuchtigkeit), oder aber die Zusammensetzung kann mit einer Wasser enthaltenden Komponente in Kontakt gebracht werden, zum Beispiel durch Bestreichen, beispielsweise mit einem Abglättmittel, oder durch Besprühen, oder es kann der Zusammensetzung bei der Applikation Wasser oder eine Wasser enthaltende Komponente zugesetzt werden, zum Beispiel in Form einer wasserhaltigen oder Wasser freisetzenden Flüssigkeit oder Paste. Eine Paste ist insbesondere geeignet für den Fall, dass die Zusammensetzung selber in Form einer Paste vorliegt.
Bei einer Aushärtung mittels Luftfeuchtigkeit härtet die Zusammensetzung von aussen nach innen durch, wobei zunächst eine Haut an der Oberfläche der Zusammensetzung gebildet wird. Die sogenannte Hautbildungszeit stellt ein Mass für die Aushärtungsgeschwindigkeit der Zusammensetzung dar. Die Geschwindigkeit der Aushärtung wird dabei im Allgemeinen von verschiedenen Faktoren, wie beispielsweise der Verfügbarkeit von Wasser, der Temperatur usw., bestimmt.

Die Zusammensetzung eignet sich für eine Vielzahl von Anwendungen, insbesondere als Anstrich, Lack oder Primer, als Harz zur Herstellung von Faserverbundwerkstoff (Composite), als Hartschaum, Weichschaum, Formteil, Elastomer, Faser, Folie oder Membran, als Vergussmasse, Dichtstoff, Klebstoff, Belag, Beschichtung oder Anstrich für Bau- und Industrieanwendungen, beispielsweise als Nahtabdichtung, Hohlraumversiegelung, Elektroisolationsmasse, Spachtelmasse, Fugendichtstoff, Schweiss- oder Bördelnahtdichtstoff, Montageklebstoff, Karrosserieklebstoff, Scheibenklebstoff, Sandwichelementklebstoff, Kaschierklebstoff, Laminatklebstoff, Verpackungsklebstoff, Holzklebstoff, Parkettklebstoff, Verankerungsklebstoff, Bodenbelag, Bodenbeschichtung, Balkonbeschichtung, Dachbeschichtung, Betonschutzbeschichtung, Parkhausbeschichtung, Versiegelung, Rohrbeschichtung, Korrosionsschutzbeschichtung, Textilbeschichtung, Dämpfungselement, Dichtungselement oder Spachtelmasse.
Besonders geeignet ist die Zusammensetzung als Klebstoff und/oder Dichtstoff, insbesondere für die Fugenabdichtung und für elastische Klebeverbindungen in Bau- und Industrieanwendungen, sowie als elastische Beschichtung mit rissüberbrückenden Eigenschaften, insbesondere zum Schützen und/oder Abdichten von beispielsweise Dächern, Böden, Balkonen, Parkdecks oder Betonröhren.
Bevorzugt stellt die Zusammensetzung somit einen Klebstoff oder einen Dichtstoff oder eine Beschichtung dar.

Eine solche Zusammensetzung enthält typischerweise Weichmacher, Füllstoffe, Haftvermittler und/oder Vernetzer und Trocknungsmittel und gegebenenfalls weitere Hilfs- und Zusatzstoffe.
Für eine Anwendung als Klebstoff oder Dichtstoff weist die Zusammensetzung bevorzugt eine pastöse Konsistenz mit strukturviskosen Eigenschaften auf. Ein solcher pastöser Dichtstoff oder Klebstoff wird insbesondere aus handelsüblichen Kartuschen, welche manuell, mittels Druckluft oder Batterie betrieben werden, oder aus einem Fass oder Hobbock mittels einer Förderpumpe oder eines Extruders, gegebenenfalls mittels eines Applikationsroboters, auf ein Substrat aufgetragen.
Für eine Anwendung als Beschichtung weist die Zusammensetzung bevorzugt eine bei Raumtemperatur flüssige Konsistenz mit selbstverlaufenden Eigenschaften auf. Gegebenenfalls ist sie leicht thixotrop, so dass die Beschichtung an abschüssigen bis senkrechten Flächen applizierbar ist, ohne sofort wegzufliessen. Sie wird insbesondere appliziert mittels Rolle oder Pinsel oder durch Ausgiessen und Verteilen mittels beispielsweise einem Roller, einem Schaber oder einer Zahntraufel.

Bei der Applikation wird die Zusammensetzung bevorzugt auf mindestens ein Substrat appliziert.
Geeignete Substrate sind insbesondere
- Glas, Glaskeramik, Beton, Mörtel, Backstein, Ziegel, Gips oder Natursteine wie Kalkstein, Granit oder Marmor;
- Metalle und Legierungen, wie Aluminium, Eisen, Stahl oder Buntmetalle, sowie oberflächenveredelte Metalle oder Legierungen, wie verzinkte oder verchromte Metalle;
- Leder, Textilien, Papier, Holz, mit Harzen, beispielsweise Phenol-, Melamin- oder Epoxidharzen, gebundene Holzwerkstoffe, Harz-Textil-Verbundwerkstoffe und weitere sogenannte Polymer-Composites;
- Kunststoffe, wie Polyvinylchlorid (Hart- und Weich-PVC), Acrylonitril-Butadien-Styrol-Copolymere (ABS), Polycarbonat (PC), Polyamid (PA), Polyester, Poly(methylmethacrylat) (PMMA), Epoxidharze, Polyurethane (PUR), Polyoxymethylen (POM), Polyolefine (PO), Polyethylen (PE) oder Polypropylen (PP), Ethylen/Propylen-Copolymere (EPM) oder Ethylen/Propylen/Dien-Terpolymere (EPDM), oder faserverstärkte Kunststoffe wie Kohlefaserverstärkte Kunststoffe (CFK), Glasfaser-verstärkte Kunststoffe (GFK) oder Sheet Moulding Compounds (SMC), wobei die Kunststoffe bevorzugt mittels Plasma, Corona oder Flammen oberflächenbehandelt sein können;
- beschichtete Substrate, wie pulverbeschichtete Metalle oder Legierungen;
- Farben oder Lacke, insbesondere Automobildecklacke.
Die Substrate können bei Bedarf vor dem Applizieren der Zusammensetzung vorbehandelt werden, insbesondere durch physikalische und/oder chemische Reinigungsverfahren oder durch das Aufbringen eines Haftvermittlers, einer Haftvermittlerlösung oder eines Primers.
Besonders geeignet ist die Zusammensetzung für den Kontakt mit Substraten, die besonders empfindlich sind auf Störungen durch migrierende Substanzen, insbesondere durch das Ausbilden von Verfärbungen oder Flecken. Dies sind insbesondere feinporige Substrate wie Marmor, Kalkstein oder andere Natursteine, Gips, Zementmörtel oder Beton, aber auch Kunststoffe. Insbesondere auf PVC werden bei der Anwesenheit von Katalysatoren wie beispielsweise DBU oder TMG starke Verfärbungen beobachtet, die sich durch Reinigung nicht entfernen lassen. Solche Effekte werden mit dem Katalysator der Formel (I) nicht beobachtet.
Verklebt oder abgedichtet werden können zwei gleichartige oder zwei verschiedene Substrate, insbesondere die vorgängig genannten Substrate.

Nach der Aushärtung der Zusammensetzung mit Wasser, insbesondere in Form von Luftfeuchtigkeit, und/oder mit mindestens einem geeigneten Vernetzer wird eine ausgehärtete Zusammensetzung erhalten.

Aus der Anwendung der Zusammensetzung entsteht ein Artikel, welcher mit der Zusammensetzung insbesondere verklebt, abgedichtet oder beschichtet wurde. Beim Artikel handelt es sich insbesondere um ein Bauwerk, insbesondere ein Bauwerk des Hoch- oder Tiefbaus, um ein industriell gefertigtes Gut oder um ein Konsumgut, insbesondere ein Fenster, eine Haushaltmaschine oder ein Transportmittel wie insbesondere ein Automobil, ein Bus, ein Lastkraftwagen, ein Schienenfahrzeug, ein Schiff, ein Flugzeug oder ein Helikopter; oder der Artikel kann ein Anbauteil davon sein.

### Beispiele

Im Folgenden sind Ausführungsbeispiele aufgeführt, welche die beschriebene Erfindung näher erläutern sollen. Als "Normklima" wird eine Temperatur von 23±1 °C und eine relative Luftfeuchtigkeit von 50±5% bezeichnet.
"EEW" steht für das Epoxid-Equivalentgewicht.
**¹H-NMR-Spektren** wurden auf einem Spektrometer des Typs Bruker Ascend 400 bei 400.14 MHz gemessen; die chemischen Verschiebungen δ sind angegeben in ppm relativ zu Tetramethylsilan (TMS). Kopplungskonstanten J sind angegeben in Hz. Echte und Pseudo-Kopplungsmuster wurden nicht unterschieden.
**Infrarotspektren** (FT-IR) wurden auf einem mit horizontaler ATR-Messeinheit mit Diamant-Kristall ausgestatteten FT-IR Gerät Nicolet iS5 von Thermo Scientific gemessen. Flüssige Proben wurden unverdünnt als Filme aufgetragen, feste Proben wurden in CH₂Cl₂ gelöst. Die Absorptionsbanden sind in Wellenzahlen (cm⁻¹) angegeben (Messfenster: 4000-650 cm⁻¹).
**Gaschromatogramme** (GC) wurden gemessen im Temperaturbereich von 60 bis 320 °C mit einer Aufheizrate von 15 °C/min und 10 min Verweilzeit bei 320 °C. Die Injektortemperatur betrug 250 °C. Es wurde eine Zebron ZB-5 Säule verwendet (L = 30 m, ID = 0.25 mm, dj = 0.5 µm) bei einem Gasfluss von 1.5 ml/Min. Die Detektion erfolgte mittels Flammenionisation (FID), wobei die Signale via Flächenprozent-Methode ausgewertet wurden.
Die **Hautbildungszeit** (HBZ) wurde dadurch bestimmt, dass einige Gramm der Zusammensetzung in einer Schichtdicke von ca. 2 mm auf Pappkarton aufgetragen wurden und im Normklima die Zeitdauer gemessen wurde, bis beim leichten Antippen der Oberfläche der Zusammensetzung mittels einer Pipette aus LDPE erstmals keine Rückstände auf der Pipette mehr zurückblieben.
Die Beschaffenheit der **Oberfläche** wurde haptisch geprüft.

Die mechanischen Eigenschaften **Zugfestigkeit, Bruchdehnung** und **E-Modul** (bei 0-5% sowie bei 0-50% bzw. 0-100% Dehnung) wurden gemäss DIN EN 53504 bei einer Zuggeschwindigkeit von 200 mm/min. gemessen.

### verwendete Reagentien:

- DCC: N,N'-Dicyclohexylcarbodiimid (Sigma-Aldrich)
- DIC: N,N'-Diisopropylcarbodiimid (Sigma-Aldrich)

### verwendete funktionelle Verbindungen:

- TMPTA: 1,1,1-Trimethylolpropantriacrylat (Sartomer SR-351, von Sartomer)
- Polymer-NCO-1: Umsetzungsprodukt von 500 g Polyol Acclaim® 4200 (Polyoxypropylendiol mit niedrigem Ungesättigtheitsgrad, von Bayer; OH-Zahl 28.0 mg KOH/g) und 2000 g Polyol Caradol® MD34-02 (Polyoxypropylenpolyoxyethylen-Triol, von Shell; OH-Zahl 35.0 mg KOH/g) mit 253 g Toluylendiisocyanat (Desmodur® T80 P, von Bayer); NCO-Gehalt 2.06 Gew.-%
- Polymer-HEA-1: Umsetzungsprodukt von 169.47 g Polymer-NCO-1 mit 9.60 g 2-Hydroxyethylacrylat (HEA) und 0.2 g Bismutneodecanoat während 3 h bei 80 °C (bis zur vollständigen Umsetzung der Isocyanat-Bande bei 2277 cm⁻¹ im FT-IR-Spektrum)
- Polymer-NCO-2: Umsetzungsprodukt von 3.2 kg Polyol Acclaim® 12200 (Polyoxypropylendiol mit niedrigem Ungesättigtheitsgrad, von Bayer; OH-Zahl 11.0 mg KOH/g) mit 149.4 g Isophorondiisocyanat (Vestanat® IPDI, von Evonik) und 0.5 g Bismuttris(neodecanoat); NCO-Gehalt 0.88 Gew.-%
- EHGE: 2-Ethylhexylglycidylether (D.E.R.™ 728, von Dow, EEW ca. 210 g/Eq)
- DY-E: Monoglycidylether von C₁₂-C₁₄ Alkoholen (Araldite® DY-E, von Huntsman, EEW ca. 282 g/Eq)
- DY-K: o-Kresylglycidylether (Araldite® DY-E, von Huntsman, EEW ca. 282 g/Eq)
- BADGE: Bisphenol A-diglycidylether (Araldite® MY 790, von Huntsman, EEW ca. 186 g/Eq)
- DY-F: Polypropylenglykoldiglycidylether (Araldite® DY-F, von Huntsman, EEW ca. 510 g/Eq)

2,2-Dimethyl-3-lauroyloxypropanal (hergestellt wie in US 7,625,993, Aldehyd A1) 2,2-Dimethyl-3-(N-morpholino)propanal (hergestellt wie in US 8,252,859) 1,2-Propylencarbonat (Sigma-Aldrich)
2,4-Pentandion (Sigma-Aldrich) N,N-Diethylacetoacetamid (Sigma-Aldrich)

### Herstellung von Guanidinen der Formel (II):

### Verbindung G-1: 1-(3-Aminopropyl)-2,3-dicyclohexylguanidin

In einem Rundkolben wurden 2.50 g 1,3-Diaminopropan und 6.89 g DCC vermischt und unter Rühren auf 120 °C erwärmt. Nach 1 Stunde war die Carbodiimid-Bande bei ca. 2120 cm⁻¹ (FT-IR-Spektroskopie) vollständig verschwunden. Darauf wurde das Reaktionsgemisch im Vakuum von den flüchtigen Bestandteilen befreit. Man erhielt 9.36 g eines hellgelben, geruchsarmen Öls, welches nach einigen Tagen zu einer festen Masse erstarrte.
¹H-NMR (CDCl₃): δ 1.05-1.2 und 1.25-1.40 (2 x *m,* 10 H), 1.54-1.78 (*m,* 10 H), 1.88-2.0 (*m,* 4 H), 2.73 (*m,* 2 H), 3.12 (*m,* 2 H), 3.22 (br s, 2 H).
FT-IR: 3371 (N-H), 2921, 2849, 1627 (C=N), 1502, 1447, 1324, 1238, 1147, 1111, 888, 713.

### Verbindung G-2: 1-(2-Aminopropyl)-2,3-dicyclohexylguanidin und 1-(3-Aminoprop-2-yl)-2,3-dicyclohexylguanidin

In einem Rundkolben wurden 7.78 g 1,2-Diaminopropan und 20.63 g DCC vermischt und unter Rühren auf 120 °C erwärmt. Nach 3 Stunden war die Carbodiimid-Bande bei ca. 2120 cm⁻¹ (FT-IR-Spektroskopie) vollständig verschwunden. Darauf wurde das Reaktionsgemisch im Vakuum von den flüchtigen Bestandteilen befreit. Man erhielt 28.0 g eines hellgelben, geruchsarmen Öls. FT-IR: 3270 (N-H), 2922, 2849, 1626 (C=N), 1497, 1447, 1363, 1336, 1285, 1237, 1189, 1146, 1112, 1091, 1073, 1050, 1027, 977, 888, 860, 804, 715.

### Verbindung G-3: 1-(6-Amino-3,3(5),5-trimethyl-hexyl)-2,3-dicyclohexylguanidin und 1-(6-Amino-2,2(4),4-trimethyl-hexyl)-2,3-dicyclohexylguanidin

In einem Rundkolben wurden 16.62 g Vestamin®TMD (Gemisch von 2,2,4- und 2,4,4-Trimethyl-1,6-hexamethylendiamin, von Evonik) und 20.63 g DCC vermischt und unter Rühren auf 120 °C erwärmt. Nach 3 Stunden war die Carbodiimid-Bande bei ca. 2120 cm⁻¹ (FT-IR-Spektroskopie) vollständig verschwunden. Darauf wurde das Reaktionsgemisch im Vakuum von den flüchtigen Bestandteilen befreit. Man erhielt 36.5 g eines hellgelben, geruchsarmen Öls.
FT-IR: 3281 (N-H), 2923, 2850, 1635 (C=N), 1496, 1463, 1448, 1362, 1325, 1284, 1237, 1188, 1146, 1112, 1090, 1071, 1051, 1027, 977, 888, 860, 845, 804, 785, 714.

### Verbindung G-4: 1-(3-Cyclohexylaminopropyl)-2,3-diisopropylguanidin

In einem Rundkolben wurden 34.38 g 3-(Cyclohexylamino)propylamin (von BASF) und 25.24 g DIC vermischt und unter Rühren auf 120 °C erwärmt. Nach 2 Stunden war die Carbodiimid-Bande bei ca. 2120 cm⁻¹ (FT-IR-Spektroskopie) vollständig verschwunden. Darauf wurde das Reaktionsgemisch im Vakuum von den flüchtigen Bestandteilen befreit. Man erhielt 56.50 g eines hellgelben, geruchsarmen Öls.
FT-IR: 3292 (N-H), 2960, 2924, 2852, 1633 (C=N), 1505, 1448, 1361, 1329, 1176, 1125, 889, 714.

### Verbindung G-5: 1-(2-(2-Hydroxyethoxy)ethyl)-2,3-dicyclohexylguanidin

In einem Rundkolben wurden 3.55 g 2-(2-Aminoethoxy)ethanol (Diglycolamine® Agent, von Huntsman) und 6.81 g DCC vermischt und unter Rühren auf 120 °C erwärmt. Nach 24 Stunden war die Carbodiimid-Bande bei ca. 2120 cm⁻¹ (FT-IR-Spektroskopie) vollständig verschwunden. Man erhielt ein hellgelbes, geruchsarmes Öl.
¹H-NMR (CDCl₃): δ 1.05-1.3 und 1.3-1.45 (2 x *m,* 10 H, CH₂), 1.54-1.78 (*m*, 8 H), 1.88-2.0 (*m,* 4 H), 3.13 (*t,* 2 H, CH₂N), 3.69 (*m,* 4 H, CH₂O), 3.81 (*t,* 2 H, OC*H*₂CH₂N).
FT-IR: 3355 (O-H), 2922, 2849, 1617 (C=N), 1520, 1447, 1340, 1257, 1240, 1117, 1066, 888, 717.

### Verbindung G-6: 1-(2-(2-Hydroxyethoxy)ethyl)-2,3-diisopropylguanidin

In einem Rundkolben wurden 23.14 g 2-(2-Aminoethoxy)ethanol (Diglycolamine® Agent, von Huntsman) und 25.24 g DIC vermischt und unter Rühren auf 120 °C erwärmt. Nach 24 Stunden war die Carbodiimid-Bande bei ca. 2120 cm⁻¹ (FT-IR-Spektroskopie) vollständig verschwunden. Darauf wurde das Reaktionsgemisch im Vakuum von den flüchtigen Bestandteilen befreit. Man erhielt 56.50 g eines dünnflüssigen, hellgelben, geruchsarmen Öls.
FT-IR: 3354 (O-H), 2963, 2921, 2865, 1616 (C=N), 1524, 1465, 1362, 1337, 1178, 1121, 1066, 884, 829, 715.

### Herstellung von Amin-Zwischenprodukten:

### Verbindung A-1: N-(2,2-Dimethyl-3-lauroyloxypropyliden)-1,3-diaminopropan

In einem Rundkolben wurden 3.82 g 1,3-Diaminopropan und 14.21 g 2,2-Dimethyl-3-lauroyloxypropanal vermischt und unter Rühren auf 60 °C erwärmt. Nach 30 Minuten wurden die flüchtigen Bestandteile bei 80 °C mittels Vakuum entfernt. Man erhielt 16.96 g eines gelblichen Öls.
FT-IR: 3308, 2922, 2853, 1733, 1668, 1556, 1466, 1432, 1394, 1376, 1350, 1316, 1235, 1165, 1134, 1108, 1048, 1031, 1005, 946, 902, 877, 854, 798, 767, 722.

### Verbindung A-2: N-(2,2-Dimethyl-3-lauroyloxypropyliden)-1,2-diaminopropan

In einem Rundkolben wurden 3.81 g 1,2-Diaminopropan und 14.25 g 2,2-Dimethyl-3-lauroyloxypropanal vermischt und unter Rühren auf 60 °C erwärmt. Nach 20 Minuten wurden die flüchtigen Bestandteile bei 80 °C mittels Vakuum entfernt. Man erhielt 16.97 g eines farblosen Öls.
FT-IR: 3353, 2956, 2923, 2853, 1733, 1667, 1632, 1466, 1418, 1391, 1376, 1301, 1253, 1161, 1112, 1076, 1056, 1001, 934, 883, 826, 722.

### Verbindung A-3: N-(2,2-Dimethyl-3-(N-morpholino)propyliden)-1,3-diaminopropan

In einem Rundkolben wurden 3.16 g 1,3-Diaminopropan und 6.87 g 2,2-Dimethyl-3-(N-morpholino)propanal vermischt und unter Rühren auf 80 °C erwärmt. Nach 40 Minuten wurden die flüchtigen Bestandteile bei 80 °C mittels Vakuum entfernt. Man erhielt 8.67 g eines gelblichen Öls.
FT-IR: 3286, 2934, 2848, 2800, 1665, 1573, 1455, 1430, 1375, 1349, 1313, 1282, 1263, 1203, 1133, 1115, 1070, 1337, 1010, 983, 923, 863, 803, 767.

### Herstellung von Katalysatoren der Formel (I):

### Katalysator K-1: 1,1,1-Trimethylolpropan tris(3-(3-(2,3-dicyclohexylguanidino)-propylamino)propionat)

In einem Rundkolben wurden 28.05 g der vorgängig hergestellten Verbindung G-1 mit 9.88 g TMPTA vermischt und auf 120 °C erwärmt. Nach 4 Stunden war die Acrylat-Bande bei ca. 809 cm⁻¹ (FT-IR-Spektroskopie) vollständig verschwunden. Darauf wurde das Reaktionsgemisch im Vakuum von den flüchtigen Bestandteilen befreit. Man erhielt 37.93 g eines hochviskosen, geruchlosen Öls.
FT-IR: 3291 (N-H), 2923, 2850, 1732 (C=O), 1628 (C=N), 1515, 1448, 1381, 1362, 1343, 1254, 1170, 1113, 1051, 1025, 888, 843, 782, 707.

### Katalysator K-2: 1,1,1-Trimethylolpropan tris(3-(N-cyclohexyl-3-(2,3-diisopropylguanidino)propylamino)propionat)

In einem Rundkolben wurden 28.25 g der vorgängig hergestellten Verbindung G-4 mit 9.88 g TMPTA vermischt und auf 120 °C erwärmt. Nach 24 Stunden war die Acrylat-Bande bei ca.809 cm⁻¹ (FT-IR-Spektroskopie) vollständig verschwunden. Darauf wurde das Reaktionsgemisch im Vakuum von den flüchtigen Bestandteilen befreit. Man erhielt 38.10 g eines hochviskosen, geruchlosen Öls.
FT-IR: 2961, 2925, 2852, 1735 (C=O), 1627 (C=N), 1449, 1362, 1346, 1305, 1250, 1163, 1125, 1055, 889, 718.

### Katalysator K-3:

In einem Rundkolben wurden 179.27 g Polymer-HEA-1 vorgelegt, 23.45 g der vorgängig hergestellten Verbindung G-4 zugegeben und das Reaktionsgemisch auf 100 °C erwärmt. Nach 3 Stunden war die Acrylat-Bande bei ca.815 cm⁻¹ (FT-IR-Spektroskopie) vollständig verschwunden. Man erhielt ein mittelviskoses, geruchloses Öl.
FT-IR: 2969, 2929, 2866, 1720 (C=O), 1644 (C=N), 1525, 1452, 1372, 1343, 1296, 1096, 1013, 925, 866, 832, 700.

### Katalysator K-4:

In einem Rundkolben wurden 179.27 g Polymer-HEA-1 vorgelegt, 23.19 g der vorgängig hergestellten Verbindung G-2 zugegeben und das Reaktionsgemisch auf 100 °C erwärmt. Nach 2 Stunden war die Acrylat-Bande bei ca. 815 cm⁻¹ (FT-IR-Spektroskopie) vollständig verschwunden. Man erhielt ein mittelviskoses, geruchloses Öl.
FT-IR: 3305, 1969, 2928, 2864, 1730 (C=O), 1682, 1621 (C=N), 1533, 1450, 1372, 1344, 1296, 1226, 1096, 1013, 926, 866, 825, 768, 718.

### Katalysator K-5:

In einem Rundkolben wurden 179.27 g Polymer-HEA-1 vorgelegt, 30.15 g der vorgängig hergestellten Verbindung G-3 zugegeben und das Reaktionsgemisch auf 100 °C erwärmt. Nach 2 Stunden war die Acrylat-Bande bei ca. 815 cm⁻¹ (FT-IR-Spektroskopie) vollständig verschwunden. Man erhielt ein mittelviskoses, geruchloses Öl.
FT-IR: 3312, 2969, 2928, 2865, 1728 (C=O), 1682, 1629 (C=N), 1538, 1450, 1372, 1344, 1296, 1226, 1097, 1013, 926, 865, 825, 767, 714.

### Katalysator K-6:

In einem Rundkolben wurden 179.27 g Polymer-HEA-1 vorgelegt, 24.40 g der vorgängig hergestellten Verbindung G-1 zugegeben und das Reaktionsgemisch auf 100 °C erwärmt. Nach 2 Stunden war die Acrylat-Bande bei ca. 815 cm⁻¹ (FT-IR-Spektroskopie) vollständig verschwunden. Man erhielt ein mittelviskoses, geruchloses Öl.
FT-IR: 3309, 2970, 2930, 2867, 1730 (C=O), 1620 (C=N), 1601, 1533, 1452, 1408, 1372, 1344, 1296, 1225, 1180, 1093, 1013, 926, 867, 811, 768.

### Katalysator K-7:

In einem Rundkolben wurden 282.2 g Polymer-NCO-2 vorgelegt, mit 17.79 g der vorgängig hergestellten Verbindung G-5 unter Vakuum vermischt und auf 50 °C erwärmt. Nach 1 Stunde war die Isocyanat-Bande bei ca. 2263 cm⁻¹ (FT-IR-Spektroskopie) vollständig verschwunden. Darauf wurden 1.5 g Vinyltrimethoxysilan zugegeben. Man erhielt ein mittelviskoses, geruchloses Öl.
FT-IR: 2969, 2929, 2866, 1720 (C=O), 1644 (C=N), 1525, 1452, 1372, 1343, 1296, 1096, 1013, 925, 866, 832, 700.

### Katalysator K-8:

In einem Rundkolben wurden 282.2 g Polymer-NCO-2 vorgelegt, mit 13.21 g der vorgängig hergestellten Verbindung G-6 unter Vakuum vermischt und auf 50 °C erwärmt. Nach 5 Minuten war die Isocyanat-Bande bei ca. 2263 cm⁻¹ (FT-IR-Spektroskopie) vollständig verschwunden. Darauf wurden 1.5 g Vinyltrimethoxysilan zugegeben. Man erhielt ein mittelviskoses, geruchloses Öl.
FT-IR: 2969, 2930, 2867, 1720 (C=O), 1675, 1644 (C=N), 1525, 1454, 1372, 1343, 1296, 1096, 1013, 925, 867, 831.

### Katalysator K-9: 1-(3-((3-((2-Ethylhexyl)oxy)-2-hydroxypropyl)amino)propyl)-2,3-dicyclohexylguanidin

In einem Rundkolben wurden 2.89 g der vorgängig hergestellten Verbindung G-1 mit 1.86 g EHGE vermischt und auf 50 °C erwärmt. Nach 24 Stunden war die Epoxid-Bande bei ca. 911 cm⁻¹ (FT-IR-Spektroskopie) fast vollständig verschwunden, worauf die Reaktionsmischung während 3 Stunden bei 80 °C gerührt wurde. Man erhielt ein farbloses, mittelviskoses, geruchloses Öl, das bei Raumtemperatur nach einigen Stunden wachsartig erstarrte.
FT-IR: 2923, 2851, 1627 (C=N), 1507, 1448, 1360, 1340, 1256, 1238, 1104, 977, 888, 845, 767, 725.

### Katalysator K-10: 1-(3-((3-((C₁₂₋₁₄-Alkyl)oxy)-2-hydroxypropyl)amino)propyl)-2,3-dicyclohexylguanidin

In einem Rundkolben wurden 2.89 g der vorgängig hergestellten Verbindung G-1 mit 2.82 g DY-E vermischt und auf 80 °C erwärmt. Nach 6 Stunden war die Epoxid-Bande bei ca. 912 cm⁻¹ (FT-IR-Spektroskopie) vollständig verschwunden. Man erhielt ein farbloses, mittelviskoses, geruchloses Öl.
FT-IR: 3246, 2921, 2851, 1627 (C=N), 1505, 1463, 1449, 1405, 1361, 1344, 1304, 1257, 1239, 1112, 1028, 977, 889, 844, 814, 720.

### Katalysator K-11: 1-(3-((3-((2-Methylphenyl)oxy)-2-hydroxypropyl)amino)propyl)-2,3-dicyclohexylguanidin

In einem Rundkolben wurden 2.89 g der vorgängig hergestellten Verbindung G-1 mit 1.83 g DY-K vermischt und auf 80 °C erwärmt. Nach 6 Stunden war die Epoxid-Bande bei ca. 915 cm⁻¹ (FT-IR-Spektroskopie) vollständig verschwunden. Man erhielt ein farbloses, mittelviskoses, geruchloses Öl.
FT-IR: 3270, 2923, 2849, 1622 (C=N), 1602, 1494, 1448, 1360, 1339, 1307, 1287, 1243, 1190, 1147, 1120, 1050, 1034, 977, 924, 888, 940, 747, 712.

### Katalysator K-12: 2,2-Bis(4-(2-hydroxy-3-(3-(2,3-dicyclohexylguanidino)propyl)aminopropoxy)phenyl)propan

In einem Rundkolben wurden 2.89 g der vorgängig hergestellten Verbindung G-1 mit 1.86 g BADGE vermischt und auf 80 °C erwärmt. Nach 6 Stunden war die Epoxid-Bande bei ca. 914 cm⁻¹ (FT-IR-Spektroskopie) vollständig verschwunden. Man erhielt ein farbloses, hochviskoses, geruchloses Öl.
FT-IR: 3357 (O-H), 2924, 2851, 1606 (C=N), 1580, 1507, 1450, 1383, 1343, 1296, 1235, 1181, 1105, 1084, 1033, 967, 915, 826, 757, 737, 726, 668.

### Katalysator K-13: α,ω-Bis(2-hydroxy-3-(3-(2,3-dicyclohexylguanidino)propyl)-aminopropyl)polypropylenglykol

In einem Rundkolben wurden 2.89 g der vorgängig hergestellten Verbindung G-1 mit 5.10 g DY-F vermischt und auf 80 °C erwärmt. Nach 6 Stunden war die Epoxid-Bande bei ca. 907 cm⁻¹ (FT-IR-Spektroskopie) vollständig verschwunden. Man erhielt ein farbloses, mittelviskoses, geruchloses Öl, das bei Raumtemperatur nach einigen Stunden eine pastöse Konsistenz annahm.
FT-IR: 3270, 2969, 2926, 2852, 1623 (C=N), 1507, 1449, 1372, 1343, 1298, 1257, 1099, 1014, 926, 889, 862, 836, 712.

### Katalysator K-14: 2-Hydroxypropyl N-(3-(2,3-dicyclohexylguanidino)propyl)-carbamat und 3-Hydroxy-2-propyl N-(3-(2,3-dicyclohexylguanidino)propyl)carbamat

In einem Rundkolben wurden 2.89 g der vorgängig hergestellten Verbindung G-1 mit 1.12 g 1,2-Propylencarbonat vermischt und auf 90 °C erwärmt. Nach 30 Minuten wurden die flüchtigen Bestandteile bei 80 °C mittels Vakuum entfernt. Man erhielt 4.03 g eines transparenten geruchlosen Feststoffs.
FT-IR: 3335, 2925, 2851, 1699, 1621, 1518, 1448, 1361, 1338, 1257, 1145, 1109, 1054, 992, 978, 889, 845, 800, 775, 715.

### Katalysator K-15: 1-(3-(3-Acetylprop-2-en-2-yl)aminopropyl)-2,3-dicyclohexylguanidin

In einem Rundkolben wurden 2.89 g der vorgängig hergestellten Verbindung G-1 mit 1.10 g 2,4-Pentandion vermischt und nach 10 Minuten die flüchtigen Bestandteile bei 80 °C mittels Vakuum entfernt. Man erhielt 3.98 g eines gelben geruchlosen Feststoffs.
FT-IR: 3192, 3044, 2925, 2852, 1608, 1570, 1483, 1445, 1360, 1295, 1245, 1237, 1211, 1191, 1173, 1150, 1103, 1074, 1052, 1000, 952, 890, 846, 785, 738.

### Katalysator K-16: 1-(3-(3-(N,N-Diethylaminocarbonyl)prop-2-en-2-yl)aminopropyl)-2,3-dicyclohexylguanidin

In einem Rundkolben wurden 2.89 g der vorgängig hergestellten Verbindung G-1 mit 1.61 g N,N-Diethylacetoacetamid vermischt und nach 10 Minuten die flüchtigen Bestandteile mittels Vakuum entfernt. Man erhielt 3.89 g eines weissen geruchlosen Feststoffs.
FT-IR: 3197, 2970, 2926, 2852, 1722, 1633, 1591, 1473, 1493, 1476, 1448, 1360, 1347, 1315, 1257, 1231, 1150, 1122, 1069, 999, 980, 954, 913, 890, 843, 827, 772, 710.

### Katalysator K-17: 3-((3-(2,3-Dicyclohexylguanidino)propyl)imino)-2,2-dimethylpropyl dodecanoat

In einem Rundkolben wurden 2.89 g der vorgängig hergestellten Verbindung G-1 mit 2.97 g 2,2-Dimethyl-3-lauroyloxypropanal vermischt, 10 Minuten bei Raumtemperatur gerührt und dann die flüchtigen Bestandteile bei 80 °C mittels Vakuum entfernt. Man erhielt 5.72 g eines gelblichen, mittelviskosen, geruchlosen Öls.
FT-IR: 3202, 2923, 2852, 1739, 1661, 1627, 1564, 1450, 1417, 1392, 1364, 1348, 1288, 1246, 1190, 1151, 1107, 1067, 1028, 999, 890, 845, 800, 720.

### Katalysator K-18: 3-((3-(2,3-Dicyclohexylguanidino)propyl)imino)-2,2-dimethylpropyl dodecanoat

In einem Rundkolben wurden 7.06 g der vorgängig hergestellten Verbindung A-1 mit 4.05 g DCC vermischt und unter Rühren auf 110 °C erwärmt. Nach 48 Stunden war die Carbodiimid-Bande bei ca. 2120 cm⁻¹ (FT-IR-Spektroskopie) vollständig verschwunden. Darauf wurde das Reaktionsgemisch im Vakuum von den flüchtigen Bestandteilen befreit. Man erhielt 11.12 g eines gelben, niedrigviskosen, geruchlosen Öls.
FT-IR: 3292, 2922, 2851, 1738, 1640, 1543, 1449, 1364, 1315, 1253, 1151, 1110, 1070, 889, 845, 721.

### Katalysator K-19: 3-((3-(2,3-Diisopropylguanidino)propyl)imino)-2,2-dimethylpropyl dodecanoat

In einem Rundkolben wurden 5.79 g der vorgängig hergestellten Verbindung A-1 mit 1.96 g DIC vermischt und unter Rühren auf 110 °C erwärmt. Nach 4 Tagen war die Carbodiimid-Bande bei ca. 2120 cm⁻¹ (FT-IR-Spektroskopie) vollständig verschwunden. Darauf wurde das Reaktionsgemisch im Vakuum von den flüchtigen Bestandteilen befreit. Man erhielt 7.75 g eines gelben, niedrigviskosen, geruchlosen Öls.
FT-IR: 3290, 2958, 2923, 2853, 1738, 1640, 1538, 1465, 1364, 1258, 1166, 1124, 1012, 933, 721.

### Katalysator K-20: 3-((3-(2,3-Dicyclohexylguanidino)prop-2-yl)imino)-2,2-dimethylpropyl dodecanoat

In einem Rundkolben wurden 7.65 g der vorgängig hergestellten Verbindung A-2 mit 4.41 g DCC vermischt und unter Rühren auf 110 °C erwärmt. Nach 7 Tagen war die Carbodiimid-Bande bei ca. 2120 cm⁻¹ (FT-IR-Spektroskopie) vollständig verschwunden. Darauf wurde das Reaktionsgemisch im Vakuum von den flüchtigen Bestandteilen befreit. Man erhielt 12.06 g eines orangen, niedrigviskosen, geruchlosen Öls.
FT-IR: 3304, 2923, 2852, 1738, 1643, 1538, 1463, 1449, 1374, 1345, 1318, 1252, 1237, 1151, 1111, 1072, 1027, 978, 940, 889, 861, 845, 721.

### Katalysator K-21: 1-(3-((3-(N-Morpholino)-2,2-dimethylpropyliden)amino)propyl)-2,3-dicyclohexylguanidin

In einem Rundkolben wurden 7.56 g der vorgängig hergestellten Verbindung A-3 mit 4.11 g DCC vermischt und unter Rühren auf 90 °C erwärmt. Nach 7 Tagen war die Carbodiimid-Bande bei ca. 2120 cm⁻¹ (FT-IR-Spektroskopie) vollständig verschwunden. Darauf wurde das Reaktionsgemisch im Vakuum von den flüchtigen Bestandteilen befreit. Man erhielt 8.63 g eines gelben, niedrigviskosen, geruchlosen Öls.
FT-IR: 3355, 2923, 2849, 2807, 1636, 1504, 1449, 1360, 1345, 1282, 1268, 1257, 1236, 1136, 1116, 1070, 1051, 1012, 978, 929, 888, 864, 802, 713.

### Herstellung von Silangruppen-haltigen Polyethern:

### Polymer STP-1:

Unter Feuchtigkeitsausschluss wurden 1000 g Polyol Acclaim® 12200 (Polyoxypropylendiol mit niedrigem Ungesättigtheitsgrad, von Bayer; OH-Zahl 11.0 mg KOH/g), 43.6 g Isophorondiisocyanat (IPDI; Vestanat® IPDI, von Evonik), 126.4 g Diisodecylphthalat (DIDP) und 0.1 g Bismuttris(neodecanoat) (10 Gew.-% in DIDP) unter stetigem Rühren auf 90 °C aufgeheizt und auf dieser Temperatur belassen, bis der titrimetrisch bestimmte Gehalt an freien Isocyanatgruppen einen stabilen Wert von 0.63 Gew.-% erreicht hatte. Anschliessend wurden 63.0 g N-(3-Trimethoxysilylpropyl)-amino-bernsteinsäurediethylester (Addukt aus 3-Aminopropyltrimethoxysilan und Maleinsäurediethylester; hergestellt nach den Angaben in US 5,364,955) eingemischt und die Mischung bei 90 °C solange gerührt, bis mittels FT-IR-Spektroskopie kein freies Isocyanat mehr nachgewiesen wurde. Der so erhaltene Trimethoxysilangruppen-haltige Polyether mit einem Silan-Equivalentgewicht von ca. 6880 g/Eq (aus den Einsatzmengen berechnet) wurde auf Raumtemperatur abgekühlt und unter Ausschluss von Feuchtigkeit aufbewahrt.

### Polymer STP-2:

Unter Feuchtigkeitsausschluss wurden 1000 g Polyol Acclaim® 12200 (Polyoxypropylendiol mit niedrigem Ungesättigtheitsgrad, von Bayer; OH-Zahl 11.0 mg KOH/g), 43.6 g Isophorondiisocyanat (IPDI; Vestanat® IPDI, von Evonik), 126.4 g Diisodecylphthalat (DIDP) und 0.1 g Bismuttris(neodecanoat) (10 Gew.-% in DIDP) unter stetigem Rühren auf 90 °C aufgeheizt und auf dieser Temperatur belassen, bis der titrimetrisch bestimmte Gehalt an freien Isocyanatgruppen einen stabilen Wert von 0.64 Gew.-% erreicht hatte. Anschliessend wurden 70.6 g N-(3-Triethoxysilylpropyl)-amino-bernsteinsäurediethylester (Addukt aus 3-Aminopropyltriethoxysilan und Maleinsäurediethylester) eingemischt und die Mischung bei 90 °C solange gerührt, bis mittels FT-IR-Spektroskopie kein freies Isocyanat mehr nachgewiesen wurde. Der so erhaltene Triethoxysilangruppen-haltige Polyether mit einem Silan-Equivalentgewicht von ca. 6920 g/Eq (aus den Einsatzmengen berechnet) wurde auf Raumtemperatur abgekühlt und unter Ausschluss von Feuchtigkeit aufbewahrt.

### Verwendete kommerzielle Katalysatoren:

- DBU: 1,8-Diazabicyclo[5.4.0]undec-7-en (Lupragen® N 700, von BASF)
- TMG: 1,1,3,3-Tetramethylguanidin (von Sigma-Aldrich)
- IBAY: Bis(ethylacetoacetato)diisobutoxy-titan(IV) (Tyzor® IBAY, von Dorf Ketal)

### Zusammensetzungen auf Basis von Silangruppen-haltigen Polymeren:

Vergleichsbeispiele sind in den Tabellen 1 bis 8 mit "(Ref)" gekennzeichnet.

### Zusammensetzungen Z1 bis Z26:

Eine Zusammensetzung aus 96.5 g Polymer STP-1, 0.5 g Vinyltrimethoxysilan und 3.0 g 3-Aminopropyltrimethoxysilan wurde mit verschiedenen Katalysatoren in der angegebenen Menge gemäss Tabelle 1 vermengt und die Mischung auf Viskosität und Hautbildungszeit (HBZ) im Normklima, vor und nach Lagerung, geprüft. Die Hautbildungszeit dient dabei als Mass für die Aktivität des Katalysators in Bezug auf die Vernetzungsreaktion der Silangruppen, d.h. für die Vernetzungsgeschwindigkeit; die Veränderung der Viskosität und der Hautbildungszeit nach Lagerung sind ein Mass für die Lagerstabilität. Weiterhin wurde die applizierte Mischung nach 24 Stunden im Normklima daraufhin geprüft, ob die Oberfläche wie gewünscht trocken war oder sich ein schmieriger Film gebildet hatte, was ein Anzeichen für das Ausschwitzen des Katalysators aufgrund schlechter Verträglichkeit mit dem ausgehärteten Kunststoff ist, und/oder ob die Oberfläche klebrig war, was ein Anzeichen für eine unvollständige Aushärtung ist. Weiterhin wurde von der Mischung ein Film von 2 mm Dicke hergestellt, während 7 Tagen im Normklima aushärten lassen und auf mechanische Eigenschaften geprüft. Die Ergebnisse sind in Tabelle 1 und 2 wiedergegeben. "Zus." steht für "Zusammensetzung".

**Tabelle 1:**

| **Zus.** | **Katalysator** | **Menge** | **Konzentration¹** | **Viskosität [Pa·s]** | | | **HBZ** | |
|---|---|---|---|---|---|---|---|---|
| | | | | **frisch** | **gelagert²** | **Zunahme** | **frisch** | **gelagert²** |
| **Z1** | K-1³ | 0.70 g | 1.90 | 28.1 | 36.3 | 29% | 16' | 23' |
| **Z2** | K-2³ | 0.70 g | 1.90 | 30.2 | 41.8 | 38% | 12' | 18' |
| **Z3** | K-3 | 4.66 g | 2.00 | 26.4 | 29.5 | 12% | 38' | 30' |
| **Z4** | K-4 | 4.73 g | 2.00 | 34.6 | 47.2 | 36% | 26' | 21' |
| **Z5** | K-5 | 4.89 g | 2.00 | 32.6 | 37.6 | 15% | 20' | 29' |
| **Z6** | K-6 | 4.52 g | 2.00 | 29.0 | 31.0 | 7% | 30' | 21' |
| **Z7** | K-7 | 12.80 g | 1.90 | 32.5 | 50.8 | 56% | 32' | 11' |
| **Z8** | K-8 | 12.65 g | 1.90 | 26.5 | 49.3 | 86% | 51' | 11' |
| **Z9** | K-9³ | 0.85 g | 1.90 | 21.4 | 26.2 | 22% | 12' | 21' |
| **Z10** | K-9³ | 0.11 g | 0.25 | 25.2 | 32.9 | 31% | 44' | 80' |
| | IBAY | 0.25 g | 0.50 | | | | | |
| **Z11** | K-10 | 1.02 g | 1.90 | 22.0 | 32.8 | 49% | 10' | 25' |
| **Z12** | K-11³ | 0.84 g | 1.90 | 19.4 | 25.5 | 31% | 33' | 31' |
| **Z13** | K-12³ | 0.85 g | 1.90 | 20.8 | 26.2 | 26% | 9' | 22' |
| **Z14** | K-13³ | 1.42 g | 1.90 | 20.1 | 30.5 | 52% | 14' | 21' |
| **Z15** | K-13³ | 0.19 g | 0.25 | 27.3 | 36.3 | 33% | 75' | 80' |
| | IBAY | 0.25 g | 0.50 | | | | | |
| **Z16** | K-14³ | 0.70 g | 1.90 | 21.0 | 32.0 | 52% | 10' | 11' |
| **Z17** | K-15³ | 0.67 g | 1.90 | 25.0 | 44.6 | 78% | 14' | 9' |
| **Z18** | K-16³ | 0.77 g | 1.90 | 27.8 | 59.6 | 114% | 7' | 7' |
| **Z19** | K-17³ | 1.00 g | 1.90 | 26.4 | 43.8 | 66% | 20' | 13' |
| **Z20** | K-18³ | 1.00 g | 1.90 | 34.5 | 48.6 | 41% | 21' | 18' |
| **Z21** | K-19 | 1.86 g | 1.90 | 36.2 | 52.2 | 44% | 23' | 18' |
| **Z22** | K-20 | 1.00 g | 1.90 | 35.4 | 51.8 | 46% | 25' | 19' |
| **Z23** | K-21 | 0.80 g | 1.90 | 43.3 | 77.3 | 79% | 14' | 7' |
| **Z24** (Ref) | DBU | 0.28 g | 1.90 | 27.2 | 36.9 | 36% | 25' | 29' |
| **Z25** (Ref) | TMG | 0.21 g | 1.90 | 22.3 | 24.6 | 10% | 65' | 75' |
| **Z26** (Ref) | DBU | 0.04 g | 0.25 | 26.9 | 28.9 | 7% | 54' | 90' |
| | IBAY | 0.25 g | 0.50 | | | | | |

| | | | | | | | | |
|---|---|---|---|---|---|---|---|---|
| ¹ mmol Amidin- oder Guanidin-Gruppen bzw. Metallatome auf 100 g Silangruppen-haltiger Polyether. ² während 7 Tagen bei 60 °C in geschlossenem Gebinde. ³ als Lösung (40 Gew.-%) in N-Ethylpyrrolidon. | | | | | | | | |

**Tabelle 2:**

| **Zus.** | **Oberfläche nach 24h** | **Zugfestigkeit** | **Bruchdehnung** | **E-Modul** | |
|---|---|---|---|---|---|
| | | | | **0-5%** | **0-50%** |
| **Z1** | trocken | 0.74 MPa | 123% | 1.00 MPa | 0.72 MPa |
| **Z2** | trocken | 0.67 MPa | 104% | 1.02 MPa | 0.71 MPa |
| **Z3** | trocken | 0.82 MPa | 133% | 0.82 MPa | 0.74 MPa |
| **Z4** | trocken | 0.76 MPa | 124% | 1.26 MPa | 0.74 MPa |
| **Z5** | trocken | 0.81 MPa | 139% | 1.21 MPa | 0.72 MPa |
| **Z6** | trocken | 0.79 MPa | 126% | 1.07 MPa | 0.74 MPa |
| **Z7** | trocken | 0.73 MPa | 136% | 0.94 MPa | 0.66 MPa |
| **Z8** | trocken | 0.70 MPa | 115% | 1.00 MPa | 0.70 MPa |
| **Z9** | trocken | 0.72 MPa | 97% | 1.14 MPa | 0.79 MPa |
| **Z10** | trocken | 0.66 MPa | 88% | 0.90 MPa | 0.77 MPa |
| **Z11** | trocken | 0.73 MPa | 106% | 1.02 MPa | 0.77 MPa |
| **Z12** | trocken | 0.69 MPa | 95% | 0.98 MPa | 0.77 MPa |
| **Z13** | trocken | 0.96 MPa | 140% | 1.10 MPa | 0.84 MPa |
| **Z14** | trocken | 0.68 MPa | 95% | 1.00 MPa | 0.78 MPa |
| **Z15** | trocken | 0.75 MPa | 116% | 0.90 MPa | 0.75 MPa |
| **Z16** | trocken | 0.59 MPa | 71% | 0.94 MPa | 0.78 MPa |
| **Z17** | trocken | 0.68 MPa | 91% | 1.00 MPa | 0.78 MPa |
| **Z18** | trocken | 0.60 MPa | 77% | 0.98 MPa | 0.77 MPa |
| **Z19** | trocken | 0.61 MPa | 79% | 0.94 MPa | 0.77 MPa |
| **Z20** | fast trocken | 0.63 MPa | 82% | 1.08 MPa | 0.80 MPa |
| **Z21** | fast trocken | 0.62 MPa | 81% | 1.14 MPa | 0.81 MPa |
| **Z22** | trocken | 0.66 MPa | 89% | 1.10 MPa | 0.80 MPa |
| **Z23** | trocken | 0.57 MPa | 65% | 1.25 MPa | 0.81 MPa |
| **Z24**(Ref) | schmierig | 0.58 MPa | 72% | 1.16 MPa | 0.77 MPa |
| **Z25**(Ref) | klebrig | 0.62 MPa | 90% | 1.19 MPa | 0.75 MPa |
| **Z26**(Ref) | trocken | 0.66 MPa | 91% | 0.93 MPa | 0.74 MPa |

### Zusammensetzungen Z27 bis Z37:

Eine Zusammensetzung aus 95.9 g Polymer STP-2, 0.4 g Vinyltriethoxysilan und 3.7 g N-(2-Aminoethyl)-3-aminopropyltriethoxysilan wurde mit verschiedenen Katalysatoren in der angegebenen Menge gemäss Tabelle 3 vermengt und die Mischung wie für Zusammensetzung Z1 beschrieben auf Viskosität, Hautbildungszeit (HBZ), Oberflächenbeschaffenheit und mechanische Eigenschaften geprüft. Die Ergebnisse sind in Tabelle 3 und 4 wiedergegeben. "Zus." steht für "Zusammensetzung".

**Tabelle 3:**

| **Zus.** | **Kataly sator** | **Menge** | **Konzentration¹** | **Viskosität [Pa·s]** | | | **HBZ** | |
|---|---|---|---|---|---|---|---|---|
| | | | | **frisch** | **gelagert²** | **Zunahme** | **frisch** | **gelagert²** |
| **Z27** | K-3 | 9.72 g | 4.2 | 36.4 | 66.4 | 82% | 4h 20' | 1h 23' |
| **Z28** | K-4 | 9.86 g | 4.2 | 40.8 | 48.9 | 20% | 4h 29' | 2h 24' |
| **Z29** | K-5 | 10.20 g | 4.2 | 41.5 | 47.8 | 15% | 4h 11' | 2h 53' |
| **Z30** | K-6 | 9.43 g | 4.2 | 39.2 | 42.0 | 7% | 4h | 3h 36' |
| **Z31** | K-9 | 1.68 g | 3.8 | 79.6 | 101.9 | 28% | 1h 16' | 1h 25' |
| **Z32** | K-10 | 2.02 g | 3.8 | 76.3 | 96.6 | 27% | 1h 49' | 1h 51' |
| **Z33** | K-11³ | 1.67 g | 3.8 | 58.4 | 83.0 | 42% | 1h 33' | 1h 21' |
| **Z34** | K-12³ | 1.68 g | 3.8 | 58.2 | 97.5 | 68% | 38' | 33' |
| **Z35** | K-13 | 2.83 g | 3.8 | 36.6 | 61.0 | 67% | 1h 07' | 1h 15' |
| **Z36** (Ref) | DBU | 0.55 g | 3.8 | 48.8 | 58.1 | 19% | 2h 7' | 2h 35' |
| **Z37** (Ref) | TMG | 0.42 g | 3.8 | 44.5 | 53.4 | 20% | >12h | >12h |

| | | | | | | | | |
|---|---|---|---|---|---|---|---|---|
| ¹ mmol Amidin- oder Guanidin-Gruppen auf 100 g Silangruppen-haltiger Polyether. ² während 7 Tagen bei 60 °C in geschlossenem Gebinde. ³ als Lösung (40 Gew.-%) in N-Ethylpyrrolidon. | | | | | | | | |

**Tabelle 4:**

| **Zus.** | **Oberfläche nach 24h** | **Zugfestigkeit** | **Bruchdehnung** | **E-Modul** | |
|---|---|---|---|---|---|
| | | | | **0-5%** | **0-50%** |
| **Z27** | leicht klebrig | 0.52 MPa | 129% | 0.39 MPa | 0.43 MPa |
| **Z28** | trocken | 0.67 MPa | 160% | 0.86 MPa | 0.52 MPa |
| **Z29** | trocken | 0.67 MPa | 167% | 0.81 MPa | 0.49 MPa |
| **Z30** | trocken | 0.57 MPa | 135% | 0.67 MPa | 0.48 MPa |
| **Z31** | fast trocken | 0.59 MPa | 241% | 0.31 MPa | 0.31 MPa |
| **Z32** | fast trocken | 0.50 MPa | 194% | 0.31 MPa | 0.30 MPa |
| **Z33** | fast trocken | 0.54 MPa | 225% | 0.30 MPa | 0.29 MPa |
| **Z34** | fast trocken | 0.60 MPa | 233% | 0.30 MPa | 0.30 MPa |
| **Z35** | fast trocken | 0.56 MPa | 129% | 0.68 MPa | 0.50 MPa |
| **Z36** (Ref) | schmierig, stark klebrig | 0.43 MPa | 157% | 0.28 MPa | 0.28 MPa |
| **Z37** (Ref) | sehr stark klebrig | n.b. | n.b. | n.b. | n.b. |

| | | | | | |
|---|---|---|---|---|---|
| n.b. = nicht bestimmt bzw. nicht messbar. | | | | | |

### Zusammensetzungen Z38 bis Z42:

In einem Planetenmischer wurden 30.8 g Polymer STP-1, 25.7 g gemahlene Kreide (Omyacarb® 5 GU, von Omya), 25.7 g gefällte Kreide (Socal® U1S2, von Solvay), 15.4 g Diisononyl-1,2-cyclohexandicarboxylat (Hexamoll® DINCH, von BASF), 1.2 g Vinyltrimethoxysilan, 1.2 g 3-Aminopropyltrimethoxysilan sowie verschiedene Katalysatoren in der angegebenen Menge gemäss Tabelle 5 vermengt und die Mischung wie für Zusammensetzung Z1 beschrieben auf Hautbildungszeit (HBZ), Oberflächenbeschaffenheit und mechanische Eigenschaften geprüft. Die Ergebnisse sind in Tabelle 5 wiedergegeben. "Zus." steht für "Zusammensetzung".

**Tabelle 5:**

| **Zus.** | **Katalysator** | **Menge** | **Konzentration¹** | **HBZ** | **Oberfläche nach 24h** | **Zugfestigkeit** | **Bruchdehnung** | **E-Modul [MPa]** | |
|---|---|---|---|---|---|---|---|---|---|
| | | | | | | | | **0-5%** | **0-100%** |
| **Z38** | K-1² | 0.19 g | 0.5 | 71' | trocken | 2.0 MPa | 263% | 2.0 | 1.2 |
| **Z39** | K-2² | 0.19 g | 0.5 | 29' | trocken | 2.0 MPa | 255% | 1.9 | 1.2 |
| **Z40** | K-7 | 2.99 g | 0.4 | 23' | trocken | 2.0 MPa | 248% | 1.9 | 1.2 |
| **Z41** | K-8 | 2.96 g | 0.4 | 22' | trocken | 1.9 MPa | 275% | 1.9 | 1.1 |
| **Z42** (Ref) | DBU | 0.08 g | 0.5 | 35' | schmierig | 2.1 MPa | 283% | 1.8 | 1.2 |

| | | | | | | | | | |
|---|---|---|---|---|---|---|---|---|---|
| ¹mmol Amidin- oder Guanidin-Gruppen auf 100 g Zusammensetzung. ² als Lösung (40 Gew.-%) in N-Ethylpyrrolidon. | | | | | | | | | |

### Zusammensetzungen Z43 bis Z52:

In einem Planetenmischer wurden 36.2 g Polymer STP-1, 60.2 g gemahlene Kreide (Omyacarb® 5 GU, von Omya), 1.2 g Thixotropierpaste hergestellt wie nachstehend beschrieben, 1.2 g Vinyltrimethoxysilan, 1.2 g 3-Aminopropyltrimethoxysilan sowie verschiedene Katalysatoren in der angegebenen Menge gemäss Tabelle 6 vermengt und die Mischung wie für Zusammensetzung Z1 beschrieben auf Hautbildungszeit (HBZ), Oberflächenbeschaffenheit und mechanische Eigenschaften geprüft. Die Ergebnisse sind in Tabelle 6 wiedergegeben. "Zus." steht für "Zusammensetzung".

Die Thixotropierpaste wurde hergestellt, indem in einem Vakuummischer 300 g Diisodecylphthalat (Palatinol® Z, von BASF) und 48 g 4,4'-Methylendiphenyldiisocyanat (Desmodur® 44 MC L, von Bayer) vorgelegt und leicht aufgewärmt und anschliessend unter starkem Rühren 27 g n-Butylamin langsam zugetropft wurden. Die entstehende Paste wurde unter Vakuum und Kühlung eine Stunde weitergerührt.

**Tabelle 6:**

| **Zus.** | **Katalysator** | **Menge** | **Konzentration¹** | **HBZ** | **Oberfläche nach 24h** | **Zugfestigkeit** | **Bruchdehnung** | **E-Modul [MPa]** | |
|---|---|---|---|---|---|---|---|---|---|
| | | | | | | | | **0-5%** | **0-100%** |
| **Z43** | K-3 | 4.83 g | 2.0 | 27' | trocken | 2.7 MPa | 132% | 4.2 | 2.4 |
| **Z44** | K-4 | 4.90 g | 2.0 | 15' | trocken | 2.7 MPa | 118% | 4.5 | 2.5 |
| **Z45** | K-5 | 5.07 g | 2.0 | 16' | trocken | 2.6 MPa | 114% | 4.5 | 2.4 |
| **Z46** | K-6 | 4.68 g | 2.0 | 11' | trocken | 3.0 MPa | 134% | 4.6 | 2.5 |
| **Z47** | K-9 | 0.18 g | 0.4 | 27' | trocken | 2.9 MPa | 117% | 6.0 | 2.6 |
| **Z48** | K-10 | 0.22 g | 0.4 | 31' | trocken | 2.7 MPa | 91% | 6.1 | - |
| **Z49** | K-11² | 0.18 g | 0.4 | 21' | trocken | 2.4 MPa | 69% | 6.0 | - |
| **Z50** | K-12² | 0.18 g | 0.4 | 33' | trocken | 2.9 MPa | 116% | 5.9 | 2.6 |
| **Z51** | K-13 | 0.31 g | 0.4 | 25' | trocken | 2.9 MPa | 104% | 5.9 | 2.7 |
| **Z52** (Ref) | DBU | 0.12 g | 0.8 | 25' | leicht schmierig | 2.5 MPa | 103% | 6.1 | 2.8 |

| | | | | | | | | | |
|---|---|---|---|---|---|---|---|---|---|
| ¹ mmol Amidin- oder Guanidin-Gruppen auf 100 g Zusammensetzung. ² als Lösung (40 Gew.-%) in N-Ethylpyrrolidon. | | | | | | | | | |

### Zusammensetzungen Z53 bis Z61:

In einem Planetenmischer wurden 36.2 g Polymer STP-2, 60.2 g gemahlene Kreide (Omyacarb® 5 GU, von Omya), 1.2 g Thixotropierpaste, hergestellt wie für Zusammensetzung Z27 beschrieben, 1.2 g Vinyltriethoxysilan, 1.2 g 3-Aminopropyltriethoxysilan sowie verschiedene Katalysatoren in der angegebenen Menge gemäss Tabelle 7 vermengt und die Mischung wie für Zusammensetzung Z1 beschrieben auf Hautbildungszeit (HBZ), Oberflächenbeschaffenheit und mechanische Eigenschaften geprüft. Die Ergebnisse sind in Tabelle 7 wiedergegeben. "Zus." steht für "Zusammensetzung".

**Tabelle 7:**

| **Zus.** | **Katalysator** | **Menge** | **Konzentration¹** | **HBZ** | **Oberfläche nach 24h** | **Zugfestigkeit** | **Bruchdehnung** | **E-Modul [MPa]** | |
|---|---|---|---|---|---|---|---|---|---|
| | | | | | | | | **0-5%** | **0-100%** |
| **Z53** | K-4 | 9.79 g | 4.0 | 169' | trocken | 2.0 MPa | 167% | 3.1 | 1.5 |
| **Z54** | K-5 | 10.13 g | 4.0 | 69' | trocken | 2.1 MPa | 164% | 3.6 | 1.6 |
| **Z55** | K-6 | 9.36 g | 4.0 | 125' | trocken | 2.4 MPa | 180% | 3.1 | 1.7 |
| **Z56** | K-9 | 1.20 g | 2.6 | 67' | I. klebrig | 2.6 MPa | 108% | 4.9 | 2.5 |
| **Z57** | K-10 | 1.44 g | 2.6 | 83' | I. klebrig | 2.6 MPa | 127% | 4.6 | 2.3 |
| **Z58** | K-11² | 1.19 g | 2.6 | 96' | I. klebrig | 2.9 MPa | 145% | 5.0 | 2.4 |
| **Z59** | K-12² | 1.20 g | 2.6 | 77' | I. klebrig | 2.2 MPa | 106% | 6.7 | 1.9 |
| **Z60** | K-13 | 2.02 g | 2.6 | 79' | I. klebrig | 2.5 MPa | 127% | 5.9 | 2.2 |
| **Z61** (Ref) | DBU | 0.40 g | 2.6 | 83' | leicht schmierig | 2.5 MPa | 155% | 4.0 | 2.0 |

| | | | | | | | | | |
|---|---|---|---|---|---|---|---|---|---|
| ¹mmol Amidin- oder Guanidin-Gruppen auf 100 g Silangruppen-haltiger Polyether. ² als Lösung (40 Gew.-%) in N-Ethylpyrrolidon. "I." steht für "leicht" | | | | | | | | | |

### Zusammensetzungen Z62 und Z63:

Eine Zusammensetzung aus 96.0 g Silangruppen-haltigem Polyether GENIOSIL® STP-E 15 (von Wacker), 0.35 g Vinyltrimethoxysilan und 3.72 g 3-Aminopropyltrimethoxysilan wurde mit verschiedenen Katalysatoren in der angegebenen Menge gemäss Tabelle 8 vermengt und die Mischung wie für Zusammensetzung Z1 beschrieben auf Hautbildungszeit (HBZ), Oberflächenbeschaffenheit und mechanische Eigenschaften geprüft. Die Ergebnisse sind in Tabelle 8 wiedergegeben. "Zus." steht für "Zusammensetzung".

**Tabelle 8:**

| **Zus.** | **Katalysator** | **Menge** | **Konzentration ¹** | **HBZ** | **Oberfläche nach 24h** | **Zugfestigkeit** | **Bruchdehnung** | **E-Modul 0-5%** |
|---|---|---|---|---|---|---|---|---|
| **Z62** | K-13 | 1.51 g | 1.9 | 23' | trocken | 0.82 MPa | 60% | 1.77 MPa |
| **Z63** (Ref) | DBU | 0.28 g | 1.9 | 60' | schmierig | 0.72 MPa | 48% | 1.84 MPa |

| | | | | | | | | |
|---|---|---|---|---|---|---|---|---|
| ¹ mmol Amidin- oder Guanidin-Gruppen auf 100 g Silangruppen-haltiger Polyether. | | | | | | | | |

## Patentansprüche

1. Verwendung eines Katalysators der Formel (I), 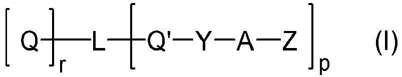 wobei
p für eine ganze Zahl von 1 bis 6 steht und r für eine ganze Zahl von 0 bis 5 steht, wobei (p+r) für eine ganze Zahl von 1 bis 6 steht,
L für
einen (p+r)-wertigen Kohlenwasserstoffrest mit einem mittleren Molekulargewicht im Bereich von 14 bis 20'000 g/mol, welcher gegebenenfalls Heteroatome aufweist,
oder für einen (p+r+1)-wertigen Kohlenwasserstoffrest mit 4 bis 12 C-Atomen, welcher zusammen mit Q' einen gegebenenfalls substituierten 5- oder 6-gliedrigen Ring bildet,
oder für eine kovalente Bindung,
oder für einen Wasserstoff-Rest steht,
Q für eine Reaktivgruppe ausgewählt aus Epoxid, Aziridin, Carbonat, Carbonsäureanhydrid, Carbonsäure, Carbonsäureester, Lacton, Carbonsäurechlorid, Keton, Aldehyd, 1,3-Diketon, 1,3-Ketoester, 1,3-Ketoamid, Cyanat, Thiocyanat, Isocyanat, Isothiocyanat, (Meth)acrylat, (Meth)acrylamid, (Meth)acrylnitril, Maleat, Maleamid, Maleimid, Fumarat, Fumaramid, Itaconat, Itaconamid, Crotonat und Crotonamid steht, Q' für eine zwei- oder dreiwertige Verbindungseinheit entstanden aus der Reaktion von Q mit HX steht,
Y für N oder X steht, wobei X für O oder S oder NR³ steht, wobei R³ für einen Wasserstoff-Rest oder für einen Alkyl- oder Cycloalkyl- oder Aralkyl-Rest mit 1 bis 8 C-Atomen, welcher gegebenenfalls eine tertiäre Aminogruppe oder eine Guanidin-Gruppe enthält, steht,
A für einen zweiwertigen Kohlenwasserstoff-Rest mit 2 bis 30 C-Atomen, welcher gegebenenfalls ungesättigte Anteile und gegebenenfalls Ether-Sauerstoff oder sekundären oder tertiären Amin-Stickstoff enthält, steht,
wobei A auch zusammen mit R³ für einen dreiwertigen Kohlenwasserstoff-Rest mit 5 bis 10 C-Atomen, welcher gegebenenfalls einen tertiären Amin-Stickstoff enthält, stehen kann, und
Z für eine über ein Stickstoffatom gebundene Guanidingruppe steht, welche kein Stickstoffatom enthält, welches direkt an einen aromatischen Ring gebunden oder Teil eines heteroaromatischen Ringsystems ist,
für die Vernetzung einer härtbaren Zusammensetzung.

2. Verwendung gemäss Anspruch 1, **dadurch gekennzeichnet, dass** A entweder ausgewählt ist aus der Gruppe bestehend aus 1,2-Ethylen, 1,2-Propylen, 1,3-Propylen, 1,3-Pentylen, 1,1-Dimethyl-1,2-ethyl, 1,5-Pentylen, 2-Methyl-1,5-pentylen, 1,6-Hexylen, 2,2(4),4-Trimethyl-1,6-hexamethylen, 1,8-Octylen, 1,10-Decylen, 1,12-Dodecylen, (1,5,5-Trimethylcyclohexan-1-yl)methan-1,3, 1,3-Cyclohexylen-bis(methylen), 1,4-Cyclohexylen-bis(methylen), 1,3-Phenylen-bis(methylen), 2- und/oder 4-Methyl-1,3-cyclohexylen, N-Methyl-4-aza-1,7-heptylen, 3-Oxa-1,5-pentylen, 3,6-Dioxa-1,8-octylen, 4,7-Dioxa-1,10-decylen und einem Polyoxypropylen-Rest mit einem mittleren Molekulargewicht im Bereich von etwa 200 bis 250 g/mol, oder A steht zusammen mit R³ einschliesslich des Stickstoffatoms von X für einen Rest ausgewählt aus der Gruppe bestehend aus Piperidin-4-ylmethyl, 2-(Piperidin-4-yl)ethyl und 2-Piperazinoethyl.

3. Verwendung gemäss einem der vorhergehenden Ansprüche, **dadurch gekennzeichnet, dass** Z für 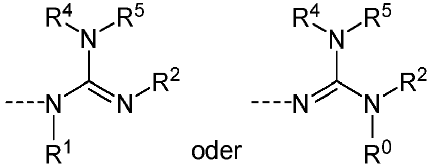 steht, wobei
R⁰ und R¹ unabhängig voneinander für einen Wasserstoff-Rest oder für einen Alkyl- oder Cycloalkyl- oder Aralkyl-Rest mit 1 bis 8 C-Atomen stehen,
R² für einen Wasserstoff-Rest oder für einen Alkyl-, Cycloalkyl- oder Aralkyl-Rest mit 1 bis 18 C-Atomen, welcher gegebenenfalls Ether-Sauerstoff oder tertiären Amin-Stickstoff enthält, steht,
R⁴ und R⁵ unabhängig voneinander jeweils für einen Wasserstoff-Rest oder für einen Alkyl-, Cycloalkyl- oder Aralkyl-Rest mit 1 bis 18 C-Atomen, welcher gegebenenfalls einen Ether-Sauerstoff oder einen tertiären Amin-Stickstoff enthält, stehen,
wobei
R² und R⁰ auch zusammen für einen Alkylen-Rest mit 3 bis 6 C-Atomen, welcher gegebenenfalls einen Ether-Sauerstoff oder einen tertiären Amin-Stickstoff enthält, stehen können,
R⁴ und R⁵ auch zusammen für einen Alkylen-Rest mit 4 bis 7 C-Atomen, welcher gegebenenfalls einen Ether-Sauerstoff oder einen tertiären Amin-Stickstoff enthält, stehen können, und
R² und R⁵ auch zusammen für einen Alkylen-Rest mit 2 bis 12 C-Atomen stehen können.

4. Verwendung gemäss Anspruch 3, **dadurch gekennzeichnet, dass** Z für 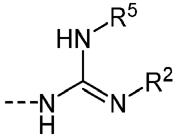 steht.

5. Verwendung gemäss einem der vorhergehenden Ansprüche, **dadurch gekennzeichnet, dass** Q' ausgewählt ist aus der Gruppe bestehend aus 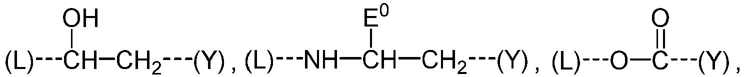 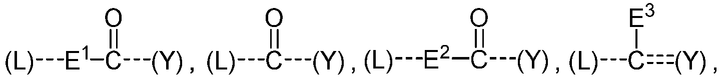 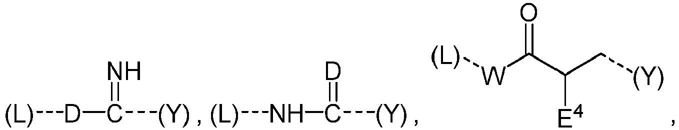 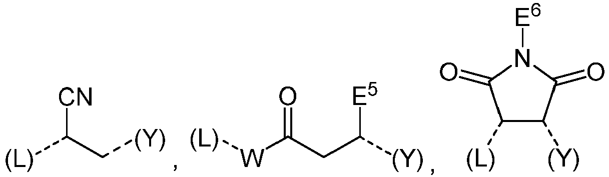 und 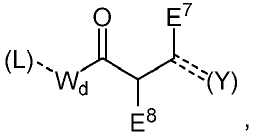 wobei
D für O oder S steht,
W für O oder NR⁶ steht, wobei R⁶ für einen Wasserstoff-Rest oder für einen einwertigen Kohlenwasserstoffrest mit 1 bis 8 C-Atomen steht, E⁰ für einen Wasserstoff-Rest oder für einen Methyl-Rest steht,
E¹ für einen Carboxyl-substituierten Alkylen-, Alkendiyl- oder PhenylenRest mit 2 bis 8 C-Atomen steht,
E² für einen Hydroxyalkylen-Rest mit 2 bis 5 C-Atomen oder für einen über O gebundenen Hydroxyalkylenoxy-Rest mit 2 oder 3 C-Atomen steht,
E³ für einen Wasserstoff-Rest oder für einen einwertigen Kohlenwasserstoffrest mit 1 bis 6 C-Atomen, welcher gegebenenfalls Heteroatome in Form von Ether-, Ester-, Amino- oder Amid-Gruppen aufweist, oder zusammen mit L für einen gegebenenfalls substituierten 1,4-Butylen- oder 1,5-Pentylen-Rest steht,
E⁴ für einen Wasserstoff- oder Methyl- oder Alkoxycarbonylmethyl-Rest mit 2 bis 9 C-Atomen steht,
E⁵ für einen Alkyoxycarbonyl-Rest mit 1 bis 8 C-Atomen oder für einen Methyl-Rest steht,
E⁶ für einen Wasserstoff-Rest oder für einen Alkyl-Rest mit 1 bis 8 C-Atomen steht,
E⁷ für einen einwertigen Kohlenwasserstoffrest mit 1 bis 6 C-Atomen steht,
E⁸ für einen Wasserstoff-Rest oder für einen einwertigen Kohlenwasserstoffrest mit 1 bis 6 C-Atomen steht, und
d für 0 oder 1 steht.

6. Verwendung gemäss einem der vorhergehenden Ansprüche, **dadurch gekennzeichnet, dass** p für 1 oder 2 oder 3 und r für 0 stehen.

7. Verwendung gemäss einem der vorhergehenden Ansprüche, **dadurch gekennzeichnet, dass** die härtbare Zusammensetzung eine Epoxidharz-Zusammensetzung oder eine Polyurethan-Zusammensetzung oder eine Epoxidharz-Polyurethan-Zusammensetzung oder eine Cyanatesterharz-Zusammensetzung oder eine Silangruppen-haltige Zusammensetzung ist.

8. Verwendung gemäss einem der vorhergehenden Ansprüche, **dadurch gekennzeichnet, dass** die härtbare Zusammensetzung eine Zusammensetzung auf Basis von Silangruppen-haltigen Polymeren ist.

9. Verwendung gemäss Anspruch 8, **dadurch gekennzeichnet, dass** das Silangruppen-haltige Polymer ausgewählt ist aus der Gruppe bestehend aus Polyorganosiloxanen mit endständigen Silangruppen und Silangruppen-haltigen organischen Polymeren.

10. Verfahren zur Herstellung des Katalysators der Formel (I) wie in einem der Ansprüche 1 bis 6 beschrieben, **dadurch gekennzeichnet, dass**
- mindestens ein Guanidin der Formel (II)
HX-A-Z (II)
- mit mindestens einer funktionellen Verbindung, welche mindestens eine Reaktivgruppe ausgewählt aus Epoxid, Aziridin, Carbonat, Carbonsäureanhydrid, Carbonsäure, Carbonsäureester, Lacton, Carbonsäurechlorid, Keton, Aldehyd, 1,3-Diketon, 1,3-Ketoester, 1,3-Ketoamid, Cyanat, Thiocyanat, Isocyanat, Isothiocyanat, (Meth)acrylat, (Meth)acrylamid, (Meth)acrylnitril, Maleat, Maleamid, Maleimid, Fumarat, Fumaramid, Itaconat, Itaconamid, Crotonat und Crotonamid aufweist,
umgesetzt wird.

11. Härtbare Zusammensetzung enthaltend mindestens einen Katalysator der Formel (I) wie in einem der Ansprüche 1 bis 6 beschrieben.

12. Zusammensetzung gemäss Anspruch 11, **dadurch gekennzeichnet, dass** sie weiterhin mindestens ein Silangruppen-haltiges Polymer enthält.

13. Zusammensetzung gemäss Anspruch 12, **dadurch gekennzeichnet, dass** das Silangruppen-haltige Polymer ein Polyorganosiloxan mit endständigen Silangruppen ist.

14. Zusammensetzung gemäss Anspruch 12, **dadurch gekennzeichnet, dass** das Silangruppen-haltige Polymer ein Silangruppen-haltiges organisches Polymer ist.

15. Zusammensetzung gemäss einem der Ansprüche 11 bis 14, **dadurch gekennzeichnet, dass** sie einen Klebstoff oder einen Dichtstoff oder eine Beschichtung darstellt.

## Claims

1. Use of a catalyst of the formula (I) 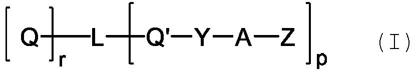 where
p is an integer from 1 to 6 and r is an integer from 0 to 5, where (p+r) is an integer from 1 to 6,
L is
a (p+r)-valent hydrocarbyl radical having a mean molecular weight in the range from 14 to 20'000 g/mol, optionally having heteroatoms,
or is a (p+r+1)-valent hydrocarbyl radical having 4 to 12 carbon atoms, which together with Q' forms an optionally substituted 5- or 6-membered ring,
or is a covalent bond,
or is a hydrogen radical,
Q is a reactive group selected from epoxide, aziridine, carbonate, carboxylic anhydride, carboxylic acid, carboxylic ester, lactone, carbonyl chloride, ketone, aldehyde, 1,3-diketone, 1,3-keto ester, 1,3-keto amide, cyanate, thiocyanate, isocyanate, isothiocyanate, (meth)acrylate, (meth)acrylamide, (meth)acrylonitrile, maleate, maleamide, maleimide, fumarate, fumaramide, itaconate, itaconamide, crotonate and crotonamide,
Q' is a di- or trivalent connecting unit formed from the reaction of Q with HX,
Y is N or X, where X is O or S or NR³, where R³ is a hydrogen radical or is an alkyl or cycloalkyl or aralkyl radical which has 1 to 8 carbon atoms and optionally contains a tertiary amino group or a guanidine group,
A is a divalent hydrocarbyl radical which has 2 to 30 carbon atoms and optionally contains unsaturated components and optionally ether oxygen or secondary or tertiary amine nitrogen,
where A together with R³ may also be a trivalent hydrocarbyl radical which has 5 to 10 carbon atoms and optionally contains a tertiary amine nitrogen, and
Z is a guanidine group which is bonded via a nitrogen atom and does not contain any nitrogen atom which is bonded directly to an aromatic ring or is part of a heteroaromatic ring system,
for the crosslinking of a curable composition.

2. Use according to Claim 1, **characterized in that** A is either selected from the group consisting of 1,2-ethylene, 1,2-propylene, 1,3-propylene, 1,3-pentylene, 1,1-dimethyl-1,2-ethyl, 1,5-pentylene, 2-methyl-1,5-pentylene, 1,6-hexylene, 2,2(4),4-trimethyl-1,6-hexamethylene, 1,8-octylene, 1,10-decylene, 1,12-dodecylene, (1,5,5-trimethylcyclohexan-1-yl)methane-1,3, 1,3-cyclohexylenebis(methylene), 1,4-cyclohexylene-bis(methylene), 1,3-phenylenebis(methylene), 2- and/or 4-methyl-1,3-cyclohexylene, N-methyl-4-aza-1,7-heptylene, 3-oxa-1,5-pentylene, 3,6-dioxa-1,8-octylene, 4,7-dioxa-1,10-decylene and a polyoxypropylene radical having a mean molecular weight in the range from about 200 to 250 g/mol, or A together with R³ including the nitrogen atom of X is a radical selected from the group consisting of piperidin-4-ylmethyl, 2-(piperidin-4-yl)ethyl and 2-piperazinoethyl.

3. Use according to either of the preceding claims, **characterized in that** Z is 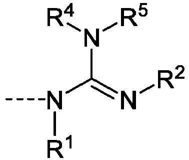 or 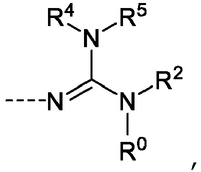 where
R⁰ and R¹ are independently a hydrogen radical or an alkyl or cycloalkyl or aralkyl radical having 1 to 8 carbon atoms,
R² is a hydrogen radical or an alkyl, cycloalkyl or aralkyl radical which has 1 to 18 carbon atoms and optionally contains ether oxygen or tertiary amine nitrogen,
R⁴ and R⁵ are each independently a hydrogen radical or an alkyl, cycloalkyl or aralkyl radical which has 1 to 18 carbon atoms and optionally contains an ether oxygen or a tertiary amine nitrogen, where
R² and R⁰ together may also be an alkylene radical which has 3 to 6 carbon atoms and optionally contains an ether oxygen or a tertiary amine nitrogen,
R⁴ and R⁵ together may also be an alkylene radical which has 4 to 7 carbon atoms and optionally contains an ether oxygen or a tertiary amine nitrogen, and
R² and R⁵ together may also be an alkylene radical having 2 to 12 carbon atoms.

4. Use according to Claim 3, **characterized in that** Z is

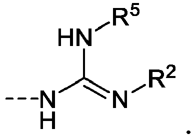

5. Use according to any of the preceding claims, **characterized in that** Q' is selected from the group consisting of 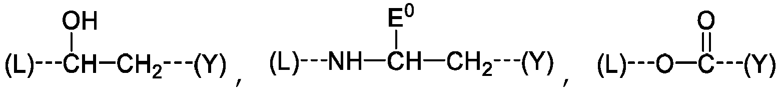 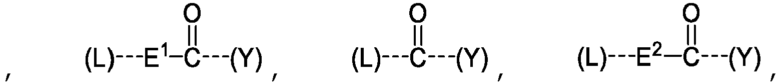 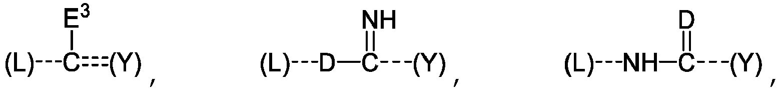 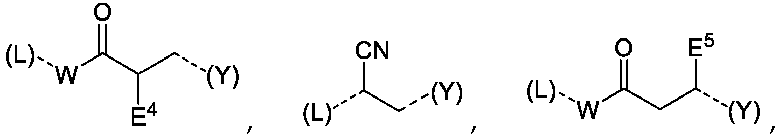 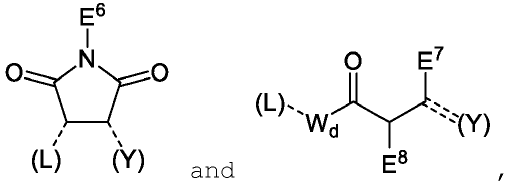 where
D is O or S,
W is O or NR⁶ where R⁶ is a hydrogen radical or is a monovalent hydrocarbyl radical having 1 to 8 carbon atoms,
E⁰ is a hydrogen radical or is a methyl radical,
E¹ is a carboxyl-substituted alkylene, alkenediyl or phenylene radical having 2 to 8 carbon atoms,
E² is a hydroxyalkylene radical having 2 to 5 carbon atoms or is an O-bonded hydroxyalkyleneoxy radical having 2 or 3 carbon atoms,
E³ is a hydrogen radical or is a monovalent hydrocarbyl radical which has 1 to 6 carbon atoms and optionally has heteroatoms in the form of ether, ester, amino or amide groups, or together with L is an optionally substituted 1,4-butylene or 1,5-pentylene radical,
E⁴ is a hydrogen or methyl or alkoxycarbonylmethyl radical having 2 to 9 carbon atoms,
E⁵ is an alkoxycarbonyl radical having 1 to 8 carbon atoms or is a methyl radical,
E⁶ is a hydrogen radical or is an alkyl radical having 1 to 8 carbon atoms,
E⁷ is a monovalent hydrocarbyl radical having 1 to 6 carbon atoms,
E⁸ is a hydrogen radical or is a monovalent hydrocarbyl radical having 1 to 6 carbon atoms, and d is 0 or 1.

6. Use according to any of the preceding claims, **characterized in that** p is 1 or 2 or 3 and r is 0.

7. Use according to any of the preceding claims, **characterized in that** the curable composition is an epoxy resin composition or a polyurethane composition or an epoxy resin/polyurethane composition or a cyanate ester resin composition or a composition containing silane groups.

8. Use according to any of the preceding claims, **characterized in that** the curable composition is a composition based on polymers containing silane groups.

9. Use according to Claim 8, **characterized in that** the polymer containing silane groups is selected from the group consisting of polyorganosiloxanes having terminal silane groups and organic polymers containing silane groups.

10. Process for preparing the catalyst of the formula (I) as described in any of Claims 1 to 6, **characterized in that**
- at least one guanidine of the formula (II)
**HX-A-Z** (II)
- is reacted with at least one functional compound having at least one reactive group selected from epoxide, aziridine, carbonate, carboxylic anhydride, carboxylic acid, carboxylic ester, lactone, carbonyl chloride, ketone, aldehyde, 1,3-diketone, 1,3-keto ester, 1,3-keto amide, cyanate, thiocyanate, isocyanate, isothiocyanate, (meth)acrylate, (meth)acrylamide, (meth)acrylonitrile, maleate, maleamide, maleimide, fumarate, fumaramide, itaconate, itaconamide, crotonate and crotonamide.

11. Curable composition comprising at least one catalyst of the formula (I) as described in any of Claims 1 to 6.

12. Composition according to Claim 11, **characterized in that** it further comprises at least one polymer containing silane groups.

13. Composition according to Claim 12, **characterized in that** the polymer containing silane groups is a polyorganosiloxane having terminal silane groups.

14. Composition according to Claim 12, **characterized in that** the polymer containing silane groups is an organic polymer containing silane groups.

15. Composition according to any of Claims 11 to 14, **characterized in that** it is an adhesive or a sealant or a coating.

## Revendications

1. Utilisation d'un catalyseur de la formule (I), 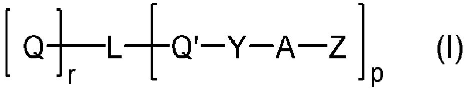 dans laquelle
p représente un nombre entier de 1 à 6 et r représente un nombre entier de 0 à 5, (p+r) représentant un nombre entier de 1 à 6,
L représente
un radical hydrocarboné (p+r)-valent ayant un poids moléculaire moyen dans la plage allant de 14 à 20 000 g/mol, qui comprend éventuellement des hétéroatomes,
ou un radical hydrocarboné (p+r+1)-valent de 4 à 12 atomes C, qui forme conjointement avec Q' un cycle à 5 ou 6 chaînons éventuellement substitué,
ou une liaison covalente,
ou un radical hydrogène,
Q représente un groupe réactif choisi parmi époxyde, aziridine, carbonate, anhydride d'acide carboxylique, acide carboxylique, ester d'acide carboxylique, lactone, chlorure d'acide carboxylique, cétone, aldéhyde, 1,3-dicétone, 1,3-cétoester, 1,3-cétoamide, cyanate, thiocyanate, isocyanate, isothiocyanate, (méth)acrylate, (méth)acrylamide, (méth)acrylonitrile, maléate, maléamide, maléimide, fumarate, fumaramide, itaconate, itaconamide, crotonate et crotonamide,
Q' représente une unité de liaison bi- ou trivalente formée par la réaction de Q avec HX,
Y représente N ou X, X représentant O ou S ou NR³, R³ représentant un radical hydrogène ou un radical alkyle ou cycloalkyle ou aralkyle de 1 à 8 atomes C, qui contient éventuellement un groupe amino tertiaire ou un groupe guanidine,
A représente un radical hydrocarboné bivalent de 2 à 30 atomes C, qui contient éventuellement des fractions insaturées et éventuellement un oxygène d'éther ou un azote d'amine secondaire ou tertiaire,
A pouvant également représenter conjointement avec R³ un radical hydrocarboné trivalent de 5 à 10 atomes C, qui contient éventuellement un azote d'amine tertiaire, et
Z représente un groupe guanidine relié par l'intermédiaire d'un atome d'azote, qui ne contient pas d'atome d'azote, qui est relié directement à un cycle aromatique ou fait partie d'un système cyclique hétéroaromatique,
pour la réticulation d'une composition durcissable.

2. Utilisation selon la revendication 1, **caractérisée en ce que** soit A est choisi dans le groupe constitué par 1,2-éthylène, 1,2-propylène, 1,3-propylène, 1,3-pentylène, 1,1-diméthyl-1,2-éthyle, 1,5-pentylène, 2-méthyl-1,5-pentylène, 1,6-hexylène, 2,2(4),4-triméthyl-1,6-hexaméthylène, 1,8-octylène, 1,10-décylène, 1,12-dodécylène, (1,5,5-triméthylcyclohexan-1-yl)méthane-1,3, 1,3-cyclohexylène-bis(méthylène), 1,4-cyclohexylène-bis(méthylène), 1,3-phénylène-bis(méthylène), 2- et/ou 4-méthyl-1,3-cyclohexylène, N-méthyl-4-aza-1,7-heptylène, 3-oxa-1,5-pentylène, 3,6-dioxa-1,8-octylène, 4,7-dioxa-1,10-décylène et un radical polyoxypropylène ayant un poids moléculaire moyen dans la plage allant d'environ 200 à 250 g/mol, soit A représente conjointement avec R³ y compris l'atome d'azote de X un radical choisi dans le groupe constitué par pipéridin-4-ylméthyle, 2-(pipéridin-4-yl)éthyle et 2-pipérazinoéthyle.

3. Utilisation selon l'une quelconque des revendications précédentes, **caractérisée en ce que** Z représente 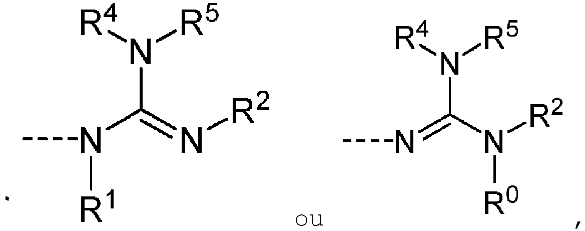
R⁰ et R¹ représentant indépendamment l'un de l'autre un radical hydrogène ou un radical alkyle ou cycloalkyle ou aralkyle de 1 à 8 atomes C,
R² représentant un radical hydrogène ou un radical alkyle, cycloalkyle ou aralkyle de 1 à 18 atomes C, qui contient éventuellement un oxygène d'éther ou un azote d'amine tertiaire,
R⁴ et R⁵ représentent chacun indépendamment l'un de l'autre un radical hydrogène ou un radical alkyle, cycloalkyle ou aralkyle de 1 à 18 atomes C, qui contient éventuellement un oxygène d'éther ou un azote d'amine tertiaire,
R² et R⁰ pouvant également représenter ensemble un radical alkylène de 3 à 6 atomes C, qui contient éventuellement un oxygène d'éther ou un azote d'amine tertiaire,
R⁴ et R⁵ pouvant également représenter ensemble un radical alkylène de 4 à 7 atomes C, qui contient éventuellement un oxygène d'éther ou un azote d'amine tertiaire, et
R² et R⁵ pouvant également représenter ensemble un radical alkylène de 2 à 12 atomes C.

4. Utilisation selon la revendication 3, **caractérisée en ce que** Z représente

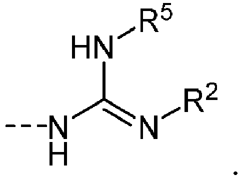

5. Utilisation selon l'une quelconque des revendications précédentes, **caractérisée en ce que** Q' est choisi dans le groupe constitué par : 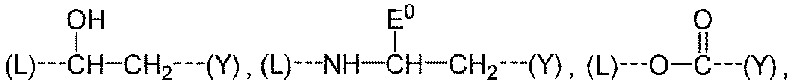 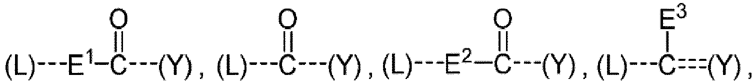 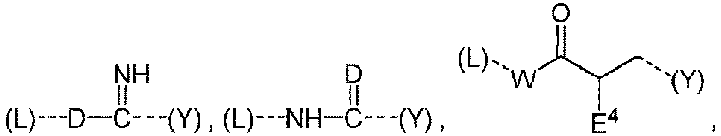 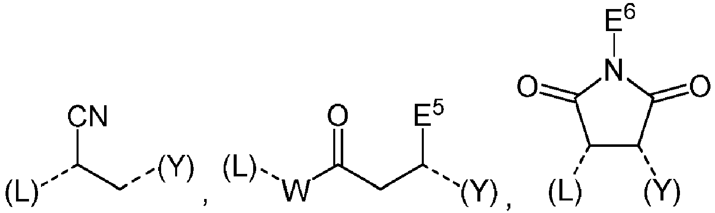 et 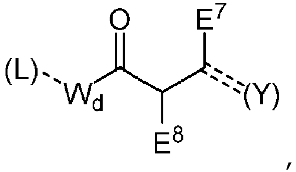
D représentant O ou S,
W représentant O ou NR⁶, R⁶ représentant un radical hydrogène ou un radical hydrocarboné monovalent de 1 à 8 atomes C,
E⁰ représentant un radical hydrogène ou un radical méthyle,
E¹ représentant un radical alkylène, alcènediyle ou phénylène à substitution carboxyle de 2 à 8 atomes C,
E² représentant un radical hydroxyalkylène de 2 à 5 atomes C ou un radical hydroxyalkylène-oxy de 2 ou 3 atomes C relié par O,
E³ représentant un radical hydrogène ou un radical hydrocarboné monovalent de 1 à 6 atomes C, qui comprend éventuellement des hétéroatomes sous la forme de groupes éther, ester, amino ou amide, ou, conjointement avec L, un radical 1,4-butylène ou 1,5-pentylène éventuellement substitué,
E⁴ représentant un radical hydrogène ou méthyle ou alcoxycarbonylméthyle de 2 à 9 atomes C,
E⁵ représentant un radical alkyloxycarbonyle de 1 à 8 atomes C ou un radical méthyle,
E⁶ représentant un radical hydrogène ou un radical alkyle de 1 à 8 atomes C,
E⁷ représentant un radical hydrocarboné monovalent de 1 à 6 atomes C,
E⁸ représentant un radical hydrogène ou un radical hydrocarboné monovalent de 1 à 6 atomes C, et
d représentant 0 ou 1.

6. Utilisation selon l'une quelconque des revendications précédentes, **caractérisée en ce que** p représente 1 ou 2 ou 3 et r représente 0.

7. Utilisation selon l'une quelconque des revendications précédentes, **caractérisée en ce que** la composition durcissable est une composition de résine époxyde ou une composition de polyuréthane ou une composition de résine époxyde-polyuréthane ou une composition de résine d'ester de cyanate ou une composition contenant des groupes silane.

8. Utilisation selon l'une quelconque des revendications précédentes, **caractérisée en ce que** la composition durcissable est une composition à base de polymères contenant des groupes silane.

9. Utilisation selon la revendication 8, **caractérisée en ce que** le polymère contenant des groupes silane est choisi dans le groupe constitué par les polyorganosiloxanes munis de groupes silane terminaux et les polymères organiques contenant des groupes silane.

10. Procédé de fabrication du catalyseur de la formule (I) tel que décrit dans l'une quelconque des revendications 1 à 6, **caractérisé en ce que**
- au moins une guanidine de la formule (II)
HX-A-Z (II)
- est mise en réaction avec au moins un composé fonctionnel qui comprend au moins un groupe réactif choisi parmi époxyde, aziridine, carbonate, anhydride d'acide carboxylique, acide carboxylique, ester d'acide carboxylique, lactone, chlorure d'acide carboxylique, cétone, aldéhyde, 1,3-dicétone, 1,3-cétoester, 1,3-cétoamide, cyanate, thiocyanate, isocyanate, isothiocyanate, (méth)acrylate, (méth)acrylamide, (méth)acrylonitrile, maléate, maléamide, maléimide, fumarate, fumaramide, itaconate, itaconamide, crotonate et crotonamide.

11. Composition durcissable contenant au moins un catalyseur de la formule (I) tel que décrit dans l'une quelconque des revendications 1 à 6.

12. Composition selon la revendication 11, **caractérisée en ce qu'**elle contient en outre au moins un polymère contenant des groupes silane.

13. Composition selon la revendication 12, **caractérisée en ce que** le polymère contenant des groupes silane est un polyorganosiloxane muni de groupes silane terminaux.

14. Composition selon la revendication 12, **caractérisée en ce que** le polymère contenant des groupes silane est un polymère organique contenant des groupes silane.

15. Composition selon l'une quelconque des revendications 11 à 14, **caractérisée en ce qu'**elle consiste en un adhésif ou un agent d'étanchéité ou un revêtement.
